# EUROPEAN PATENT APPLICATION

(11) **EP 4 729 122 A2**
(43) Date of publication of application: **22.04.2026**
(21) Application number: 25223916.5
(22) Date of filing: 13.02.2023
(51) Int. Cl.: A61P 25/18

(54) **USE OF ROLUPERIDONE IN PREVENTING RELAPSE IN SCHIZOPHRENIA PATIENTS**

(30) Priority: 14.02.2022 US 202263309874 P
(62) Divisional of application: 23710619.0
(71) Applicant: Minerva Neurosciences, Inc., Burlington, MA 01803 (US)
(72) Inventor: LUTHRINGER, Remy, 1204 Geneva (CH)
(74) Representative: Cooley (UK) LLP

(57) **Abstract**

This application pertains to methods of preventing relapse in schizophrenia patients that include administering a therapeutically effective amount of Roluperidone to the schizophrenia patient.

## Description

### RELATED APPLICATIONS

This application claims priority to, and the benefit of, U.S. Provisional Application No. 63/309,874, filed February 14, 2022, which is incorporated herein by reference it its entirety for all purposes.

### FIELD OF THE DISCLOSURE

The present disclosure relates generally to the prevention of relapse in schizophrenia patients.

### BACKGROUND

Roluperidone ("MIN-101") is a novel cyclic amido derivative, that has antagonistic activities for serotoninergic 5-HT2A, sigma2, α1A-adrenergic, and to a lesser extent, α1B-adrenergic receptors. Roluperidone has very low or no affinity for dopaminergic (DA), muscarinic, cholinergic, and histaminergic receptors. The molecule was specifically designed to allow improvement of primary negative symptoms without blocking the brain's DA-driven reward systems as it is the case with currently available treatments called antipsychotics that interfere with DA neurotransmission. Also, because Roluperidone does not bind to DA or histaminergic receptors, it does not produce secondary negative symptoms such as Parkinsonism and sedation.

Schizophrenia is a chronic, severe and debilitating type of mental illness characterized by distortions in thinking, perception, emotions, language, sense of self and behavior. According to the World Health Organization, schizophrenia affects about 1% of the general population. Most individuals affected by schizophrenia suffer from psychosis or positive symptoms, negative symptoms and cognitive impairment. Positive symptoms manifest as delusions and hallucinations while negative symptoms are characterized by blunted affect, alogia, avolition, anhedonia, and asociality (Marder, et al. "The Current Conceptualization of Negative Symptoms in Schizophrenia," World Psychiatry, 2017, 16(1), 14-24).

Negative symptoms are the main cause of the poor functional outcome of patients suffering from schizophrenia (Harvey et al., "Effects of Roluperidone (MIN-101) on Two Dimensions of the Negative Symptoms Factor Score: Reduced Emotional Experience and Reduced Emotional Expression," Schizophr. Res. 2020, 215, 352-356) and in ultrahigh risk adolescents are associated with transition to full blown schizophrenia (Gomes and Grace, "Adolescent Stress as a Driving Factor for Schizophrenia Development - a Basic Science Perspective" Schizophrenia Bulletin, 2017, 43, 10.1093/schbul/sbx033). There are currently no treatments approved for negative symptoms of schizophrenia in the United States.

Schizophrenia is a life-long chronic disease characterized by periods of acute exacerbation or symptomatic worsening and periods of remission or symptomatic improvement. Relapse is associated with substantial worsening of social and vocational functioning and an overall decrease in quality of life (Jørgensen, "Predicting time to relapse in patients with schizophrenia according to patients' relapse history: a historical cohort study using real-world data in Sweden," BMC Psychiatry, 2021, 21(634), 1-12).

### SUMMARY

In one aspect, the application pertains to a method of preventing relapse in a schizophrenia patient, wherein the method comprises administering a therapeutically effective amount of Roluperidone to the schizophrenia patient.

In one aspect, the application pertains to Roluperidone for use in a method of preventing relapse in a schizophrenia patient, wherein the method comprises administering a therapeutically effective amount of Roluperidone to the schizophrenia patient.

In some embodiments, the therapeutically effective amount of Roluperidone is administered to the schizophrenia patient once or twice a day.

In some embodiments, the therapeutically effective amount of Roluperidone is administered to the schizophrenia patient once a day.

In some embodiments, the therapeutically effective amount of Roluperidone is administered orally to the schizophrenia patient.

In some embodiments, the therapeutically effective amount of Roluperidone is between about 1 and about 100 mg.

In some embodiments, the therapeutically effective amount of Roluperidone is between 1 and 100 mg.

In some embodiments, the therapeutically effective amount of Roluperidone is about 16 mg, about 24 mg, about 32 mg, about 40 mg, about 48 mg, about 56 mg, about 64 mg, about 72 mg, about 80 mg, about 88 mg, or about 96 mg.

In some embodiments, the therapeutically effective amount of Roluperidone is 16 mg, 24 mg, 32 mg, 40 mg, 48 mg, 56 mg, 64 mg, 72 mg, 80 mg, 88 mg, or 96 mg.

In some embodiments, the therapeutically effective amount of Roluperidone is about 32 mg.

In some embodiments, the therapeutically effective amount of Roluperidone is 32 mg.

In some embodiments, the therapeutically effective amount of Roluperidone is about 64 mg.

In some embodiments, the therapeutically effective amount of Roluperidone is 64 mg.

In some embodiments, the schizophrenia patient does not manifest any behavior which puts the patient, or those around the patient, at risk of bodily harm.

In some embodiments, the schizophrenia patient has low levels of symptoms related to agitation, impulse control, hostility, suspiciousness, or uncooperativeness.

In some embodiments, the schizophrenia patient does not experience a high level of depression or anxiety.

In some embodiments, the schizophrenia patient has positive symptoms that are stable before starting treatment with Roluperidone.

In some embodiments, the schizophrenia patient does not have positive symptoms before starting treatment with Roluperidone.

In some embodiments, the schizophrenia patient has positive symptoms that are stable for about 1 to about 6 months before starting treatment with Roluperidone.

In some embodiments, the schizophrenia patient does not have positive symptoms for about 1 to about 6 months before starting treatment with Roluperidone.

In some embodiments, the schizophrenia patient has positive symptoms that are stable for about 3 to about 6 months before starting treatment with Roluperidone.

In some embodiments, the schizophrenia patient does not have positive symptoms for about 3 to about 6 months before starting treatment with Roluperidone.

In some embodiments, the schizophrenia patient has negative symptoms that are moderate to severe.

In some embodiments, the schizophrenia patient's PANSS (positive and negative syndrome scale) negative subscore is greater than 20.

In some embodiments, the schizophrenia patient's negative symptoms are primary negative symptoms.

In some embodiments, the schizophrenia patient's negative symptoms are stable for about 1 to about 6 months before starting treatment with Roluperidone.

In some embodiments, the schizophrenia patient's negative symptoms are stable for about 3 to about 6 months before starting treatment with Roluperidone.

In some embodiments, the schizophrenia patient was previously administered an antipsychotic.

In some embodiments, the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months, or at least 12 months before the schizophrenia patient was administered Roluperidone.

In some embodiments, the relapse is an increase in positive symptoms in the schizophrenia patient, optionally wherein the increase in positive symptoms in the schizophrenia patient is marked by an increase in the patient's PANSS positive subscore on one or more consecutive visits.

In one aspect, the application pertains to a method of selecting a schizophrenia patient and preventing relapse in the schizophrenia patient, wherein the method comprises:
(a) selecting the schizophrenia patient as having a form of schizophrenia characterized by the schizophrenia patient having moderate to severe negative symptoms that are stable for about 3 to about 6 months; and
(b) administering a therapeutically effective amount of Roluperidone to the schizophrenia patient.

In one aspect, the application pertains to Roluperidone for use in a method of preventing relapse in a schizophrenia patient, wherein the method comprises:
(a) selecting the schizophrenia patient as having a form of schizophrenia characterized by the schizophrenia patient having moderate to severe negative symptoms that are stable for about 3 to about 6 months; and
(b) administering a therapeutically effective amount of Roluperidone to the schizophrenia patient.

In some embodiments, the schizophrenia patient has a PANSS negative subscore that is greater than 20.

In some embodiments, the schizophrenia patient's negative symptoms are primary negative symptoms.

In some embodiments, the schizophrenia patient has positive symptoms that are stable before starting treatment with Roluperidone; optionally wherein the schizophrenia patient has positive symptoms that are stable for about 1 to about 6 months, or about 3 to about 6 months, before starting treatment with Roluperidone.

In some embodiments, the schizophrenia patient does not have positive symptoms before starting treatment with Roluperidone; optionally wherein the schizophrenia patient does not have positive symptoms for about 1 to about 6 months, or about 3 months to about 6 months, before starting treatment with Roluperidone.

In some embodiments, the schizophrenia patient does not manifest any behavior which puts the patient, or those around the patient, at risk of bodily harm; has low levels of symptoms related to agitation, impulse control, hostility, suspiciousness, or uncooperativeness; and/or does not experience a high level of depression or anxiety.

In some embodiments, the schizophrenia patient was previously administered an antipsychotic.

In some embodiments, the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months, or at least 12 months before the schizophrenia patient was administered Roluperidone.

In some embodiments, the relapse is an increase in positive symptoms in the schizophrenia patient, optionally wherein the increase in positive symptoms in the schizophrenia patient is marked by an increase in the patient's PANSS positive subscore on one or more consecutive visits.

In some embodiments, the therapeutically effective amount of Roluperidone is administered to the schizophrenia patient once or twice a day.

In some embodiments, the therapeutically effective amount of Roluperidone is administered to the schizophrenia patient once a day.

In some embodiments, the therapeutically effective amount of Roluperidone is administered orally to the schizophrenia patient.

In some embodiments, the therapeutically effective amount of Roluperidone is between about 1 and about 100 mg.

In some embodiments, the therapeutically effective amount of Roluperidone is between 1 and 100 mg.

In some embodiments, the therapeutically effective amount of Roluperidone is about 16 mg, about 24 mg, about 32 mg, about 40 mg, about 48 mg, about 56 mg, about 64 mg, about 72 mg, about 80 mg, about 88 mg, or about 96 mg.

In some embodiments, the therapeutically effective amount of Roluperidone is 16 mg, 24 mg, 32 mg, 40 mg, 48 mg, 56 mg, 64 mg, 72 mg, 80 mg, 88 mg, or 96 mg.

In some embodiments, the therapeutically effective amount of Roluperidone is about 32 mg.

In some embodiments, the therapeutically effective amount of Roluperidone is 32 mg.

In some embodiments, the therapeutically effective amount of Roluperidone is about 64 mg.

In some embodiments, the therapeutically effective amount of Roluperidone is 64 mg.

### BRIEF DESCRIPTION OF THE DRAWINGS

FIG. 1 shows the effect of Roluperidone administered orally at 32 mg/day and 64 mg/day compared to placebo on the PANSS positive symptom subscore in schizophrenic patients, ITT Population (Study #1).

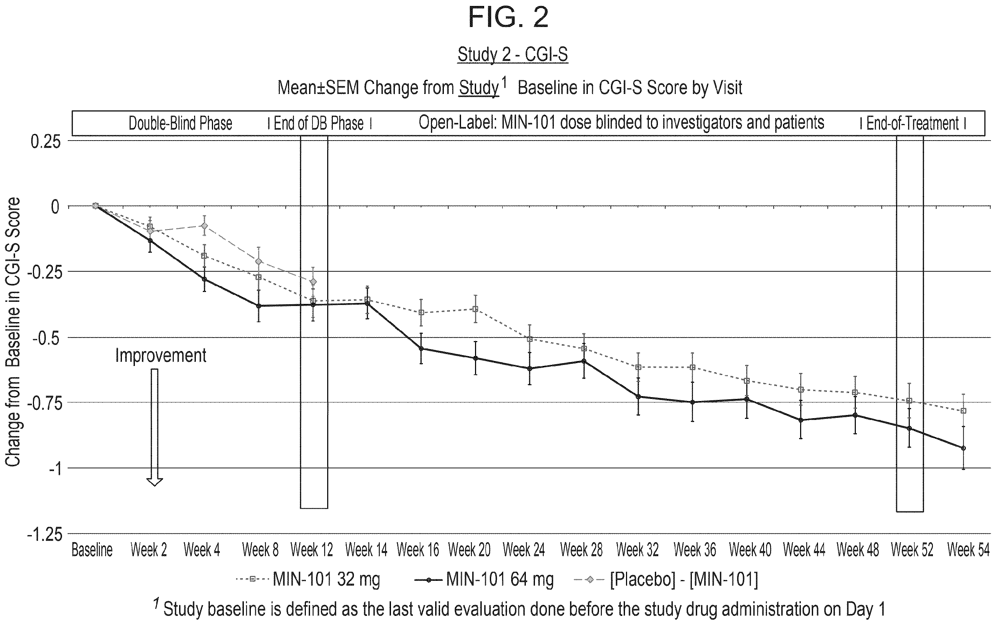

FIG. 2 shows the effect of Roluperidone administered orally at 32 mg/day and 64 mg/day compared to placebo on the clinical global scale-severity scale (CGI-S) score in schizophrenic patients (Study #2).

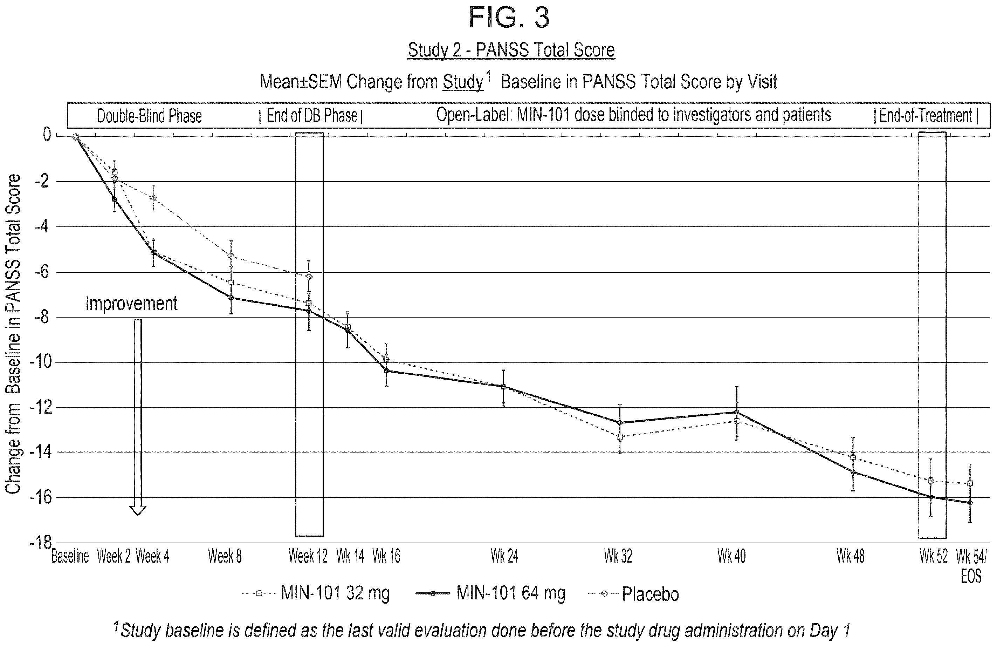

FIG. 3 shows the effect of Roluperidone administered orally at 32 mg/day and 64 mg/day compared to placebo on the PANSS total score in schizophrenic patients (Study #2).
FIG. 4 shows the effect of Roluperidone administered orally at 32 mg/day and 64 mg/day compared to placebo on the PANSS positive symptom subscore in schizophrenic patients, ITT Population (Study #2).

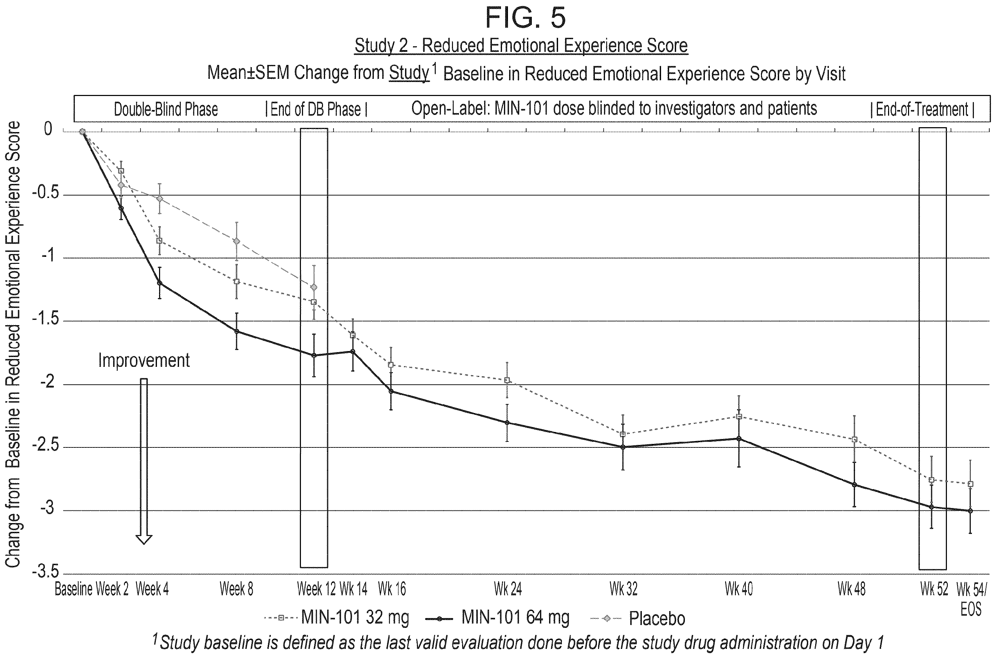

FIG. 5 shows the effect of Roluperidone administered orally at 32 mg/day and 64 mg/day compared to placebo on the Reduced Emotional Experience Score in schizophrenic patients (Study #2).
FIG. 6 shows the time to relapse in the Double-Blind Period, ITT Population for Study #2 in schizophrenic patients orally administered Roluperidone at 32 mg/day and 64 mg/day compared to placebo.
FIG. 7 shows the time to relapse in the Open-Label Period, ITT Population for Study #2 in schizophrenic patients who were administered placebo in the DB Period, then either orally administered Roluperidone at 32 mg/day or 64 mg/day, or who were orally administered Roluperidone at 32 mg/day or 64 mg/day in both the DB and OL periods.
FIG. 8 is a graphic summarizing Global Statistical Tests for Study No. 1 ("MIN-101C03"), Study No. 2 ("MIN-101C07"), and the Integrated Study for the Combined ITT Population ("ISE").
FIG. 9 is a Kaplan-Meier Plot of Time to Relapse - Double-blind Period, Pooled ITT Population, showing the time to relapse in the Double-Blind Period, Pooled ITT Populations, in schizophrenic patients orally administered: (1) Roluperidone at 32 mg/day; (2) Roluperidone at 64 mg/day; or (3) placebo.
FIG. 10 is a Plot of Change from Active Baseline in NSFS Score - Open-label Period, Pooled ITT Population, showing the change in NSFS score in the four treatment groups: (1) placebo in DB period → oral Roluperidone at 32 mg/day in OL period; (2) placebo in DB period → oral Roluperidone at 64 mg/day in OL period; (3) oral Roluperidone at 32 mg/day in DB and OL periods; and (4) oral Roluperidone at 64 mg/day in DB and OL periods.
FIG. 11 is a Plot of Change from Active Baseline in PSP Total Score - Open-label Period, ITT Population, showing the change in PSP Total Score in the four treatment groups: (1) placebo in DB period → oral Roluperidone at 32 mg/day in OL period; (2) placebo in DB period → oral Roluperidone at 64 mg/day in OL period; (3) oral Roluperidone at 32 mg/day in DB and OL periods; and (4) oral Roluperidone at 64 mg/day in DB and OL periods.
FIG. 12 is a Plot of Change from Active Baseline in GSI-S Score - Open-label Period, Pooled ITT Population, showing the change in CGI-S score in the four treatment groups: (1) placebo in DB period → oral Roluperidone at 32 mg/day in OL period; (2) placebo in DB period → oral Roluperidone at 64 mg/day in OL period; (3) oral Roluperidone at 32 mg/day in DB and OL periods; and (4) oral Roluperidone at 64 mg/day in DB and OL periods.
FIG. 13 is a Plot of Change from Active Baseline in GSI-I Score - Open-label Period, Pooled ITT Population, showing the change in CGI-I score in the four treatment groups: (1) placebo in DB period → oral Roluperidone at 32 mg/day in OL period; (2) placebo in DB period → oral Roluperidone at 64 mg/day in OL period; (3) oral Roluperidone at 32 mg/day in DB and OL periods; and (4) oral Roluperidone at 64 mg/day in DB and OL periods.
FIG. 14 is a Kaplan-Meier Plot of Time to Relapse - Whole Study, Pooled ITT Population, showing the time to relapse in the Whole Study, Pooled ITT Populations, in schizophrenic patients administered: (1) placebo in DB period, followed by oral administration of Roluperidone at 32 mg/day in the OL period; (2) placebo in DB period, followed by oral administration of Roluperidone at 64 mg/day in the OL period, (3) Roluperidone (oral) at 32 mg/day throughout the Whole Study; (4) Roluperidone (oral) at 64 mg/day throughout the Whole Study; and (5) placebo (in the DB period only).

### DETAILED DESCRIPTION

The terms used herein have their ordinary meaning and the meaning of such terms is independent at each occurrence thereof. Notwithstanding the foregoing, and except where stated otherwise, the following definitions apply throughout the specification and claims.

The term "about" is used herein to mean approximately, in the region of, roughly, or around. When the term "about" is used in conjunction with a numerical range, it modifies that range by extending the boundaries above and below the numerical values set forth. In general, unless otherwise specified or unless the context clearly dictates otherwise, the term "about" is used herein to modify a numerical value above and below the stated value by a variance of 20%. In some embodiments, the term "about" refers to a variance of 10%, a variance of 5%, a variance of 3%, or a variance of 1%. For example, "about 64 mg" is equivalent to a range of 57.6 mg to 70.4 (10% variance), or a range of 60.8 mg to 67.2 mg (5% variance), or a range of 63.36 mg to 64.64 mg (1% variance).

"Administration" refers to introducing an agent, such as a Roluperidone or a dosage form thereof, into a subject. The related terms "administering" and "administration of" (and grammatical equivalents) refer both to direct administration, which may be administration to a subject by a medical professional or by self-administration by the subject, and/or to indirect administration, which may be the act of prescribing a drug such as a dosage form described herein. For example, a physician or investigator who instructs a patient to self-administer a drug and/or provides a patient with a prescription for a drug is administering the drug to the patient.

As used herein, Personal and Social Performance ("PSP"), is a validated clinician-rated scale intended to reflect real-life situations that measures personal and social functioning in 4 domains: (a) socially useful activities, (b) personal and social relationships, (c) self-care, and (d) disturbing and aggressive behaviors. The score is based on the assessment of a patient's performance in the 4 domains. The PSP Total score is a single measurement of functioning ranging from 1 to 100, where a higher score represents better functioning. A score of 91 to 100 indicates excellent functioning in all 4 main areas; the patient is held in high consideration for their good qualities, copes adequately with life problems, and is involved in a wide range of interests and activities. A score of 1 to 10 indicates a lack of autonomy in basic functioning with extreme behaviors but without survival risk (score 6 to 10) or with survival risk (score 1 to 5). An increase in the scale demonstrates a beneficial response. Review of the relevant scientific literature and the analysis of the psychometric properties of the 4 domains that result in the generation of the PSP Total score were supportive of the appropriateness and cross-cultural applicability of the use of the PSP in drug development for the indication of negative symptoms of schizophrenia and preventing relapse.

As used herein, Clinical Global Impression - Severity Scale ("CGI-S") is a clinician-rated scale that is designed to rate the severity of the patient's illness at the time of assessment, including knowledge of the patient's history, psychosocial circumstances, symptoms, behavior, and the impact of the symptoms on the patient's ability to function relative to the clinician's past experience with patients who have the same diagnosis and similar improvement with treatment. Considering total clinical experience, a patient is assessed on severity of mental illness at the time of rating, where 1 = normal (not at all ill); 2 = borderline mentally ill; 3 = mildly ill; 4 = moderately ill; 5 = markedly ill; 6 = severely ill; or 7 = extremely ill.

As used herein, Clinical Global Impression - Improvement Scale ("CGI-I") is a 7-point scale that requires the clinician to assess how much the patient's illness has improved or worsened relative to a baseline state at the beginning of the intervention and is rated as 1 = very much improved; 2 = much improved; 3 = minimally improved; 4 = no change; 5 = minimally worse; 6 = much worse; or 7 = very much worse.

As used herein, "BNSS" is the Brief Negative Symptom Scale. "BNSS" is 13-item instrument designed to measure negative symptoms, specifically: blunted affect, alogia, asociality, anhedonia, and avolition (Kirkpatrick, et al., "The Brief Negative Symptom Scale: Psychometric Properties," Schizophr. Bull., 2011, 37(2), 300-5.).

"Time to relapse" in the DB period is defined as the number of days from Day 1 to the date of early trial termination due to relapse. If the patient did not relapse during the DB period, he or she was censored at the date of termination from or completion of the DB period. Treatment differences in time to relapse in the DB period were analyzed using a Cox-regression model with treatment group and Baseline PANSS total score as covariates. For the analysis of the OL period, this analysis was repeated to analyze treatment differences in time to relapse in the study. For the OL period analysis, patients who were not observed to relapse in the study were censored on the date on which they ended the study. Kaplan-Meier plots of time to relapse in the DB period, OL period, and in the study were generated.

"Comprising" or "comprises" as applied to a particular dosage form, composition, method or process described or claimed herein means that the dosage form, composition or method includes all of the recited elements in a specific description or claim, but does not exclude other elements. "Consists essentially of" and "consisting essentially of" means that the described or claimed composition, dosage form, method or process does not exclude other materials or steps that do not materially affect the recited physical, pharmacological, pharmacokinetic properties or therapeutic effects of the composition, dosage form, method or process. "Consists of" and "consisting of" means the exclusion of more than trace elements of other ingredients and substantial method or process steps.

"CYP2D6 allele" refers to one of over 100 named versions of the CYP2D6 gene that are present in the general population, and typically classified into one of three categories: active (functional); decreased activity (partially active or decreased function) and inactive (non-functional).

Active CYP2D6 alleles include: *1, *2, *2A, *33, *35, *39, *48, and *53.

Decreased activity CYP2D6 alleles include: *9, *10, *17, *29, *41, *49, *50, *54, *55, *59, *69, and *72.

Inactive CYP2D6 alleles include: *3, *4, *5 (deletion), *6, *7, *8, *11, *12, *13, *14A, *14B, *15, *18, *19, *20, *21, *38, *40, *42, *44, *56, *56A, *56B, and *68.

"CYP2D6 Extensive Metabolizer (EM) genotype" as applied to a subject means the subject has a CYP2D6 which results in CYP2D6 metabolic activity considered as normal. CYP2D6 EM genotypes include combinations of: (a) two active CYP2D6 alleles, (b) one active and one decreased activity CYP2D6 allele, and (c) one active and one inactive CYP2D6 allele.

"CYP2D6 Intermediate Metabolizer (IM) genotype" as applied to a subject means the subject has a CYP2D6 genotype, which results in reduced CYP2D6 metabolic activity. CYP2D6 IM genotypes include combinations of: (a) one inactive and one decreased activity CYP2D6 allele; and (c) two decreased activity CYP2D6 alleles.

"CYP2D6 PM genotype" as applied to a subject means the subject has a positive test result for a CYP2D6 poor metabolizer genotype and thus likely to have no CYP2D6 activity. A CYP2D6 PM genotype is 2 inactive alleles.

"CYP2D6 UM genotype" as applied to a subject means the subject has a positive test result for a CYP2D6 ultrarapid metabolizer genotype and thus likely to have higher than average CYP2D6 activity. A CYP2D6 UM genotype is 3 or more active alleles.

As used here, the terms "patient" or "subject" are used interchangeably and refer to a human of any age.

A "schizophrenia patient," as used herein, refers to a human who was previously diagnosed with schizophrenia, i.e., the patient met the diagnostic criteria for schizophrenia as defined in the Diagnostic and Statistical Manual of Mental Disorders (DSM-5), as established by a full psychiatric interview in conjunction with the Mini International Neuropsychiatric Interview.

As used herein, "antipsychotic" refers to a drug that is administered to a treat symptoms of schizophrenia. In some embodiments, the antipsychotic is a first generation antipsychotic. In some embodiments, the antipsychotic is a second generation antipsychotic. In some embodiments, the antipsychotic is an atypical antipsychotic. In some embodiments, the antipsychotic is amisulpride, clozapine, olanzapine, quetiapine, risperidone, sertindole, ziprasidone, zotepine, haloperidol, chlorpromazine, perphenazine, brexiprazole, cariprazine, or lumateperone.

Although not desiring to be limiting to the definition, in some embodiments, the antipsychotic is described in the clinical literature, for example in: BAGNALL, et al., "A systematic review of atypical antipsychotic drugs in schizophrenia," Health Technology Assessment, 2003, 7(13), 1-214; BARMAN, et al., "Newer antipsychotics: Brexpiprazole, cariprazine, and lumateperone: A pledge or another unkept promise?" World J. Psychiatr., December 19, 2021, 11(12), 1228-1238; BEASLEY, Jr., et al., "A Double-Blind, Randomized, Placebo-Controlled Trial of Olanzapine in the Prevention of Psychotic Relapse," Journal of Clinical Psychopharmacology, December 2003, 23(6), 582-594, and KRAUSE, et al., "Antipsychotic drugs for patients with schizophrenia and predominant or prominent negative symptoms: a systematic review and meta-analysis," European Archives of Psychiatry and Clinical Neuroscience, 2018, 268, 625-639; the contents of which are incorporated herein in their entirety.

"PANSS", as used herein, refers to the positive and negative syndrome scale, which is used by physicians and clinicians to measure the severity of the symptoms in a schizophrenia patient. The scale is divided into three parts, a positive scale, which provides a "PANSS positive subscore", a negative scale, which provides a "PANSS negative subscore", and general psychopathology scale. The sum of these three parts provides a PANSS total score, which ranges from 30 to 210 (where higher scores indicate more severe symptoms). (Kay, S.R., et al. "The positive and negative syndrome scale (PANSS) for schizophrenia," Schizophr Bulletin, 13(2), 261-276 (1987)).

In some embodiments, a schizophrenia patient having moderate negative symptoms has a PANSS negative subscore greater than 20, but less than 35. In some embodiments, a schizophrenia patient having moderate negative symptoms has a PANSS negative subscore greater than 15, but less than 35. In some embodiments, a schizophrenia patient having moderate negative symptoms has a PANSS negative subscore greater than 10, but less than 35.

In some embodiments, a schizophrenia patient having severe negative symptoms has a PANSS negative subscore greater than or equal to 35.

As used herein, PANSS Marder's negative symptoms factor score ("NSFS"), refers to selection of seven of the PANSS items used by physicians and clinicians to measure the severity of the negative symptoms in a schizophrenia patient. (See Tables 5A and 5B.) The NSFS total score ranges from 7 to 49. (Marder, S. R. et al. "The effects of risperidone on the five dimensions of schizophrenia derived by factor analysis: combined results of the North American trials," J Clin Psychiatry. 1997; 58:538-46.)

In some embodiments, a schizophrenia patient having moderate negative symptoms has a NSFS score greater than 20, but less than 35. In some embodiments, a schizophrenia patient having moderate negative symptoms has a NSFS score greater than 15, but less than 35. In some embodiments, a schizophrenia patient having moderate negative symptoms has a NSFS score greater than 10, but less than 35.

In some embodiments, a schizophrenia patient having severe negative symptoms has a NSFS score greater than or equal to 35.

In some embodiments, a schizophrenia patient will be referred to herein as having "stable" positive or negative symptoms as judged by the treating physician or investigator.

In some embodiments, a treating physician or investigator would determine that a schizophrenia patient's PANSS positive or negative subscores are stable if the patient's subscores are within about ± 4 points on two successive PANSS assessments, wherein the duration between the two successive assessments is 1 day, 2 days, 3 days, 4 days, 5 days, 6 days, 1 week, 2 weeks, 3 weeks, 4 weeks, 5 weeks, 6 weeks, 7 weeks, 8 weeks, 2 months, 3 months, or any duration in between.

In some embodiments, a treating physician or investigator would determine that a schizophrenia patient's PANSS positive or negative subscores are stable if the patient's subscores are within about ± 4 points on two out of three successive PANSS assessments, wherein the duration between the two successive assessments is 1 day, 2 days, 3 days, 4 days, 5 days, 6 days, 1 week, 2 weeks, 3 weeks, 4 weeks, or any duration in between.

Positive symptoms generally involve the experience of something in perception or ideations that should not normally be present. For example, hallucinations and delusions represent perceptions or beliefs that should not normally be experienced. In addition to hallucinations and delusions, patients with schizophrenia frequently have marked disturbances in the logical process of their thoughts. Specifically, psychotic thought processes are characteristically loose, disorganized, illogical, or bizarre. These disturbances in thought process frequently produce observable patterns of behavior that are also disorganized and bizarre. The severe disturbances of thought content and process that comprise the positive symptoms often are the most recognizable and striking features of schizophrenia. Positive symptoms such as hallucinations and delusions are responsible for much of the acute distress associated with schizophrenia.

Negative symptoms appear to be responsible for much of the chronic and long-term social and vocational disability associated with schizophrenia. Negative symptoms generally refer to a reduction in normal functioning, and include five major sub-domains: blunted affect (affective flattening, blunted expression, reduced emotional response), alogia (poverty of speech), amotivation (loss of volition), anhedonia (reduced ability to experience or anticipate pleasure) and asociality (social withdrawal). As used herein, the term "negative symptoms" is to be understood as including primary negative symptoms typically associated with schizophrenia, the negative symptoms measured in the PANSS negative symptoms subscale score, the negative factor score based on the pentagonal structure model method (White, "Empirical Assessment of the Factorial Structure of Clinical Symptoms in Schizophrenia," Psychopathology 1997, 30(5), 263-74), the Marder negative symptoms subscore, and the negative symptoms measured in the BNSS.

In some embodiments, the negative symptom is one of the five major sub-domains of negative symptoms: blunted affect, alogia, amotivation, anhedonia and asociality. Blunted affect (affective flattening, blunted expression) is characterized by reduced intensity and range of emotional expression as manifested via vocal and non-verbal modes of communication including intonation (prosody), facial expression, hand-gestures and body movements. Alogia (poverty of speech) is characterized by decreased quantity of speech, reduced spontaneous speech and loss of conversational fluency. Amotivation (loss of volition) is characterized by deficits in the initiation and maintenance of goal-directed behaviors like work, study, sport, personal hygiene and daily tasks, especially when requiring and effort (cognitive or physical) and significant organization, as well as deficits in desire to undertake such activities. This sub-domain is related to apathy and lack of energy. Anhedonia (reduced ability to experience or anticipate pleasure) is characterized by the looking forward to a reward, recreational or other pleasurable experience ("wanting") being more markedly and consistently impaired (anticipatory anhedonia) than the appreciation ("liking") of the experience itself (consummatory anhedonia). Asociality (social withdrawal) is characterized by diminished interest in, motivation for, and appreciation of social interactions with others, like family and friends, loss of interest in intimate (sexual) relationships independent of any somatic problems, and for a child, may include loss of interest in playing with other children.

In some embodiments, negative symptoms are primary negative symptoms, which include, for example, blunted affect, alogia, amotivation, anhedonia and asociality.

In some embodiments, negative symptoms are secondary negative symptoms, which may overlap with primary negative symptoms, but, in contrast to primary negative symptoms, are related to comorbidities or treatment side effects. In some embodiments, secondary negative symptoms are caused by, for example, comorbid depression and/or medication side effects.

In some embodiments, secondary negative symptoms occur in association with positive symptoms (Kirkpatrick, "Recognizing Primary vs. Secondary Negative Symptoms and Apathy vs. Expression Domains," J. Clin. Psychiatry, 2014, 75(4):e09. (doi: 10.4088/JCP.13049tx3c.).

In some embodiments, the secondary negative symptom is a movement disorder.

In some embodiments, the secondary negative symptom is an extrapyramidal symptom, such as akathisia, tardive dyskinesia, dystonia, or Parkinsonism.

In some embodiments, the secondary negative symptom is demoralization.

### ROLUPERIDONE

Roluperidone, *i.e.,* 1H-Isoindol-1-one,2-[[1-[2-(4-fluorophenyl)-2-oxoethyl]-4-piperidinyl]methyl]-2,3-dihydro-, hydrochloride, hydrate (1:1:2), refers to a compound having the following structure:

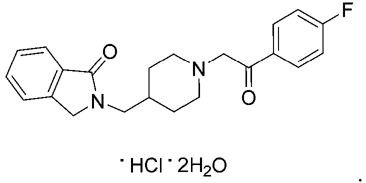

Roluperidone may be synthesized using standard synthetic methods and procedures for the preparation of organic molecules and functional group transformations and manipulations, including the use of protective groups, as can be obtained from the relevant scientific literature or from standard reference textbooks in the field. Although not limited to any one or several sources, recognized reference textbooks of organic synthesis include: Smith, M.B.; March, J. March's Advanced Organic Chemistry: Reactions, Mechanisms, and Structure, 5th ed.; John Wiley & Sons: New York, 2001; and Greene, T.W.; Wuts, P.G. M. Protective Groups in Organic Synthesis, 3rd; John Wiley & Sons: New York, 1999. A method for preparing Roluperidone is described in U.S. Patent No. 7,166,617, the contents of which are incorporated herein in their entirety.

Roluperidone is a dihydrate of a hydrochloride salt. The "therapeutically effective amount" of Roluperidone referred to in the methods of the disclosure is based on the corresponding amount of the free base form of Roluperidone that is sufficient to prevent relapse, wherein relapse is defined herein. For instance, a therapeutically effective amount of 32.0 mg refers to 32.0 mg of the free base, which is equivalent to 38.4 mg of Roluperidone (the dihydrate hydrochloride salt); and a therapeutically effective amount of 64.0 mg refers to 64.0 mg of the free base, which is equivalent to 76.8 mg of Roluperidone (the dihydrate hydrochloride salt).

Dosage forms comprising Roluperidone are disclosed in U.S. Patent Nos. 9,458,130, 9,730,920, 10,258,614, 10,799,493, and 11,464,744, each of which is incorporated herein by reference in its entirety. In some embodiments, the methods of this disclosure are performed using any of the dosage forms disclosed therein.

### RELAPSE

Relapse in schizophrenia patients is a recurring feature of the disease and can have serious economic and personal consequences. It manifests as recurrence of delusions hallucinations and/or the occurrence or worsening of negative symptoms. Relapse may be characterized by the acute increase in positive and/or negative symptoms. In addition to relapse presenting a risk of patients to harm themselves or others, relapse can jeopardize personal relationships, educational pursuits, and/or employment status. Additionally, the relapse results in the patient being further stigmatized from the illness (EMSLEY, et al., "The nature of relapse in schizophrenia," BMC Psychiatry, 2013, 13(50), 1-8).

Relapse carries an additional risk in that patients may not return to their previous levels of functioning once they begin treatment again. Patients who experience multiple relapses may need longer recovery times and may have a decreased likelihood of regaining previous (pre-relapse) levels of health and functioning (Jørgensen, et al., "Predicting time to relapse in patients with schizophrenia according to patients' relapse history: a historical cohort study using real-world data in Sweden," BMC Psychiatry, 2021, 21(634), 1-12).

One factor that can lead to an increased risk of a schizophrenia patient's relapse is discontinuing their prescribed antipsychotic medication currently available to reduce risk of relapse. Trials comparing antipsychotic maintenance with antipsychotic discontinuation have shown that patients who have medication withdrawn are more likely to relapse (Leucht S., et al., "Antipsychotic drugs versus placebo for relapse prevention in schizophrenia: a systematic review and meta-analysis. Lancet. 2012 Jun 2;379(9831):2063-71. doi: 10.1016/S0140-6736(12)60239-6. Epub 2012 May 3. PMID: 22560607).

Many antipsychotics, including second generation antipsychotics, cause weight gain, with some "atypical" antipsychotics causing more extreme weight gain, as well as glucose and lipid abnormalities, or even diabetes hence enhancing risk of cardiovascular disorders. Antipsychotics are also reported to cause sexual dysfunction and other undesired effects (Moncrieff, et al., "Antipsychotic Maintenance Treatment: Time to Rethink? PLOS Medicine, August 4, 2015, 1-7). These treatment-emergent side effects of antipsychotic medications are often a limiting factor for their long-term use, despite the fact that antipsychotics have been shown to reduce risk of relapse.

In some embodiments, a relapse, as used herein, refers to a psychiatric hospitalization of the patient, i.e., either an involuntary or voluntary admission to a psychiatric hospital for decomposition of the patient's schizophrenia symptoms.

In some embodiments, a relapse, as used herein, refers to an increase in the level of care (e.g., from out-patient to in-patient care).

In some embodiments, a relapse, as used herein, refers to the patient having suicidal ideations.

In some embodiments, a relapse, as used herein, refers to the patient having homicidal ideations.

In some embodiments, a relapse, as used herein, refers to the patient inflicting the deliberate self-injury of herself or himself.

In some embodiments, a relapse, as used herein, is the patient manifesting aggressiveness.

In some embodiments, a relapse is the patient manifesting aggressive behavior towards others.

In some embodiments, a relapse, as used herein, refers to the patient not adequately caring for herself or himself. For example, by not eating or by not washing/cleaning herself/himself.

In some embodiments, a relapse, as used herein, is the patient manifesting agitation.

In some embodiments, a relapse, as used herein, is indicated by an increase in PANSS total score.

In some embodiments, a relapse, as used herein, is indicated by about a 20% increase in PANSS total score in the patient in two consecutive assessments.

In some embodiments, a relapse, as used herein, is indicated by about a 20% increase in PANSS total score in the patient in two consecutive assessments, where the patient scored greater than 40 initially.

In some embodiments, a relapse, as used herein, is indicated by about a 20% increase in PANSS total score in the patient in two consecutive assessments, where the patient scored greater than 50 initially.

In some embodiments, a relapse, as used herein, is indicated by about a 25% increase in PANSS total score in the patient in two consecutive assessments.

In some embodiments, a relapse, as used herein, is indicated by about a 25% increase in PANSS total score in the patient in two consecutive assessments, where the patient scored greater than 40 initially.

In some embodiments, a relapse, as used herein, is indicated by about a 25% increase in PANSS total score in the patient in two consecutive assessments, where the patient scored greater than 50 initially.

In some embodiments, a relapse, as used herein, is indicated by about a 30% increase in PANSS total score in the patient in two consecutive assessments.

In some embodiments, a relapse, as used herein, is indicated by a about 30% increase in PANSS total score in the patient in two consecutive assessments, where the patient scored greater than 40 initially.

In some embodiments, a relapse, as used herein, is indicated by a about 30% increase in PANSS total score in the patient in two consecutive assessments, where the patient scored greater than 50 initially.

In some embodiments, a relapse, as used herein, is indicated by about a 35% increase in PANSS total score in the patient in two consecutive assessments.

In some embodiments, a relapse, as used herein, is indicated by about a 35% increase in PANSS total score in the patient in two consecutive assessments, where the patient scored greater than 40 initially.

In some embodiments, a relapse, as used herein, is indicated by about a 35% increase in PANSS total score in the patient in two consecutive assessments, where the patient scored greater than 50 initially.

In some embodiments, a relapse, as used herein, is indicated by an increase of about 5 or more points in PANSS total score in the patient in two consecutive assessments.

In some embodiments, a relapse, as used herein, is indicated by an increase of about 5 or more points in PANSS total score in the patient in two consecutive assessments, where the patient baseline PANSS total score was less than or equal to 40.

In some embodiments, a relapse, as used herein, is indicated by an increase of 5 or more points in PANSS total score in the patient in two consecutive assessments.

In some embodiments, a relapse, as used herein, is indicated by an increase of 5 or more points in PANSS total score in the patient in two consecutive assessments, where the patient baseline PANSS total score was less than or equal to 40.

In some embodiments, a relapse, as used herein, is indicated by an increase of about 10 or more points in PANSS total score in the patient in two consecutive assessments.

In some embodiments, a relapse, as used herein, is indicated by an increase of about 10 or more points in PANSS total score in the patient in two consecutive assessments, where the patient baseline PANSS total score was less than or equal to 40.

In some embodiments, a relapse, as used herein, is indicated by an increase of 10 or more points in PANSS total score in the patient in two consecutive assessments.

In some embodiments, a relapse, as used herein, is indicated by an increase of 10 or more points in PANSS total score in the patient in two consecutive assessments, where the patient baseline PANSS total score was less than or equal to 40.

In some embodiments, a relapse, as used herein, is indicated by an increase of about 12 or more points in PANSS total score in the patient in two consecutive assessments.

In some embodiments, a relapse, as used herein, is indicated by an increase of about 12 or more points in PANSS total score in the patient in two consecutive assessments, where the patient baseline PANSS total score was less than or equal to 40.

In some embodiments, a relapse, as used herein, is indicated by an increase of 12 or more points in PANSS total score in the patient in two consecutive assessments.

In some embodiments, a relapse, as used herein, is indicated by an increase of 12 or more points in PANSS total score in the patient in two consecutive assessments, where the patient baseline PANSS total score was less than or equal to 40.

In some embodiments, a relapse, as used herein, is indicated by an increase of about 15 or more points in PANSS total score in the patient in two consecutive assessments.

In some embodiments, a relapse, as used herein, is indicated by an increase of about 15 or more points in PANSS total score in the patient in two consecutive assessments, where the patient baseline PANSS total score was less than or equal to 40.

In some embodiments, a relapse, as used herein, is indicated by an increase of 15 or more points in PANSS total score in the patient in two consecutive assessments.

In some embodiments, a relapse, as used herein, is indicated by an increase of 15 or more points in PANSS total score in the patient in two consecutive assessments, where the patient baseline PANSS total score was less than or equal to 40.

In some embodiments, a relapse, as used herein, is indicated by an increase of about 20 or more points in PANSS total score in the patient in two consecutive assessments.

In some embodiments, a relapse, as used herein, is indicated by an increase of about 20 or more points in PANSS total score in the patient in two consecutive assessments, where the patient baseline PANSS total score was less than or equal to 40.

In some embodiments, a relapse, as used herein, is indicated by an increase of 20 or more points in PANSS total score in the patient in two consecutive assessments.

In some embodiments, a relapse, as used herein, is indicated by an increase of 20 or more points in PANSS total score in the patient in two consecutive assessments, where the patient baseline PANSS total score was less than or equal to 40.

In some embodiments, a relapse, as used herein, is indicated by an increase of about 25 or more points in PANSS total score in the patient in two consecutive assessments.

In some embodiments, a relapse, as used herein, is indicated by an increase of about 25 or more points in PANSS total score in the patient in two consecutive assessments, where the patient baseline PANSS total score was less than or equal to 40.

In some embodiments, a relapse, as used herein, is indicated by an increase of 25 or more points in PANSS total score in the patient in two consecutive assessments.

In some embodiments, a relapse, as used herein, is indicated by an increase of 25 or more points in PANSS total score in the patient in two consecutive assessments, where the patient baseline PANSS total score was less than or equal to 40.

In some embodiments, a relapse, as used herein, is indicated by an increase in the schizophrenia patient's PANSS positive subscore in 2 successive visits, wherein the visits are 1 day apart, 1 week apart, 1 month apart, or any duration in between.

In some embodiments, for any of the methods disclosed herein, the schizophrenia patient has not relapsed if the schizophrenia patient's PANSS positive subscore is within four 4 points (absolute difference) in 2 out of 3 visits, wherein the visits are 1 day apart, 1 week apart, 1 month apart, or any duration in between.

In some embodiments, for any of the methods disclosed herein, a relapse refers to an increase in positive symptoms in the schizophrenia patient as determined by any of the methods of assessing the same know to the skilled person in the art.

In some embodiments, for any of the methods disclosed herein, a relapse refers to an increase in positive symptoms in the schizophrenia patient as determined by the schizophrenia patient's PANSS positive subscore.

In some embodiments, for any of the methods disclosed herein, a relapse refers to an increase of 4 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 5 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 6 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 7 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 8 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 9 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 10 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 11 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 12 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 13 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 14 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 15 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 16 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 17 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 18 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 19 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 20 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 21 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 22 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 23 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 24 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 25 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 26 points or more in the schizophrenia patient's PANSS positive subscore two successive visits, wherein the visits are 1 day apart, 1 week apart, 1 month apart, or any duration in between.

In some embodiments, for any of the methods disclosed herein, a relapse refers to an increase of 27 points or more in the schizophrenia patient's PANSS positive subscore two successive visits, wherein the visits are 1 day apart, 1 week apart, 1 month apart, or any duration in between.

In some embodiments, a relapse, as used herein, is indicated by a patient being rated a 6 (severely ill) or a 7 (among the most extremely ill patients) on the CGI-S.

In some embodiments, a relapse, as used herein, is indicated by a patient being rated a 6 (clinically much worse compared to baseline visit) or a 7 (clinically very much worse compared to the baseline visit) on the CGI-I.

In some embodiments, for any of the methods disclosed herein, a relapse refers to a patient who is terminated early during treatment (or a study trial) due to worsening of psychosis or an adverse event or generalized symptom in Table 1.

**Table 1. Terms Defining Relapse**

| **Adverse Event Verbatim Examples** | **Generalized Term** |
|---|---|
| Worsening of paranoid schizophrenia with symptom of agitation | Agitation |
| Completed suicide | Completed suicide |
| Hallucination increase | Hallucination |
| Worsening of mental status | Mental Impairment |
| Worsening of psychosis | Psychotic disorder |
| Increase psychotic symptoms | Psychotic symptom |
| Agitation and aggressivity inside schizophrenia | Schizophrenia |
| Schizophrenia worsening | Schizophrenia |
| Exacerbation of schizophrenia | Schizophrenia |
| Schizophrenia relapse | Schizophrenia |
| Symptom recurrence | Reoccurrence of positive symptoms |

### METHODS OF PREVENTING RELAPSE IN A SCHIZOPHRENIA PATIENT

In one aspect, the present disclosure relates to a method of preventing relapse in a schizophrenia patient that includes administering a therapeutically effective amount of Roluperidone to the schizophrenia patient.

In some embodiments, the therapeutically effective amount of Roluperidone is administered to the schizophrenia patient once or twice a day.

In some embodiments, the therapeutically effective amount of Roluperidone is administered to the schizophrenia patient once a day.

In some embodiments, the therapeutically effective amount of Roluperidone is administered to the schizophrenia patient twice a day.

In some embodiments, the therapeutically effective amount of Roluperidone is administered orally to the schizophrenia patient.

In some embodiments, the therapeutically effective amount of Roluperidone is between about 1 mg to about 100 mg, about 4 mg to about 96 mg, about 5 mg to about 90 mg, about 6 mg to about 85 mg, about 16 mg to about 80 mg, about 25 mg and about 75 mg, or about 30 mg to about 70 mg.

In some embodiments, the therapeutically effective amount of Roluperidone is between 1 mg to 100 mg, 4 mg to 96 mg, 5 mg to 90 mg, 6 mg to 85 mg, 16 mg to 80 mg, 25 mg and 75 mg, or 30 mg to 70 mg.

In some embodiments, the therapeutically effective amount of Roluperidone is about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, about 78 mg, about 79 mg, about 80 mg, about 81 mg, about 82 mg, about 83 mg, about 84 mg, about 85 mg, about 86 mg, about 87 mg, about 88 mg, about 89 mg, about 90 mg, about 91 mg, about 92 mg, about 93 mg, about 94 mg, about 95 mg, about 96 mg, about 97 mg, about 98 mg, about 99 mg, or about 100 mg.

In some embodiments, the therapeutically effective amount of Roluperidone is 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, or 100 mg.

In some embodiments, the therapeutically effective amount of Roluperidone is about 16 mg, about 24 mg, about 32 mg, about 40 mg, about 48 mg, about 56 mg, about 64 mg, about 72 mg, about 80 mg, about 88 mg, or about 96 mg.

In some embodiments, the therapeutically effective amount of Roluperidone is 16 mg, 24 mg, 32 mg, 40 mg, 48 mg, 56 mg, 64 mg, 72 mg, 80 mg, 88 mg, or 96 mg.

In some embodiments, the therapeutically effective amount of Roluperidone is 16 mg.

In some embodiments, the therapeutically effective amount of Roluperidone is about 16 mg.

In some embodiments, the therapeutically effective amount of Roluperidone is 24 mg.

In some embodiments, the therapeutically effective amount of Roluperidone is about 24 mg.

In some embodiments, the therapeutically effective amount of Roluperidone is 32 mg.

In some embodiments, the therapeutically effective amount of Roluperidone is about 32 mg.

In some embodiments, the therapeutically effective amount of Roluperidone is 40 mg.

In some embodiments, the therapeutically effective amount of Roluperidone is about 40 mg.

In some embodiments, the therapeutically effective amount of Roluperidone is 48 mg.

In some embodiments, the therapeutically effective amount of Roluperidone is about 48 mg.

In some embodiments, the therapeutically effective amount of Roluperidone is 56 mg.

In some embodiments, the therapeutically effective amount of Roluperidone is about 56 mg.

In some embodiments, the therapeutically effective amount of Roluperidone is 64 mg.

In some embodiments, the therapeutically effective amount of Roluperidone is about 64 mg.

In some embodiments, the schizophrenia patient does not manifest any behavior which puts the patient, or those around the patient, at risk of bodily harm.

In some embodiments, the schizophrenia patient has low levels of symptoms related to agitation, impulse control, hostility, suspiciousness, or uncooperativeness.

In some embodiments, the schizophrenia patient has low levels of symptoms related to agitation.

In some embodiments, the schizophrenia patient has low levels of symptoms related to impulse control.

In some embodiments, the schizophrenia patient has low levels of symptoms related to hostility.

In some embodiments, the schizophrenia patient has low levels of symptoms related to suspiciousness.

In some embodiments, the schizophrenia patient has low levels of symptoms related to uncooperativeness.

In some embodiments, the schizophrenia patient does not experience a high level of depression or anxiety.

In some embodiments, the schizophrenia patient has positive symptoms that are stable before starting treatment with Roluperidone.

In some embodiments, the schizophrenia patient has positive symptoms that are stable for 1 to 6 months before starting treatment with Roluperidone.

In some embodiments, the schizophrenia patient has positive symptoms that are stable for 3 to 6 months before starting treatment with Roluperidone.

In some embodiments, the schizophrenia patient has positive symptoms that are stable for at least 1 day before starting treatment with Roluperidone.

In some embodiments, the schizophrenia patient has positive symptoms that are stable for at least 1 week before starting treatment with Roluperidone.

In some embodiments, the schizophrenia patient has positive symptoms that are stable for at least 2 weeks before starting treatment with Roluperidone.

In some embodiments, the schizophrenia patient has positive symptoms that are stable for at least 3 weeks before starting treatment with Roluperidone.

In some embodiments, the schizophrenia patient has positive symptoms that are stable for at least 1 month before starting treatment with Roluperidone.

In some embodiments, the schizophrenia patient has positive symptoms that are stable for at least 2 months before starting treatment with Roluperidone.

In some embodiments, the schizophrenia patient has positive symptoms that are stable for at least 3 months before starting treatment with Roluperidone.

In some embodiments, the schizophrenia patient has positive symptoms that are stable for at least 4 months before starting treatment with Roluperidone.

In some embodiments, the schizophrenia patient has positive symptoms that are stable for at least 5 months before starting treatment with Roluperidone.

In some embodiments, the schizophrenia patient has positive symptoms that are stable for at least 6 months before starting treatment with Roluperidone.

In some embodiments, the schizophrenia patient has positive symptoms that are stable for at least 7 months before starting treatment with Roluperidone.

In some embodiments, the schizophrenia patient has positive symptoms that are stable for at least 8 months before starting treatment with Roluperidone.

In some embodiments, the schizophrenia patient has positive symptoms that are stable for at least 9 months before starting treatment with Roluperidone.

In some embodiments, the schizophrenia patient has positive symptoms that are stable for at least 10 months before starting treatment with Roluperidone.

In some embodiments, the schizophrenia patient has positive symptoms that are stable for at least 11 months before starting treatment with Roluperidone.

In some embodiments, the schizophrenia patient has positive symptoms that are stable for at least 12 months before starting treatment with Roluperidone.

In some embodiments, the schizophrenia patient does not have positive symptoms before starting treatment with Roluperidone.

In some embodiments, the schizophrenia patient does not have positive symptoms for 1 to 6 months before starting treatment with Roluperidone.

In some embodiments, the schizophrenia patient does not have positive symptoms for 3 to 6 months before starting treatment with Roluperidone.

In some embodiments, the schizophrenia patient does not have positive symptoms for at least 1 day before starting treatment with Roluperidone.

In some embodiments, the schizophrenia patient does not have positive symptoms for at least 1 week before starting treatment with Roluperidone.

In some embodiments, the schizophrenia patient does not have positive symptoms for at least 2 weeks before starting treatment with Roluperidone.

In some embodiments, the schizophrenia patient does not have positive symptoms for at least 3 weeks before starting treatment with Roluperidone.

In some embodiments, the schizophrenia patient does not have positive symptoms for at least 1 month before starting treatment with Roluperidone.

In some embodiments, the schizophrenia patient does not have positive symptoms for at least 2 months before starting treatment with Roluperidone.

In some embodiments, the schizophrenia patient does not have positive symptoms for at least 3 months before starting treatment with Roluperidone.

In some embodiments, the schizophrenia patient does not have positive symptoms for at least 4 months before starting treatment with Roluperidone.

In some embodiments, the schizophrenia patient does not have positive symptoms for at least 5 months before starting treatment with Roluperidone.

In some embodiments, the schizophrenia patient does not have positive symptoms for at least 6 months before starting treatment with Roluperidone.

In some embodiments, the schizophrenia patient does not have positive symptoms for at least 7 months before starting treatment with Roluperidone.

In some embodiments, the schizophrenia patient does not have positive symptoms for at least 8 months before starting treatment with Roluperidone.

In some embodiments, the schizophrenia patient does not have positive symptoms for at least 9 months before starting treatment with Roluperidone.

In some embodiments, the schizophrenia patient does not have positive symptoms for at least 10 months before starting treatment with Roluperidone.

In some embodiments, the schizophrenia patient does not have positive symptoms for at least 11 months before starting treatment with Roluperidone.

In some embodiments, the schizophrenia patient does not have positive symptoms for at least 12 months before starting treatment with Roluperidone.

In some embodiments, the schizophrenia patient does not have positive symptoms if the schizophrenia patient has a PANSS positive subscore of 15 or less.

In some embodiments, the schizophrenia patient does not have positive symptoms if the schizophrenia patient has a PANSS positive subscore of 14 or less.

In some embodiments, the schizophrenia patient does not have positive symptoms if the schizophrenia patient has a PANSS positive subscore of 13 or less.

In some embodiments, the schizophrenia patient does not have positive symptoms if the schizophrenia patient has a PANSS positive subscore of 12 or less.

In some embodiments, the schizophrenia patient does not have positive symptoms if the schizophrenia patient has a PANSS positive subscore of 11 or less.

In some embodiments, the schizophrenia patient does not have positive symptoms if the schizophrenia patient has a PANSS positive subscore of 10 or less.

In some embodiments, the schizophrenia patient does not have positive symptoms if the schizophrenia patient has a PANSS positive subscore of 9 or less.

In some embodiments, the schizophrenia patient does not have positive symptoms if the schizophrenia patient has a PANSS positive subscore of or 8 or less.

In some embodiments, the schizophrenia patient does not have positive symptoms if the schizophrenia patient has a PANSS positive subscore of 7.

In some embodiments, the schizophrenia patient has negative symptoms that are moderate to severe. In some embodiments, the schizophrenia patient has negative symptoms that are moderate. In some embodiments, the schizophrenia patient has negative symptoms that are severe.

In some embodiments, the schizophrenia patient's PANSS negative subscore is in between 20-25, in between 25-30, in between 30-35, in between 35-40, in between 40-45, or in between 45-49.

In some embodiments, the schizophrenia patient's PANSS negative subscore is in between 20-29, in between 30-39, or in between 40-49.

In some embodiments, the schizophrenia patient's PANSS negative subscore is in between 20-35 or in between 35-49.

In some embodiments, the schizophrenia patient's PANSS negative subscore is greater than 20.

In some embodiments, the schizophrenia patient's PANSS negative subscore is greater than 21.

In some embodiments, the schizophrenia patient's PANSS negative subscore is greater than 22.

In some embodiments, the schizophrenia patient's PANSS negative subscore is greater than 23.

In some embodiments, the schizophrenia patient's PANSS negative subscore is greater than 24.

In some embodiments, the schizophrenia patient's PANSS negative subscore is greater than 25.

In some embodiments, the schizophrenia patient's PANSS negative subscore is greater than 26.

In some embodiments, the schizophrenia patient's PANSS negative subscore is greater than 27.

In some embodiments, the schizophrenia patient's PANSS negative subscore is greater than 28.

In some embodiments, the schizophrenia patient's PANSS negative subscore is greater than 29.

In some embodiments, the schizophrenia patient's PANSS negative subscore is greater than 30.

In some embodiments, the schizophrenia patient's PANSS negative subscore is greater than 31.

In some embodiments, the schizophrenia patient's PANSS negative subscore is greater than 32.

In some embodiments, the schizophrenia patient's PANSS negative subscore is greater than 33.

In some embodiments, the schizophrenia patient's PANSS negative subscore is greater than 34.

In some embodiments, the schizophrenia patient's PANSS negative subscore is greater than 35.

In some embodiments, the schizophrenia patient's PANSS negative subscore is greater than 36.

In some embodiments, the schizophrenia patient's PANSS negative subscore is greater than 37.

In some embodiments, the schizophrenia patient's PANSS negative subscore is greater than 38.

In some embodiments, the schizophrenia patient's PANSS negative subscore is greater than 39.

In some embodiments, the schizophrenia patient's PANSS negative subscore is greater than 40.

In some embodiments, the schizophrenia patient's PANSS negative subscore is greater than 41.

In some embodiments, the schizophrenia patient's PANSS negative subscore is greater than 42.

In some embodiments, the schizophrenia patient's PANSS negative subscore is greater than 43.

In some embodiments, the schizophrenia patient's PANSS negative subscore is greater than 44.

In some embodiments, the schizophrenia patient's PANSS negative subscore is greater than 45.

In some embodiments, the schizophrenia patient's PANSS negative subscore is greater than 46.

In some embodiments, the schizophrenia patient's PANSS negative subscore is greater than 47.

In some embodiments, the schizophrenia patient's PANSS negative subscore is greater than 48.

In some embodiments, the schizophrenia patient's PANSS negative subscore is 49.

In some embodiments, the schizophrenia patient's negative symptoms are primary negative symptoms.

In some embodiments, the schizophrenia patient's negative symptoms are not secondary negative symptoms.

In some embodiments, the schizophrenia patient's negative symptoms are stable before starting treatment with Roluperidone.

In some embodiments, the schizophrenia patient's negative symptoms are stable for 1 to 6 months before starting treatment with Roluperidone.

In some embodiments, the schizophrenia patient's negative symptoms are stable for 3 to 6 months before starting treatment with Roluperidone.

In some embodiments, the schizophrenia patient's negative symptoms are stable for at least 1 day before starting treatment with Roluperidone.

In some embodiments, the schizophrenia patient's negative symptoms are stable for at least 1 week before starting treatment with Roluperidone.

In some embodiments, the schizophrenia patient's negative symptoms are stable for at least 2 weeks before starting treatment with Roluperidone.

In some embodiments, the schizophrenia patient's negative symptoms are stable for at least 3 weeks before starting treatment with Roluperidone.

In some embodiments, the schizophrenia patient's negative symptoms are stable for at least 1 month before starting treatment with Roluperidone.

In some embodiments, the schizophrenia patient's negative symptoms are stable for at least 2 months before starting treatment with Roluperidone.

In some embodiments, the schizophrenia patient's negative symptoms are stable for at least 3 months before starting treatment with Roluperidone.

In some embodiments, the schizophrenia patient's negative symptoms are stable for at least 4 months before starting treatment with Roluperidone.

In some embodiments, the schizophrenia patient's negative symptoms are stable for at least 5 months before starting treatment with Roluperidone.

In some embodiments, the schizophrenia patient's negative symptoms are stable for at least 6 months before starting treatment with Roluperidone.

In some embodiments, the schizophrenia patient's negative symptoms are stable for at least 7 months before starting treatment with Roluperidone.

In some embodiments, the schizophrenia patient's negative symptoms are stable for at least 8 months before starting treatment with Roluperidone.

In some embodiments, the schizophrenia patient's negative symptoms are stable for at least 9 months before starting treatment with Roluperidone.

In some embodiments, the schizophrenia patient's negative symptoms are stable for at least 10 months before starting treatment with Roluperidone.

In some embodiments, the schizophrenia patient's negative symptoms are stable for at least 11 months before starting treatment with Roluperidone.

In some embodiments, the schizophrenia patient's negative symptoms are stable for at least 12 months before starting treatment with Roluperidone.

In some embodiments, the schizophrenia patient was previously administered an antipsychotic.

In some embodiments, the administration of the antipsychotic to the schizophrenia patient was discontinued at the same time that the schizophrenia patient was administered Roluperidone.

In some embodiments, the administration of the antipsychotic to the schizophrenia patient was discontinued before the schizophrenia patient was administered Roluperidone.

In some embodiments, the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months, or at least 12 months before the schizophrenia patient was administered Roluperidone.

In some embodiments, the administration of the antipsychotic to the schizophrenia patient was discontinued at least 1 day before the schizophrenia patient was administered Roluperidone.

In some embodiments, the administration of the antipsychotic to the schizophrenia patient was discontinued at least 2 days before the schizophrenia patient was administered Roluperidone.

In some embodiments, the administration of the antipsychotic to the schizophrenia patient was discontinued at least 3 days before the schizophrenia patient was administered Roluperidone.

In some embodiments, the administration of the antipsychotic to the schizophrenia patient was discontinued at least 4 days before the schizophrenia patient was administered Roluperidone.

In some embodiments, the administration of the antipsychotic to the schizophrenia patient was discontinued at least 5 days before the schizophrenia patient was administered Roluperidone.

In some embodiments, the administration of the antipsychotic to the schizophrenia patient was discontinued at least 6 days before the schizophrenia patient was administered Roluperidone.

In some embodiments, the administration of the antipsychotic to the schizophrenia patient was discontinued at least 1 week before the schizophrenia patient was administered Roluperidone.

In some embodiments, the administration of the antipsychotic to the schizophrenia patient was discontinued at least 2 weeks before the schizophrenia patient was administered Roluperidone.

In some embodiments, the administration of the antipsychotic to the schizophrenia patient was discontinued at least 3 weeks before the schizophrenia patient was administered Roluperidone.

In some embodiments, the administration of the antipsychotic to the schizophrenia patient was discontinued at least 1 month before the schizophrenia patient was administered Roluperidone.

In some embodiments, the administration of the antipsychotic to the schizophrenia patient was discontinued at least 2 months before the schizophrenia patient was administered Roluperidone.

In some embodiments, the administration of the antipsychotic to the schizophrenia patient was discontinued at least 3 months before the schizophrenia patient was administered Roluperidone.

In some embodiments, the administration of the antipsychotic to the schizophrenia patient was discontinued at least 4 months before the schizophrenia patient was administered Roluperidone.

In some embodiments, the administration of the antipsychotic to the schizophrenia patient was discontinued at least 5 months before the schizophrenia patient was administered Roluperidone.

In some embodiments, the administration of the antipsychotic to the schizophrenia patient was discontinued at least 6 months before the schizophrenia patient was administered Roluperidone.

In some embodiments, the administration of the antipsychotic to the schizophrenia patient was discontinued at least 7 months before the schizophrenia patient was administered Roluperidone.

In some embodiments, the administration of the antipsychotic to the schizophrenia patient was discontinued at least 8 months before the schizophrenia patient was administered Roluperidone.

In some embodiments, the administration of the antipsychotic to the schizophrenia patient was discontinued at least 9 months before the schizophrenia patient was administered Roluperidone.

In some embodiments, the administration of the antipsychotic to the schizophrenia patient was discontinued at least 10 months before the schizophrenia patient was administered Roluperidone.

In some embodiments, the administration of the antipsychotic to the schizophrenia patient was discontinued at least 11 months before the schizophrenia patient was administered Roluperidone.

In some embodiments, the administration of the antipsychotic to the schizophrenia patient was discontinued at least 12 months before the schizophrenia patient was administered Roluperidone.

In some embodiments, the schizophrenia patient is also currently being administered an antipsychotic when starting treatment with Roluperidone to prevent relapse.

In some embodiments, the schizophrenia patient that is also currently being administered an antipsychotic when starting treatment with Roluperidone to prevent relapse is tapered off of the antipsychotic over a period of time. For example, the tapering off of the antipsychotic occurs over 1 week, 2 weeks, 3 weeks, 1 month, 2 months, 3 months, 4 months, 5 months, 6 months, 7 months, 8 months, 9 months, 10 months, 11 months, 12 months, or more, until the schizophrenia patient is only being treated with Roluperidone.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms, and
   having moderate to severe negative symptoms that are stable; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable, and
   not manifesting any behavior which puts the patient, or those around the patient, at risk of bodily harm; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable,
   not manifesting any behavior which puts the patient, or those around the patient, at risk of bodily harm, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable,
   not manifesting any behavior which puts the patient, or those around the patient, at risk of bodily harm, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having stable positive symptoms,
   having moderate to severe negative symptoms that are stable,
   not manifesting any behavior which puts the patient, or those around the patient, at risk of bodily harm,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months, and
   having moderate to severe negative symptoms that are stable for 3 to 6 months; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months, and
   not manifesting any behavior which puts the patient, or those around the patient, at risk of bodily harm; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months, and not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months,
   not manifesting any behavior which puts the patient, or those around the patient, at risk of bodily harm, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months,
   not manifesting any behavior which puts the patient, or those around the patient, at risk of bodily harm, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   having positive symptoms that are stable for 3 to 6 months,
   having moderate to severe negative symptoms that are stable for 3 to 6 months,
   not manifesting any behavior which puts the patient, or those around the patient, at risk of bodily harm,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by
   the schizophrenia patient:
   not having positive symptoms, and
   having moderate to severe negative symptoms that are stable; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable, and
   not manifesting any behavior which puts the patient, or those around the patient, at risk of bodily harm; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable,
   not manifesting any behavior which puts the patient, or those around the patient, at risk of bodily harm, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable,
   not manifesting any behavior which puts the patient, or those around the patient, at risk of bodily harm, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms,
   having moderate to severe negative symptoms that are stable,
   not manifesting any behavior which puts the patient, or those around the patient, at risk of bodily harm,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months, and
   having moderate to severe negative symptoms that are stable for 3 to 6 months; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months, and
   not manifesting any behavior which puts the patient, or those around the patient, at risk of bodily harm; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months, and not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months, having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months,
   not manifesting any behavior which puts the patient, or those around the patient, at risk of bodily harm, and
   not experiencing a high level of depression or anxiety; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months,
   not manifesting any behavior which puts the patient, or those around the patient, at risk of bodily harm, and
   having low levels of symptoms related to agitation, impulse control, hostility, suspiciousness, or uncooperativeness; and
(b) administering a therapeutically effective amount of Roluperidone to the schizophrenia patient.

In another aspect, the present disclosure relates to a method of selecting a schizophrenia patient and preventing relapse in the schizophrenia patient, wherein the method comprises:
(a) selecting the schizophrenia patient as having a form of schizophrenia characterized by the schizophrenia patient:
   not having positive symptoms for 3 to 6 months,
   having moderate to severe negative symptoms that are stable for 3 to 6 months,
   not manifesting any behavior which puts the patient, or those around the patient, at risk of bodily harm,
   having low levels of symptoms related to agitation, impulse control, hostility, suspiciousness, or uncooperativeness, and
   not experiencing a high level of depression or anxiety; and
(b) administering a therapeutically effective amount of Roluperidone to the schizophrenia patient.

In some embodiments, for any of the methods disclosed herein, the schizophrenia patient has stable positive symptoms over the last 1, 2, 3, 4, 5, or 6 months according to his or her treating psychiatrist and based on documentation in the clinical chart.

In some embodiments, for any of the methods disclosed herein, the schizophrenia patient does not have positive symptoms over the last 1, 2, 3, 4, 5, or 6 months according to his or her treating psychiatrist and based on documentation in the clinical chart.

In some embodiments, for any of the methods disclosed herein, the schizophrenia patient has stable negative symptoms over the last 1, 2, 3, 4, 5, or 6 months according to his or her treating psychiatrist and based on documentation in the clinical chart.

In some embodiments, for any of the methods disclosed herein, the schizophrenia patient has stable positive and negative symptoms over the last 1, 2, 3, 4, 5, or 6 months according to his or her treating psychiatrist and based on documentation in the clinical chart.

In some embodiments, for any of the methods disclosed herein, the schizophrenia patient has a PANSS negative subscore of greater than 20, i.e., the original PANSS scale [sum of N1+N2+N3+N4+N5+N6+N7]) at their first visit (i.e., screening).

In some embodiments, for any of the methods disclosed herein, the schizophrenia patient's PANSS negative subscore is within four 4 points (absolute difference) in 2 successive visits, wherein the visits are 1 day apart, 1 week apart, 1 month apart, or any duration in between.

In some embodiments, for any of the methods disclosed herein, the schizophrenia patient's PANSS negative subscore is within four 4 points (absolute difference) in 2 out of 3 visits, wherein the visits are 1 day apart, 1 week apart, 1 month apart, or any duration in between.

In some embodiments, for any of the methods disclosed herein, the schizophrenia patient's PANSS negative subscore is within four 4 points (absolute difference) in 2 out of 4 successive visits, wherein the visits are 1 day apart, 1 week apart, 1 month apart, or any duration in between.

In some embodiments, for any of the methods disclosed herein, the schizophrenia patient's PANSS positive subscore is within four 4 points (absolute difference) in 2 successive visits, wherein the visits are 1 day apart, 1 week apart, 1 month apart, or any duration in between.

In some embodiments, for any of the methods disclosed herein, the schizophrenia patient's PANSS positive subscore is within four 4 points (absolute difference) in 2 out of 3 visits, wherein the visits are 1 day apart, 1 week apart, 1 month apart, or any duration in between.

In some embodiments, for any of the methods disclosed herein, the schizophrenia patient's PANSS positive subscore is within four 4 points (absolute difference) in 2 out of 4 successive visits, wherein the visits are 1 day apart, 1 week apart, 1 month apart, or any duration in between.

In some embodiments, for any of the methods disclosed herein, the schizophrenia patient is an extensive metabolizer for cytochrome P450 (CYP2D6), defined as a subject that has at least one functional allele (e.g., *1 or *2), as determined by study-specific genotyping test before the first drug dose is administered.

In some embodiments, for any of the methods disclosed herein, the schizophrenia patient is not diagnosed with or suffering from major depressive disorder, bipolar disorder, panic disorder, obsessive compulsive disorder, or an intellectual disability (e.g., an intellectual developmental disorder diagnosed by age 14).

In some embodiments, for any of the methods disclosed herein, the schizophrenia patient does not have a PANSS item score of greater than 4 on P4 (excitement/hyperactivity), P6 (suspiciousness/persecution), P7 (hostility), G8 (uncooperativeness), G14 (poor impulse control).

In some embodiments, for any of the methods disclosed herein, the schizophrenia patient does not have a Calgary Depression Scale for Schizophrenia (CDSS) total score of greater than 6.

In some embodiments, for any of the methods disclosed herein, the schizophrenia patient does not have a score of ≥ 2 on any 2 items 1, 2, or 3, or a score of ≥ 3 on item 4 of the Barnes Akathisia Rating Scale (BARS).

### EXAMPLE 1 - Summaries of Studies Nos. 1 and 2 - Overview, Endpoints, and Efficacy Assessments

Results from Study Nos. 1 ("MIN-101C03") and 2 ("MIN-101C07") provide substantial clinical evidence of the effectiveness of the 64 mg Roluperidone dose administered once daily as monotherapy for the treatment of patients with moderate to severe negative symptoms and stable positive symptoms of schizophrenia. An overview of these studies is provided below in Table 2. They also provide, for the first time, evidence that Roluperidone is effective in prevent relapse in a schizophrenia patient (vide infra).

The primary integrated analysis of the efficacy of Roluperidone compared with placebo for the treatment of negative symptoms of schizophrenia is based on the change from Baseline in the PANSS Marder's negative symptoms factor score (in this document referred to as NSFS) to Week 12. Efficacy is also supported by the analysis of change from Baseline in the PSP Total score and the CGI-S to Week 12. Overall, MIN-101C03 and MIN-101C07 used comparable endpoints to assess the efficacy of Roluperidone in patients with schizophrenia (Table 3).

**Table 2. Overview of the Clinical Studies Used for the Efficacy Evaluation of Roluperidone in Patients with Moderate to Severe Negative Symptoms of Schizophrenia**

| **Study Number** | **Patient Population** | **Main Objective and Study Design** | **Dosage Regimen Route of Administration Duration; Test Product(s)** | **Total Enrolled/Dosed Completed** | **Study Status** |
|---|---|---|---|---|---|
| 1 "MIN-101C03" | Male and female patients with schizophrenia, CYP2D6 NM and 18-60 years old | Multicenter, DB, randomized, parallel-group, placebo-controlled study to evaluate efficacy, tolerability, and safety of Roluperidone in patients with schizophrenia with moderate to severe negative symptoms and stable positive symptoms, followed by 24 weeks of OL extension | Multiple oral administration; Roluperidone MR formulation; placebo, 32, and 64 mg Roluperidone, QD | 244/244 | Completed |
| | | | | 142 completed DB period (12 weeks) | |
| | | | | 88 completed OL period (24 weeks) | |
| 2 "MIN-101C07" | Male and female patients with schizophrenia, CYP2D6 NM, and 18-55 years old | Multicenter, DB, randomized, parallel-group, placebo-controlled study to evaluate efficacy, tolerability, and safety of Roluperidone in patients with schizophrenia with moderate to severe negative symptoms and stable positive symptoms, followed by 40 weeks of OL extension | Multiple oral administration; Roluperidone GR01/B formulation; placebo, 32, and 64 mg Roluperidone, QD | 515/513 | Completed |
| | | | | 333 completed DB period (12 weeks) | |
| | | | | 202 completed OL period (40 weeks) | |

| | | | | | |
|---|---|---|---|---|---|
| CYP2D6 = cytochrome P450 2D6; DB = double-blind; NM = normal metabolizers; GR = gastro-resistant; MR = modified-release; OL = open label; QD = once a day. | | | | | |

**Table 3. Overview of the Key Endpoints Used in the Clinical Studies for the Efficacy Evaluation of Roluperidone in Patients with Schizophrenia**

| **Study Number** | **Primary** | **Secondary and Exploratory** | **Post-hoc** |
|---|---|---|---|
| MIN-101C03 | Change from Baseline in the PANSS negative factor score of the PSM to end of Week 12 | • Change from Baseline in the PSP Total and subscale score over 12 weeks | • Change in NSFS from Baseline to end of Week 12 |
| | | • Change from Baseline in the CGI-S score over 12 weeks | • Bar graph showing percent of patients achieving predefined improvements on NSFS |
| | | • The CGI-I score over 12 weeks | |
| | | • Change from Baseline in PANSS total and subscale scores over 12 weeks | |
| MIN-101C07 | Change in NSFS from Baseline to end of Week 12 | • Key Secondary: Change from Baseline in the PSP Total score over 12 weeks | • Bar graph showing percent of patients achieving predefined improvements on NSFS |
| | | • Change from Baseline in the CGI-S score over 12 weeks | |
| | | • The CGI-I score over 12 weeks | |
| | | • Change from Baseline in PANSS total and subscale scores over 12 weeks | |
| | | • Change from Baseline in PSP subscale scores over 12 weeks | |

| | | | |
|---|---|---|---|
| CGI-I = Clinical Global Impression - Improvement Scale; CGI-S = Clinical Global Impression - Severity Scale; NSFS = PANSS Marder's negative symptoms factor score; PANSS = Positive and Negative Syndrome Scale; PSM = Pentagonal Structure Model; PSP = Personal and Social Performance. | | | |

### Efficacy Assessments

The efficacy assessments described below were performed in both Studies MIN-101C03 and MIN-101C07. The results of other efficacy assessments that were performed only in the individual studies are summarized in Table 8 and Table 9, respectively.

### Endpoints Based on PANSS Items

The PANSS was designed to be used in patients with schizophrenia to measure the overall severity of schizophrenic symptoms. The scale has been validated in different cultures and languages and is reliable and acceptable to regulatory health authorities as a primary scale to determine efficacy of intervention in schizophrenia. The name refers to the 2 types of symptoms in schizophrenia, as defined by the American Psychiatric Association: positive symptoms, which refer to an excess or distortion of normal functions (e.g., hallucinations and delusions), and negative symptoms, which represent a diminution or loss of normal functions.

The patient is rated from 1 to 7 on 30 different symptoms based on the interview as well as reports of family members or primary care hospital workers. The original PANSS included 3 groups of symptoms and subscale scores Positive, Negative, and General Psychopathology. Since the inception of PANSS, investigators have proposed additional grouping (structures) of PANSS such as the Marder structure (Marder, S.R. et al. "Issues and perspectives in designing clinical trials for negative symptoms in schizophrenia: consensus statements," Schizophrenia Bulletin Open. 2020;1(1). doi:10.1093/schizbullopen/sgz001) and the White structure (White, L. et al. "Empirical assessment of the factorial structure of clinical symptoms in schizophrenia". Psychopathology. 1997;30263-274). Also, additional structures of negative symptoms assessed with the help of the PANSS items have been suggested, such as emotional expression and emotional experience subgrouping (Harvey, P. D., et al., "Effects of Roluperidone (MIN-101) on two dimensions of the negative symptoms factor score: reduced emotional experience and reduced emotional expression" Schizophrenia Res. 2020;215:352-356.)

The PANSS was assessed at the time points described in Table 4. Because the PANSS Marder's negative symptoms factor score (in this document referred to as NSFS) was considered to be the most appropriate measurement of negative symptoms of schizophrenia, the MIN-101C03 data were re-analyzed using the NSFS to ensure results from this study and MIN-101C07 are comparable and able to be integrated.

**Table 4. Timing of PANSS Assessments**

| | **DB Period** | **OL Period** |
|---|---|---|
| Study MIN-101C03 | **SCR, BL, Weeks 2, 4, 8, 12** | Week 12 +1-2 days, Weeks **18, 24,** 36, 37/EOS |
| Study MIN-101C07 | **SCR, BL, Weeks 2, 4, 8, 12** | Weeks 14, 16, **24,** 32, 40, 28, 52, 54/EOS |

| | | |
|---|---|---|
| BL = Baseline; DB = double-blind; EOS = end of study; OL = open-label; PANSS = Positive and Negative Syndrome Scale; SCR = Screening Note: Timepoints that were assessed in both studies are in bold. | | |

The PANSS-derived endpoints used in these studies and the integrated analyses are described in Table 5A and a summary of the individual PANSS items used by physicians and clinicians to measure the severity of the symptoms in a schizophrenia patient is provided in Table 5B. The scale is divided into three parts, a positive scale, which provides a "PANSS positive subscore", a negative scale, which provides a "PANSS negative subscore", and general psychopathology scale. The sum of these three parts provides a PANSS total score, which ranges from 30 to 210 (where higher scores indicate more severe symptoms). (See Kay, S.R., et al. "The positive and negative syndrome scale (PANSS) for schizophrenia," Schizophr Bulletin, 13(2), 261-276 (1987) and Gopal, S., et al. "Improvement of Negative Symptoms in Schizophrenia with Paliperidone Palmitate 1-Month and 3-Month Long-Acting Injectables: Results from a Phase 3 Non-Inferiority Study" Neuropsychiatric Disease and Treatment, , 681-690, DOI: 10.2147/NDT.S226296, both of which are incorporated by reference.)

**Table 5A. Efficacy Endpoints Based on PANSS Items**

| **Endpoint** | **PANSS Items** | **Range of Values** | **Analysis Included in:** | | |
|---|---|---|---|---|---|
| | | | **MIN-101C03** | **MIN-101C07** | **ISE** |
| NSFS | N1+N2+N3+N4+N6+ G7+G16 | 7 to 49 | X (post-hoc) | X | X |
| PANSS PSM | N1+N2+N3+N4+N6+G5+G7+G8 +G13+G14 | 10 to 70 | X | ND | ND |
| PANSS Total | N1+N2+N3+N4+N5+N6+N7+P1+ P2+P3+P4+P5+P6+P7+G1+G2+G 3+G4+G5+G6+G7+G8+G9+G10+ G11+G12+G13+G14+G15+G16 | 30 to 210 | X | X | X |
| PANSS Positive Symptoms Subscale | P1+P2+P3+P4+P5+P6+P7 | 7 to 49 | X | X | X |
| PANSS Negative Symptoms Subscale | N1+N2+N3+N4+N5+N6+N7 | 7 to 49 | X | X | X |
| PANSS General Psychopathology Subscale | G1+G2+G3+G4+G5+G6+G7+G8 +G9+G10+G11+G12+G13+G14+ G15+G16 | 16 to 112 | X | X | X |
| NSFS emotional experience^{a} | N2+N4+G16 | 3 to 21 | X | X | X |
| NSFS emotional expression^{a} | N1+N3+N6+G7 | 4 to 28 | X | X | X |
| Marder positive symptoms | P1+P3+P5+P6+N7+G1+G9+G12 | 8 to 56 | X | X | X |
| Marder disorganized thought | P2+N5+G5+G10+G11+G13+G15 | 7 to 49 | X | X | X |
| Marder uncontrolled hostility/ excitement | P4+P7+G8+G14 | 4 to 28 | X | X | X |
| Marder anxiety/ depression | G2+G3+G4+G6 | 4 to 28 | X | X | X |

| | | | | | |
|---|---|---|---|---|---|
| ND = not done; NSFS = PANSS Marder's negative symptoms factor score; PANSS = Positive and Negative Syndrome Scale; PSM = pentagonal structure model; ISE = Integrated Summary of Efficacy. ^{a} The NSFS underwent a large-scale factor analysis (Khan, A., et al., "Negative symptom dimensions of the positive and negative syndrome scale across geographical regions: implications for social, linguistic, and cultural consistency" Innov Clin Neurosci. 2017;14(11-12):30-40), and a 2-factor model reflected as reduced emotional experience and reduced expression were proposed and are referred to in this document as NSFS emotional experience and NSFS emotional expression, respectively. | | | | | |

**Table 5B. Individual PANSS Items**

| | | |
|---|---|---|
| PANSS Positive Items | P1 | Delusions |
| | P2 | Conceptual disorganization |
| | P3 | Hallucinatory behavior |
| | P4 | Excitement/hyperactivity |
| | P5 | Grandiosity |
| | P6 | Suspiciousness/persecution |
| | P7 | Hostility |
| | | |
| PANSS Negative Items | N1 | Blunted affect |
| | N2 | Emotional withdrawal |
| | N3 | Poor rapport |
| | N4 | Passive/apathetic social withdrawal |
| | N5 | Difficulty in abstract thinking |
| | N6 | Lack of spontaneity and flow of conversation |
| | N7 | Stereotyped thinkine |
| | | |
| PANSS General Items | G1 | Somatic concerns |
| | G2 | Anxiety |
| | G3 | Guilt feelings |
| | G4 | Tension |
| | G5 | Mannerisms and posturing |
| | G6 | Depression |
| | G7 | Motor retardation |
| | G8 | Uncooperativeness |
| | G9 | Unusual thought content |
| | G10 | Disorientation |
| | G11 | Poor attention |
| | G12 | Lack of judgement and insight |
| | G13 | Disturbance of volition |
| | G14 | Poor impulse control |
| | G15 | Preoccupation |
| | G16 | Active social avoidance |

### PSP

The PSP is a validated clinician-rated scale intended to reflect real-life situations that measures personal and social functioning in 4 domains: (a) socially useful activities, (b) personal and social relationships, (c) self-care, and (d) disturbing and aggressive behaviors. The score is based on the assessment of a patient's performance in the 4 domains. The PSP Total score is a single measurement of functioning ranging from 1 to 100, where a higher score represents better functioning. A score of 91 to 100 indicates excellent functioning in all 4 main areas; the patient is held in high consideration for their good qualities, copes adequately with life problems, and is involved in a wide range of interests and activities. A score of 1 to 10 indicates a lack of autonomy in basic functioning with extreme behaviors but without survival risk (score 6 to 10) or with survival risk (score 1 to 5). An increase in the scale demonstrates a beneficial response. Review of the relevant scientific literature and the analysis of the psychometric properties of the 4 domains that result in the generation of the PSP Total score were supportive of the appropriateness and cross-cultural applicability of the use of the PSP in drug development for the indication of negative symptoms of schizophrenia The PSP was assessed at the timepoints described in Table 6. The changes from Baseline in the PSP Total score and individual domain scores after 12 weeks of treatment were exploratory efficacy endpoints in Study MIN-101C03, and the Total score was the key secondary efficacy endpoint and the PSP domain scores were exploratory endpoints in Study MIN-101C07.

**Table 6 Timing of PSP Assessments**

| | **DB Period** | **OL Period** |
|---|---|---|
| Study MIN-101C03 | **BL, Weeks 4, 12** | **Weeks 24,** 36 |
| Study MIN-101C07 | **BL, Weeks 4,** 8, **12** | **Weeks** 16, **24,** 32, 40, 48, 52 |

| | | |
|---|---|---|
| BL = Baseline; DB = double-blind; OL = open-label; PSP = Personal and Social Performance. Timepoints that were assessed in both studies are in bold. | | |

### CGI-S AND CGI-I

The CGI-S is a clinician-rated scale that is designed to rate the severity of the patient's illness at the time of assessment, including knowledge of the patient's history, psychosocial circumstances, symptoms, behavior, and the impact of the symptoms on the patient's ability to function relative to the clinician's past experience with patients who have the same diagnosis and similar improvement with treatment. Considering total clinical experience, a patient is assessed on severity of mental illness at the time of rating, where 1 = normal (not at all ill); 2 = borderline mentally ill; 3 = mildly ill; 4 = moderately ill; 5 = markedly ill; 6 = severely ill; or 7 = extremely ill.

The CGI-I is a 7-point scale that requires the clinician to assess how much the patient's illness has improved or worsened relative to a Baseline state at the beginning of the intervention and is rated as 1 = very much improved; 2 = much improved; 3 = minimally improved; 4 = no change; 5 = minimally worse; 6 = much worse; or 7 = very much worse.

The CGI-S and CGI-I were assessed at the timepoints described in Table 7. The CGI-S and CGI-I were exploratory endpoints in Studies MIN-101C03 and MIN-101C07.

**Table 7. Timing of CGI-S and CGI-I Assessments**

| | | **DB Period** | **OL Period** |
|---|---|---|---|
| CGI-S | | | |
| | Study 1 | **SCR, BL, Weeks 4, 8, 12** | Weeks 18, **24,** 36 |
| | Study 2 | **SCR, BL, Weeks 2, 4, 8, 12** | Weeks 14, 16, 20, **24,** 28, 32, 40, 48, 52 |
| CGI-I | | | |
| | Study 1 | **Weeks 4, 8, 12** | Weeks 18, **24,** 36, 37/EOS |
| | Study 2 | **Weeks** 2, **4**, **8, 12** | Weeks 14, 16, 20, **24,** 28, 32, 40, 48, 52 |

| | | | |
|---|---|---|---|
| BL = Baseline; CGI-I = Clinical Global Impression - Improvement Scale; CGI-S = Clinical Global Impression - Severity Scale; DB = double-blind; EOS = end of study; OL = open-label; SCR = Screening Timepoints that were assessed in both studies are in bold. | | | |

### EXAMPLE 2 - SUMMARY OF PHASE 2B STUDY #1

Phase 2b Study #1 (also referred to herein as "MIN-101C03") was a Phase 2b, double-blind ("DB"), placebo-controlled, randomized, multicenter 12-week study to evaluate the efficacy, safety, and tolerability of Roluperidone in patients ≥ 18 years to ≤ 60 years with negative symptoms of schizophrenia, followed by a 24-week open-label ("OL") extension. The primary objective of the study was to evaluate the efficacy of Roluperidone compared to placebo in improving the negative symptoms of schizophrenia as measured by the change from Baseline in the PANSS negative factor score of the PSM ("PANSS PSM") over 12 weeks of treatment.

The study consisted of a pretreatment screening period of up to 28 days (including washout), a 12-week, DB, placebo-controlled period, and a 24-week OL period. Eligible patients (who were symptomatically stable for at least 3 months and had scores of at least 20 on the PANSS negative subscale score) were randomized in a 1:1:1 ratio to receive oral Roluperidone (a modified-release, "MR", formulation) 32 mg QD, Roluperidone 64 mg QD, or placebo for 12 weeks. If patients were taking antipsychotic treatments, they discontinued these and had a washout period of 2 days before beginning their assigned study treatment. During the 24-week, OL treatment extension period, patients who were initially randomized to Roluperidone 32 mg or 64 mg continued on the same dose, while patients who were initially randomized to placebo crossed over to Roluperidone 32 mg or 64 mg in a 1:1 ratio.

### Summary of Inclusion Criteria

### Diagnosis/ Disease Criteria/ Medical Condition:

- Patient must have met the diagnostic criteria for schizophrenia as defined in the DSM-5, as established by a full psychiatric interview in conjunction with the Mini International Neuropsychiatric Interview.
- Patient was stable in terms of both positive and negative symptoms of schizophrenia over the last 3 months according to his or her treating psychiatrist.
- Patient with a score of at least 20 on the PANSS negative subscale score.
- Patients with PANSS item score of < 4 on: P4 Excitement, hyperactivity. P7 Hostility; P6 Suspiciousness; G8 Uncooperativeness; and G14 Poor impulse control.

### Medications:

- Patients may have been on any psychotropic before the study if the psychotropics were discontinued at the beginning of the washout phase without risking the patient's safety.
- No change in any psychotropic medication during the last month (changes were allowed if done for administrative reasons or with the permission of the Sponsor Responsible Medical Officer).
- Patient in whom, in the opinion of the investigator, a switch to another antipsychotic medication or initiation of an antipsychotic medication was indicated.

### Reliability/ Compliance/ Consent:

- Patient or patient's legal representative must have provided informed consent, and the patient had to be able to understand the nature of the study.
- Patient was considered by the investigator to be reliable and likely to cooperate with the assessment procedures.

### Gender:

- Male or female.
- Female patient, if of childbearing potential, must have tested negative for pregnancy and must have been using a double barrier contraceptive method.

### Age:

- 18-60 years, inclusive.

### CYP2D6 P450 Status:

- Patient must have been extensive metabolizers for CYP2D6 P450, as determined by genotyping test before the first drug dose was administered.

### History of violence:

- No history of violence against self, others, or property.

### Summary of Exclusion Criteria

### Diagnosis/ Disease Criteria/ Medical Condition:

Current bipolar disorder, panic disorder, obsessive compulsive disorder, or evidence of mental retardation.

### Medications:

- Patient who could not be discontinued from psychotropics other than those allowed.
- Patient who received clozapine within 6 months of the Screening visit. (country-specific exception for patients in Russia: a dose of ≤ 100 mg/day for the treatment of insomnia was allowed).
- Patient receiving treatment with depot antipsychotic medication had to be enrolled in the study 4 weeks after the last injection.

### Other Medical Conditions:

- Patient's condition was due to direct physiological effects of a substance (e.g., a drug of abuse, or medication) or a general medical condition.
- Patient who had electroconvulsive therapy, vagal nerve stimulation, or repetitive transcranial magnetic stimulation within the 3 months prior to the Screening visit or who were scheduled for electroconvulsive therapy, vagal nerve stimulation, or repetitive transcranial magnetic stimulation at any time during the study.
- Patient with a history of significant other major or unstable neurological, neurosurgical (e.g., head trauma), metabolic, hepatic, renal, hematological, pulmonary, cardiovascular, metabolic, gastrointestinal, or urological disorder.
- Patient with a history of epilepsy seizure disorder (patient with a history of a single childhood febrile seizure could be enrolled in this study).
- Patient with clinically significant abnormalities in hematology, blood chemistry, electrocardiogram, or physical examination not resolved by the Baseline visit.
- Current systemic infection (e.g., hepatitis B, hepatitis C, HIV, tuberculosis).
- Patients with positive hepatitis B core antibody test and negative HBsAg could be included in the study if aminotransferase levels (alanine aminotransferase/serum glutamic pyruvic transaminase and aspartate aminotransferase/ serum glutamic oxaloacetic transaminase) did not exceed 2 × upper limit of normal (ULN).
- Patient who required or could require concomitant treatment with any other medication likely to increase QT interval (e.g., paroxetine, fluoxetine, duloxetine, amiodarone).
- Patient who required medication inhibiting CYP2D6.
- Patient with a clinically significant ECG abnormality that could have been a safety issue in the study, including QT interval value corrected for heart rate using QT interval value > 430 msec for males and > 450 msec for females.
- Patient with a history of myocardial infarction based on medical history or ECG findings at Screening.
- Familial or personal history of long QT syndrome or with additional risk factors for Torsade de Pointes.

### Gender:

- Woman of childbearing potential, or man, who was unwilling or unable to use accepted methods of birth control.
- Woman with a positive pregnancy test, who was lactating, or who planned to become pregnant during the study.

### BMI:

- ≥ 35 kg/m².

### Substance Use:

- Patient had a history of substance abuse [Country-specific exception for patients in Romania: "or dependence according to DSM criteria"] within 3 months of the Screening visit (excluding caffeine and cigarette smoking).
- Positive urine drug screen except when related to prescribed benzodiazepines and opiates recently prescribed for an episode of acute pain (e.g., dental extraction).

### History of Suicidal Behavior:

- Significant risk of suicide or attempted suicide, or of danger to self or others.

### Other:

- Patient who had participated in another clinical study within 3 months prior to Screening.

### DOUBLE-BLIND (DB) PERIOD

Patient Disposition and Baseline Characteristics in the DB period.

A total of 244 patients were randomized and all received treatment (Safety population) (83 patients in the placebo group, 78 patients in the 32 mg Roluperidone group, and 83 patients in the 64 mg Roluperidone group). The intent-to-treat ("ITT") population (234 patients; 95.9%) consisted of all patients in the Safety population who had at least 1 post Baseline assessment of the PANSS total score. Demographic characteristics were comparable between the 3 treatment groups for age, sex, race, and BMI. The overall mean age of patients was 40 years (range: 18 to 60 years), 56% were male, all patients were White, and the mean BMI was 26 kg/m². All patients targeted for enrollment in the study were to be extensive metabolizers for CYP2D6 (as a strategy to minimize the risk of QT prolongation in patients randomized to Roluperidone).

All disease characteristics at Baseline, including NSFS, PSP Total score, CGI-S, and PANSS total and subscale scores, were comparable between the 3 treatment groups. At Baseline, the overall mean NSFS score was 25, mean PSP Total score was 52, mean CGI-S score was 4, mean PANSS total score was 80, mean PANSS negative subscale score was 27, and mean PANSS positive subscale score was 14.

### RESULTS IN THE DB PERIOD

Of the 169 patients (69%) who completed the DB period of the study through Week 12 of treatment, a larger proportion completed in the Roluperidone treatment groups: 56 (68%) patients completed in the placebo group, 55 (71%) patients completed in the 32 mg group and 58 (70%) patients completed in the 64 mg group. The most frequent reasons for patient study discontinuation across all treatment groups were unsatisfactory treatment response (14%) and withdrawn consent (7%), both with the highest rate in the placebo group.

Roluperidone was generally well tolerated with almost exclusively mild or moderate treatment-emergent adverse events (TEAEs) and few serious adverse events (SAEs), and no new safety signals were detected for up to 36 weeks of treatment in this study. Roluperidone and placebo-treated patients had comparable rates of TEAEs leading to discontinuation and these were mainly psychiatric disorders. QTcF prolongation > 450 msec and QTcF increases relative to baseline of ≥ 30 msec were observed in a dose-dependent manner in the Roluperidone groups. Clinically significant QTcF prolongation > 500 msec and change from Baseline in QTcF > 60 msec were reported in 2 patients of the 64 mg Roluperidone group, and in no patients in the 32 mg Roluperidone group or in the placebo group during the DB period of the study. The mean changes across all vital sign parameters were small from Baseline to the respective study visits in the DB period of the study.

A summary of the efficacy results of Study MIN-101C03 is provided in Table 8, below:

**Table 8. Summary of Key Efficacy Endpoints, Roluperidone Compared with Placebo, At Week 12, ITT Population**

| | | Change from Baseline LS Means (SEM) | | | p-value | | Effect Size^{e} | |
|---|---|---|---|---|---|---|---|---|
| | | Roluperidone | | | Roluperidone vs Placebo | | Roluperidone vs Placebo | |
| | | Placebo (N = 79) | 32 mg (N = 76) | 64 mg (N = 79) | 32 mg | 64 mg | 32 mg | 64 mg |
| **Primary Endpoint** | | | | | | | | |
| PANSS Negative Factor Score^{a} | | -1.53 (0.47) | -3.07 (0.49) | -3.50 (0.48) | **0.0240** | **0.0036** | 0.45 | 0.57 |

| **Post-hoc Analyses** | | | | | | | | |
|---|---|---|---|---|---|---|---|---|
| NSFS | | -1.6 (0.53) | -3.43 (0.52) | -3.9 (0.53) | **0.002** | **<0.001** | 0.61 | 0.78 |
| Responder Analyses | | | | | | | | |
| | Number of Patients with 20% reduction in NSFS at Week 12 | 9/54 (17%) | 15/51 (29%) | 18/54 (33%) | 0.124 | **0.049** | 0.06 | 0.17 |
| | Number of Patients with 30% reduction in NSFS at Week 12 | 2/54 (4%) | 2/51 (4%) | 7/54 (13%) | 0.963 | 0.099 | 0 | 0.15 |
| | Number of Patients with 7-Point improvement in PSP Total Score at Week 12 | 16/53 (30%) | 17/50 (34%) | 26/54 (48%) | 0.530 | 0.062 | 0.05 | 0.17 |
| | Number of Patients with 10-Point improvement in PSP Total Score at Week 12 | 16/53 (30%) | 17/50 (34%) | 26/54 (48%) | 0.530 | 0.062 | 0.05 | 0.17 |

| **Secondary Endpoints** | | | | | | | | |
|---|---|---|---|---|---|---|---|---|
| PANSS Total Score | | -0.56 (1.25) | -3.66 (1.28) | -5.83 (1.26) | 0.0819 | 0.0031 | 0.34 | 0.57 |
| | 3-Factor Negative Subscale Score | -1.71 (0.41) | -3.35 (0.43) | -3.82 (0.42) | **0.0064** | **0.0004** | 0.54 | 0.70 |
| | 3-Factor Positive Subscale Score | 0.99 (0.44) | 0.46 (0.45) | 0.36 (0.44) | 0.4018 | 0.3067 | 0.16 | 0.20 |

| | Change from Baseline LS Means (SEM) | | | p-value | | Effect Size^{e} | | |
|---|---|---|---|---|---|---|---|---|
| | Roluperidone | | | Roluperidone vs Placebo | | Roluperidone vs Placebo | | |
| | Placebo (N = 79) | 32 mg (N = 76) | 64 mg (N = 79) | 32 mg | 64 mg | 32 mg | 64 mg | |
| 3-Factor General Psychopathology Subscale Score | -0.05 (0.61) | -1.07 (0.62) | -2.58 (0.62) | 0.2359 | **0.0034** | 0.23 | 0.56 | |
| 5-Factor Positive Score^{a} | 0.29 (0.31) | 0.58 (0.32) | -0.25 (0.31) | 0.5045 | 0.2146 | -0.13 | 0.24 | |
| 5-Factor Dysphoric Mood Score^{a} | -0.07 (0.31) | -0.32 (0.32) | -1.04 (0.32) | 0.5644 | **0.0266** | 0.11 | 0.43 | |
| 5-Factor Activation Score^{a} | 1.09 (0.35) | -0.05 (0.36) | -0.17 (0.36) | **0.0240** | **0.0118** | 0.44 | 0.49 | |
| 5-Factor Autistic Preoccupation Score^{a} | -0.65 (0.34) | -0.85 (0.35) | -1.21 (0.34) | 0.6700 | 0.2408 | 0.08 | 0.22 | |
| Clinical Global Impression of Severity^{b} | -0.10 (0.1) | -0.40 (0.1) | -0.40 (0.1) | 0.0982 | **0.0234** | 0.35 | 0.43 | |
| Clinical Global Impression of Improvement^{c} | | - | - | 0.2378 | **0.0032** | 0.33 | 0.57 | |
| Brief Negative Symptoms Scale Total Score | -3.23 (0.90) | -5.44 (0.93) | -6.94 (0.92) | 0.0869 | **0.0040** | 0.33 | 0.56 | |
| Brief Assessment of Cognition in Schizophrenia | | | | | | | | |
| Total Score | 11.5 (3.96) | 23.8 (4.04) | 14.9 (3.99) | **0.0292** | 0.5446 | 0.43 | 0.12 | |
| Total Verbal Fluency | 0.98 (1.27) | 5.76 (1.29) | 4.37 (1.29) | **0.0076** | 0.0554 | 0.51 | 0.36 | |

| **Exploratory Endpoints** | | | | | | | | |
|---|---|---|---|---|---|---|---|---|
| Calgary Depression Scale for Schizophrenia | 0.04 (0.22) | -0.37 (0.22) | -0.71 (0.21) | 0.1756 | **0.0091** | 0.25 | 0.46 | |
| Personal and Social Performance (PSP) PSP Total Score (mapped from the 4 domains)^{d} | 1.87 (1.62) | 1.77 (1.64) | 8.52 (1.62) | 0.9682 | **0.0033** | -0.01 | 0.56 | |
| Socially Useful Activities | -0.32 (0.09) | -0.41 (0.10) | -0.57 (0.10) | 0.4775 | 0.0601 | 0.14 | 0.38 | |
| Personal and Social Relationships | -0.21 (0.10) | -0.22 (0.10) | -0.56 (0.10) | 0.9174 | **0.0129** | 0.02 | 0.53 | |
| Self-care | -0.21 (0.08) | -0.37 (0.08) | -0.48 (0.08) | 0.1736 | **0.0210** | 0.27 | 0.46 | |
| Disturbing & Aggressive Behavior | 0.06 (0.08) | -0.15 (0.08) | -0.24 (0.08) | 0.0532 | **0.0057** | 0.36 | 0.51 | |

| | | | | | | | | |
|---|---|---|---|---|---|---|---|---|
| ITT (N = 76) intent-to treat; LS (N = 76) least square; NSFS (N = 76) PANSS Marder's Negative Symptoms Factor Score; PANSS (N = 76) Positive and Negative Syndrome Scale; SEM (N = 76) standard error of mean. ^{a} From the pentagonal structure model. Analyzed using ranked data; change from Baseline and effect size are based on observed change from Baseline data. ^{c} Analyzed using ranked data; effect size is based on observed data. ^{d} Total score was not captured during the study and was mapped from the 4 domain scores according to Morosini P-L, et al. "Development, reliability and acceptability of a new version of the DSM-IV Social and Occupational Functioning Assessment Scale (SOFAS) to assess routine social functioning." Acta Psychiatr Scand. 2000;101:323-329. LS Mean, standard error and p-value are from a mixed model repeated measures (MMRM) with treatment (placebo, MIN-101 32 mg, MIN-101 64 mg), visit, pooled study center, and treatment-by-visit interaction terms as fixed effects, subject nested in treatment as a random effect with baseline value as covariates. An unstructured covariance matrix was used. ^{c} Post-hoc to the planned analysis described in the statistical analysis plan, effect size estimation was used to measure the relative size of the effect of Roluperidone during the double-blind period for the efficacy analysis parameters used in this study. Note: Statistically significant p-values are denoted in bold. | | | | | | | | |

The primary efficacy analysis of the change from Baseline to Week 12 in the PANSS PSM demonstrated a statistically significant improvement for the 64 mg Roluperidone group compared to the placebo group (p ≤ 0.003). A post-hoc analysis of the change from Baseline to Week 12 in NSFS also demonstrated a statistically significant improvement for the 64 mg Roluperidone group compared with placebo (p ≤ 0.001). Other secondary/exploratory efficacy analyses showed statistically significant improvements in the 64 mg Roluperidone group compared with placebo after 12 weeks of the DB period for the PANSS total score, PANSS dysphoric mood factor, PANSS activation factor, PANSS general psychopathology scale, PANSS negative scale, CGI-S score, CGI-I score, BNSS total score, CDSS total score, PSP Total score, and the PSP personal and social relationships, PSP self-care, and PSP disturbing and aggressive behaviors domain scores.

The effect of 64 mg Roluperidone became evident already after 2 weeks for NSFS (p ≤ 0.002) and for 4 weeks for PSP Total score (p ≤ 0.1007) and CGI-S (p ≤ 0.0032) and continued to increase during the 12 weeks of the DB period. A further increase was seen during the 36 weeks of the OL period for PANSS negative factor score. A responder analysis showed that a 20% reduction in NSFS was reported by 33% of the 64 mg Roluperidone group compared to 17% of the placebo group (p ≤ 0.049; Table 8) and a 30% reduction was reported by 13% of the 64 mg Roluperidone group compared to 4% of the placebo group (not significant). Likewise, a 7-point increase and a 10-point increase were reported by 48% of the 64 mg Roluperidone group compared to 30% of the placebo group (not significant).

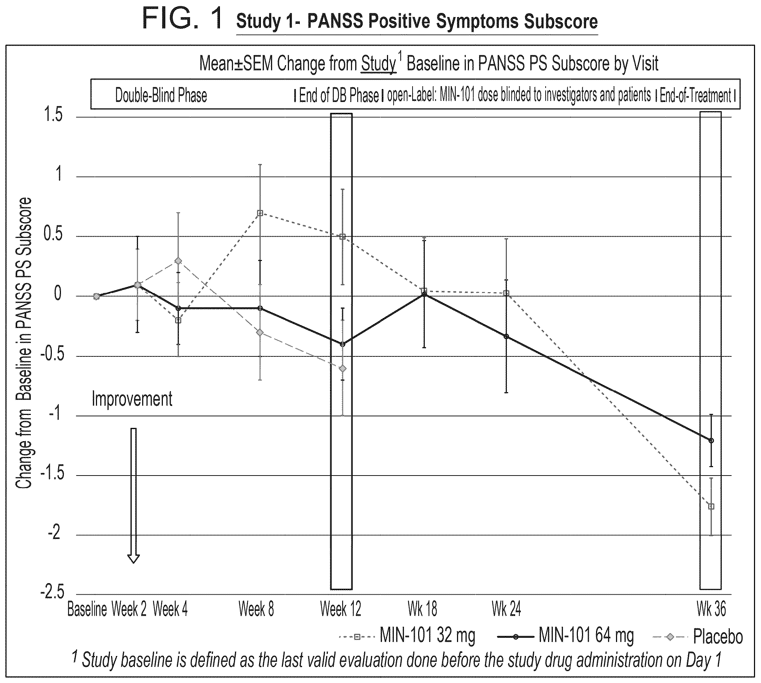

The rate of relapse (during the DB period was lowest for the 64 mg Roluperidone (7 [9%]) and 32 mg Roluperidone groups (5[7%]) and comparable to the placebo group (8 [10%]). This was consistent with the change seen in the PANSS positive symptoms scores with 64 mg Roluperidone, which remained similar to placebo (FIG. 1).

These findings support the conclusion that Roluperidone at both doses was superior to placebo, and that the observed treatment effects are true effects of Roluperidone.

### OPEN-LABEL (OL) PERIOD

Of the 169 patients who completed the 12-week DB period, 142 (84%) were enrolled in the 24-week OL period. The 98 patients who had received Roluperidone during the DB period were continued on the Roluperidone dose they had previously received (45 patients on 32 mg, and 53 patients on 64 mg), and patients who previously had been given placebo were randomized 1:1 to receive either Roluperidone 32 mg or 64 mg. Of the 44 patients from the placebo group who entered the 24-week OL period, 25 patients received 32 mg Roluperidone and 19 patients received 64 mg Roluperidone.

### RESULTS IN THE OL PERIOD

A total of 88 patients (28 in the 32 mg Roluperidone throughout group, 32 in the 64 mg Roluperidone throughout group, 15 in the placebo to 32 mg Roluperidone group, and 13 in the placebo to 64 mg Roluperidone group) completed the OL period. For the PANSS PSM, a trend of improvement continued during the OL period for patients in the placebo groups who crossed over to Roluperidone treatment as well as for the whole study ("WS") period for patients treated from the beginning of the study with Roluperidone.

During the OL period (where no placebo control was available), continued improvement was seen with Roluperidone for those efficacy parameters that had previously shown improvements during the DB period.

### EXAMPLE 3 - SUMMARY OF PHASE 3 STUDY #2

Study #2 (also referred to as "MIN101C07") was a Phase 3, randomized, DB, placebo-controlled, parallel-group 12-week study to evaluate the efficacy and safety of Roluperidone in patients ≥ 18 years to ≤ 55 years with negative symptoms of schizophrenia, followed by a 40-week OL extension. The study consisted of a pretreatment screening period of up to 28 days (including an antipsychotic medication washout period), a 12-week, DB, placebo-controlled period, and a 40-week OL period. Eligible patients (with moderate to severe negative symptoms of schizophrenia and stable positive symptoms but without severe symptoms of suspiciousness, agitation, hostility, uncooperativeness, or poor impulse control) were randomized 1:1:1 to receive Roluperidone 32 mg QD, Roluperidone 64 mg QD (GR01/B formulation, see U.S. Patent No. 11,464,744 ), or placebo, orally, for 12 weeks. If patients were taking antipsychotic treatments, they discontinued these and had a washout period of 2 days before beginning their assigned study treatment. During the 40-week OL treatment extension, patients who were initially randomized to Roluperidone 32 mg or 64 mg continued treatment with the same dose, while patients who were initially randomized to placebo crossed over to Roluperidone 32 mg or 64 mg in a 1:1 ratio.

### Summary of Inclusion Criteria

### Diagnosis/ Disease Criteria/ Medical Condition:

- Patient must have met the diagnostic criteria for schizophrenia as defined in the DSM-5, as established by a full psychiatric interview in conjunction with the Mini International Neuropsychiatric Interview.
- Documented diagnosis of schizophrenia for at least 1 year before screening into the study.
- Patient was stable in terms of both positive and negative symptoms of schizophrenia over the last 6 months according to his or her treating clinician and/or based on documentation in the clinical chart or medical record. Patients with or without positive symptoms were allowed if those symptoms were stable for the last 6 months.
- Patient with a score of > 20 on the PANSS negative subscale score (the original PANSS scale [sum of N1 + N2 + N3 + N4 + N5 + N6 + N7]) at Screening (Visit 1) and Baseline (Visit 3) AND < 4 points absolute difference between the 2 visits.

### Medications:

- Patients may have been on any psychotropic before the study if the psychotropics were discontinued at the beginning of the washout phase without risking the patient's clinical status or safety.

### Reliability/Compliance/Consent:

- Patient and patient's legal representative, if applicable, must have provided informed consent prior to the initiation of any study related procedures, and the patient must have been judged by the investigator as being capable of understanding the study requirements.
- Had a caregiver or family member or health care personnel who could provide information towards assessment and support the patient in terms of compliance with the protocol. The caregiver must have had contacts with the patient frequently and was not expected to change during the study.
- Patient and the caregiver were considered by the investigator to be reliable and likely to cooperate with the assessment procedures.

### Gender

- Male or female.
- Female patients who were not of childbearing potential, defined as women who were postmenopausal (defined as spontaneous amenorrhea for at least 1 year or spontaneous amenorrhea for at least 6 months confirmed by follicle stimulating hormone result of ≥ 40 IU/mL) or permanently sterilized (e.g., tubal occlusion, hysterectomy, bilateral salpingectomy).
- Female patient, if of childbearing potential, must have tested negative for pregnancy and must have been using a double barrier contraceptive method.

### Age:

- 18-55 years old, inclusive.

### BMI:

- < 35 kg/m², at Screening.

### CYP2D6 P450 Status:

- Patient must have been extensive metabolizers (normal) for CYP2D6 P450, defined as a patient that had at least 1 functional allele (e.g., *1, or *2), as determined by study specific genotyping test before the first drug dose was administered.

### History of Violence

- No history of violence against self or others during the last 1 year.

### Other:

- Patient was currently an outpatient and had not been hospitalized for the last 6 months for acute exacerbation or symptoms worsening. Patients hospitalized for any time period during the last 6 months for social reasons or were currently hospitalized for social reasons could be included only with Sponsor's Responsible Medical Officer's approval (in addition for Ukraine only, the following criteria must have been fulfilled: the patient had a permanent place of residence, was legally capable, and had a caregiver). The social reasons must have been documented in the electronic case report form.

### Summary of Exclusion Criteria

### Diagnosis/ Disease Criteria/ Medical Condition:

- Current major depressive disorder, bipolar disorder, panic disorder, obsessive compulsive disorder, or intellectual disability (intellectual developmental disorder diagnosed by age 14).
- Patient with PANSS item score of > 4 on: P4 Excitement/hyperactivity; P6 Suspiciousness/persecution; P7 Hostility; G8 Uncooperativeness; G14 Poor impulse control.
- A Calgary depression scale for schizophrenia total score > 6. #4) A score of ≥ 2 on any 2 of items 1, 2, or 3, or a score of ≥ 3 on item 4 of the Barnes akathisia rating scale (BARS).

### Medications:

- Patient who could not be discontinued from psychotropics other than those allowed.
- Patient who had received clozapine within 6 months of the Screening visit except when used for insomnia at doses ≤ 100 mg per day.
- Patient who was receiving treatment with long acting or depot antipsychotic medication unless his/her next scheduled dose was to occur during the protocol Screening period and could have been omitted to allow for sufficient washout before receiving the study drug.

### Other Medical Conditions:

- Patient's condition was due to direct physiological effects of a substance (e.g., a drug of abuse, or medication) or a general medical condition.
- Patient who had electroconvulsive therapy, vagal nerve stimulation, or repetitive transcranial magnetic stimulation within the 6 months prior to the Screening visit or who were scheduled for electroconvulsive therapy, vagal nerve stimulation, or repetitive transcranial magnetic stimulation at any time during the study.
- Patient with a history of significant other major or unstable neurological, neurosurgical (e.g., head trauma), metabolic, hepatic, renal, hematological, pulmonary, cardiovascular, metabolic, gastrointestinal, or urological disorder.
- Patient with a history of seizures (patient with a history of a single childhood febrile seizure could be enrolled in this study).
- Patient with clinically significant abnormalities in hematology, blood chemistry, electrocardiogram, or physical examination not resolved by the Baseline visit which according to the investigator could interfere with study participation.
- Current systemic infection (e.g., hepatitis B, hepatitis C, HIV, tuberculosis). Patients with positive hepatitis B core antibody test and negative Hepatitis B surface antigen could be included in the study if aminotransferase levels (alanine aminotransferase/serum glutamic pyruvic transaminase and aspartate aminotransferase/serum glutamic oxaloacetic transaminase) did not exceed 2 × upper limit of normal (ULN).
- Patient who required or could require concomitant treatment with any other medication likely to increase QT interval (e.g., paroxetine, fluoxetine, duloxetine, amiodarone).
- Patient who required medication inhibiting CYP2D6 or CYP3A4.
- Patient with a clinically significant ECG abnormality that could have been a safety issue in the study, including QT interval value corrected for HR using QTcF > 430 msec for males and > 450 msec for females.
- Patient with a history of myocardial infarction based on medical history or ECG findings at Screening.
- Familial or personal history of long QT syndrome or with additional risk factors for Torsade de Pointes.
- Patient whose safety laboratory results showed 1 or more of the following: potassium < 3.40 mmol/L, or calcium < 2.07 mmol/L, or magnesium < 0.70 mmol/L.
- Patients with unexplained syncope.

### Gender:

- Woman of childbearing potential, or man, who was unwilling or unable to use accepted methods of birth control.
- Woman with a positive pregnancy test, who was lactating, or who planned to become pregnant during the study.

### Substance Use:

- Patient had a history of substance use disorder within 3 months of the Screening visit (excluding caffeine and cigarette smoking).
- Positive urine drug screen for drugs of abuse (cocaine, methadone, amphetamines, cannabinoids, opiates, benzodiazepines, and barbiturates), tricyclic antidepressants, and alcohol (except for prescription benzodiazepines).

### History of suicidal behavior:

- Had a current or recent history of serious suicidal behavior within the past 1 year.

### Other:

- Patient who had participated in another clinical study within 3 months prior to Screening, or had received Roluperidone previously, or had previously participated in > 2 clinical studies with experimental medication within the past 2 years (previous participation in 3 clinical studies with experimental medication was to require approval of the Sponsor before eligibility was determined).

### Patient Disposition

A total of 515 patients were randomized and 513 received treatment (172 patients received placebo, 170 patients received 32 mg Roluperidone, and 171 patients received 64 mg Roluperidone) during the DB period. These 513 patients were included in the ITT population. Demographic characteristics were comparable between the 3 treatment groups for age, sex, race, and BMI. The overall mean age of patients was 41 years (range: 18 to 55 years), 61% were male, 88% were White, and the mean BMI was 26 kg/m². All patients targeted for enrollment in the study were to be extensive metabolizers for CYP2D6 (as a strategy to minimize the risk of QT prolongation in patients taking Roluperidone). All disease characteristics at Baseline, including NSFS, PSP Total score, CGI-S, and PANSS total and subscale scores, were comparable between the 3 treatment groups. The overall mean NSFS score was 25, mean PSP score was 53, mean CGI-S score was 4, mean PANSS total score was 79, mean PANSS negative subscale score was 27, and mean PANSS positive subscale score was 14.

### RESULTS IN THE DB PERIOD

A total of 379 of 515 patients (74%) completed the DB period of the study through Week 12 of treatment, with a similar proportion in all treatment groups: 130 of 172 patients (76%) in the placebo group, 123 of 170 patients (72%) in the 32 mg Roluperidone group, and 126 of 171 patients (74%) patients in the 64 mg Roluperidone group. The most frequent reason for patient study discontinuation over all treatment groups was withdrawal of consent (17% patients in the placebo group, 19 % patients in the 32 mg group, and 23% patients in the 64 mg group). Due to data integrity concerns raised by the blinded review of data from one study site, all patient data from this site was excluded giving rise to a modified-ITT-population. The mITT population included 496 patients: 167 patients each received placebo and 32 mg Roluperidone, and 162 patients received 64 mg Roluperidone.

Roluperidone was generally well tolerated, and no new safety signals were detected. The incidence of TEAEs was slightly higher in the Roluperidone groups than in the placebo group during the DB period (42% in the 32 mg Roluperidone group, 37% in the 64 mg Roluperidone group, and 33% in the placebo group). The majority of TEAEs reported were in the System Organ Class (SOC) psychiatric disorders. The most commonly reported TEAEs were insomnia, schizophrenia, anxiety, agitation, and headache. Roluperidone induced no significant change in safety laboratory parameters, including prolactin. It induced no significant change in vital signs, including weight and waist circumference. Dose-related QT prolongation was observed, and the great majority of clinically significant QT prolongations were observed with the 64 mg dose. In total, 6 patients met stopping criteria due to QT prolongations, and 4 of these 6 patients were discontinued from the study due to such prolongations. The remaining 2 patients were discontinued for other reasons prior to confirming their QT prolongations.

The efficacy results in this study are summarized below in Table 9.

**Table 9. Study MIN-101C07 - Summary and Change from Baseline in Primary, Key and Select Secondary and Endpoints, Week 12**

| | | **Change from Baseline (LS Means [SEM])** | | | **p-value** | | **Effect Size ^{a}** | |
|---|---|---|---|---|---|---|---|---|
| | | **Roluperidone** | | | **Roluperidone vs Placebo** | | **Roluperidone vs Placebo** | |
| | | **Placebo (N = 172)** | **32 mg (N = 170)** | **64 mg (N = 171)** | **32 mg** | **64 mg** | **32 mg** | **64 mg** |
| **Primary Endpoint** | | | | | | | | |
| NSFS (ITT) | | -3.5 (0.34) | -4.0 (0.35) | -4.3 (0.34) | 0.259 | 0.064 | 0.13 | 0.21 |
| NSFS (mITT) | | -3.5 (0.35) | -4.0 (0.35) | -4.5 (0.35) | 0.286 | **0.044** | 0.13 | 0.26 |

| **Key Secondary Endpoint** | | | | | | | | |
|---|---|---|---|---|---|---|---|---|
| PSP Total Score (ITT) | | 3.9 (0.73) | 4.5 (0.75) | 6.1 (0.73) | 0.542 | **0.021** | 0.07 | 0.27 |
| PSP Total Score (mITT) | | 3.8 (0.75) | 4.4 (0.77) | 6.2 (0.77) | 0.551 | **0.017** | 0.07 | 0.29 |

| **Secondary and Exploratory Endpoints** | | | | | | | | |
|---|---|---|---|---|---|---|---|---|
| Clinical Global Impression of Severity PANSS Constructs and subscales | | -0.3 (0.06) | -0.4 (0.06) | -0.5 (0.06) | 0.221 | 0.073 | 0.12 | 0.24 |
| | Total Score | -5.5 (0.84) | -7.1 (0.87) | -7.4 (0.85) | 0.168 | 0.098 | 0.17 | 0.20 |
| | Negative Symptoms Subscale | -3.8 (0.35) | -4.2 (0.35) | -4.7 (0.35) | 0.392 | **0.046** | 0.10 | 0.23 |
| | Positive Symptoms Subscale | -0.2 (0.25) | -0.3 (0.26) | -0.4 (0.25) | 0.783 | 0.478 | 0.04 | 0.07 |
| | General Psychopathology Subscale | -1.7 (0.45) | -2.8 (0.47) | -2.3 (0.46) | 0.092 | 0.380 | 0.22 | 0.12 |
| | NSFS Emotional Experience Score | -1.3 (0.16) | -1.5 (0.16) | -1.8 (0.16) | 0.401 | **0.020** | 0.11 | 0.28 |
| | NSFS Emotional Expression Score | -2.3 (0.22) | -2.6 (0.22) | -2.6 (0.22) | 0.352 | 0.349 | 0.17 | 0.17 |
| | Marder's Positive Symptoms Factor Score | -0.9 (0.26) | -1.3 (0.27) | -1.6 (0.27) | 0.190 | **0.039** | 0.14 | 0.24 |
| | Marder's Anxiety/Depression Factor Score | -0.5 (0.20) | -0.6 (0.20) | -0.7 (0.20) | 0.622 | 0.448 | 0.05 | 0.09 |
| | Marder's Disorganized Thought Factor Score | -1.2 (0.24) | -1.6 (0.25) | -1.4 (0.25) | 0.279 | 0.514 | 0.15 | 0.07 |
| | Marder's Uncontrolled | 0.2 (0.18) | 0.3 (0.18) | 0.5 (0.18) | 0.684 | 0.153 | -0.05 | -0.15 |
| | Hostility/Excitement Factor Score | | | | | | | |

| | | **Change from Baseline (LS Means [SEM])** | | | **p-value** | | **Effect Size ^{a}** | |
|---|---|---|---|---|---|---|---|---|
| | | **Roluperidone** | | | **Roluperidone vs Placebo** | | **Roluperidone vs Placebo** | |
| | | **Placebo (N = 172)** | **32 mg (N = 170)** | **64 mg (N = 171)** | **32 mg** | **64 mg** | **32 mg** | **64 mg** |
| PSP Domains | | | | | | | | |
| | Self-Care | -0.3 (0.06) | -0.4 (0.06) | -0.3 (0.06) | 0.261 | 0.819 | 0.15 | 0.04 |
| | Socially Useful Activities | -0.3 (0.05) | -0.3 (0.06) | -0.4 (0.05) | 0.865 | **0.047** | 0.02 | 0.18 |
| | Personal and Social Relationships | -0.3 (0.06) | -0.4 (0.06) | -0.3 (0.06) | 0.076 | 0.501 | 0.15 | 0.00 |
| | Disturbing and Aggressive Behaviors | 0.0 (0.05) | 0.0 (0.05) | 0.0 (0.05) | 0.961 | 0.186 | 0.00 | -0.07 |
| Clinical Global Impression of Improvement^{b} | | 3.3 (0.08) | 3.4 (0.08) | 3.3 (0.08) | 0.683 | 0.746 | -0.12 | 0.02 |
| Calgary Depression Scale for Schizophrenia | | 0.1 (0.13) | -0.2 (0.13) | -0.1 (0.13) | 0.093 | 0.139 | 0.21 | 0.14 |
| Total Verbal Fluency^{c} | | 2.4 (0.64) | 3.9 (0.66) | 1.7 (0.63) | 0.067 | 0.327 | 0.21 | -0.10 |

| Responder Analysis^{d} | | | | | | | | |
|---|---|---|---|---|---|---|---|---|
| | Number of Patients with 20% reduction in NSFS at Week 12 | 30/128 (23%) | 32/116 (28%) | 48/122 (39%) | 0.418 | **0.006** | 0.05 | 0.17 |
| | Number of Patients with 30% reduction in NSFS at Week 12 | 17/128 (13%) | 14/116 (12%) | 24/122 (20%) | 0.807 | 0.160 | 0.04 | 0.09 |
| | Number of Patients with 7-Point improvement in PSP Total Score at Week 12 | 37/128 (29%) | 37/116 (32%) | 50/122 (41%) | 0.589 | 0.032 | 0.03 | 0.13 |
| | Number of Patients with 10-Point improvement in PSP Total Score at Week 12 | 28/128 (22%) | 26/116 (22%) | 37/122 (30%) | 0.894 | 0.090 | 0.01 | 0.10 |

| | | | | | | | | |
|---|---|---|---|---|---|---|---|---|
| ^{a} Cohen's d (Cohen, J. (1988). Statistical power analysis (2nd ed.). Hillsdale NJ: Erlbaum.) ^{b} Observed data analyzed using MMRM with Baseline CGI-S score as covariate. ^{c} Based on sum of 3 trials. ^{d} Cohen's w. (Cohen, J. (1988). Statistical power analysis (2nd ed.). Hillsdale NJ: Erlbaum.) | | | | | | | | |

A statistically significant change in NSFS from Baseline to Week 12 for the 64 mg dose of Roluperidone compared with placebo was seen in the mITT population. Unadjusted statistical superiority was also observed at Weeks 4 and 8 for NSFS and PSP total score, despite the larger than expected placebo response on the NSFS.

In addition, statistically significant improvements (unadjusted) in NSFS from Baseline were seen at 4 and 8 weeks for the 64 mg Roluperidone group and at 4 weeks for the 32 mg Roluperidone group compared with placebo for both the ITT and mITT populations.

The key secondary efficacy endpoint, change from Baseline in PSP total score to Week 12, demonstrated nominal statistically significant improvement for the 64 mg Roluperidone group (least square (LS) mean difference vs placebo: 2.2 [95% CI: 0.3, 4.1], p ≤ 0.021) for the ITT population.

Analyses of other secondary and exploratory endpoints also demonstrated statistically significant improvements at Week 12 for the 64 mg Roluperidone group compared with the placebo group in the change from Baseline in PANSS negative subscale score, NSFS emotional experience score, Marder's PANSS positive symptoms factor score, and the "socially useful activities" PSP domain score, all in the ITT population. A responder analysis showed that a 20% reduction in NSFS was reported by 39% of the 64 mg Roluperidone group compared to 23% of the placebo group (p ≤ 0.006) and a 30% reduction was reported by 20% of the 64 mg Roluperidone group compared to 13% of the placebo group (Table 9).

Similarly, a 7-point increase in PSP Total score was reported by 20% of the 64 mg Roluperidone group compared to 13% of the placebo group (p ≤ 0.032) and a 10-point increase was reported by 30% of the 64 mg Roluperidone group compared to 22% of the placebo group (not significant).

The percentage of patients who experienced relapse in the ITT population was similar in the placebo (8 [5%]) and 64 mg Roluperidone groups (9 [5%]), and higher in the 32 mg Roluperidone group (18 [11%]) (See Table 10).

**Table 10. Study MIN-101C07 - Summary of Rate of Relapse in the Double-Blind Period, ITT Population**

| | | **Placebo** | **Roluperidone** | | | **Overall** |
|---|---|---|---|---|---|---|
| | | **(N = 172)** | **32 mg (N = 170)** | **64 mg (N = 171)** | **Total (N = 341)** | **(N = 513)** |
| Patients who experienced relapse | | 8 (5%) | 18 (11%) | 9 (5%) | 27 (8%) | 35 (7%) |
| | Logistic regression p-value^{a} | | 0.090 | 0.929 | 0.345 | |

| Days to relapse (Kaplan-Meier estimates) | | | | | | |
|---|---|---|---|---|---|---|
| | n (number censored) | 172 (164) | 170 (152) | 171 (162) | 341 (314) | 513 (478) |
| | Mean | 79.8 | 124.9 | 82.1 | 127.8 | 129.1 |
| | SE | 0.91 | 2.87 | 1.13 | 1.78 | 1.34 |
| | Median | NA | 137.0 | NA | 137.0 | 137.0 |
| | 95% CI | NA | NA | NA | NA | NA |
| | Q1, Q3 | NA | 137.0, 137.0 | NA | 137.0, 137.0 | 137.0, 137.0 |
| | p-value^{b} | 0.190 | | | | |

| | | | | | | |
|---|---|---|---|---|---|---|
| SAP = statistical analysis plan; SE = standard error; Q = quartile. ^{a} p-value is from a logistic regression with a factor for treatment and Baseline PANSS total score as a covariate. ^{b} p-value is from a test of no treatment difference based on a Cox Proportional Hazards model with covariates for treatment group and Baseline PANSS total score. Note: Relapse includes patients who may not have been included in the adjudicated list of relapse cases, but who discontinued due to AEs considered to be associated with relapse as specified in the SAP. | | | | | | |

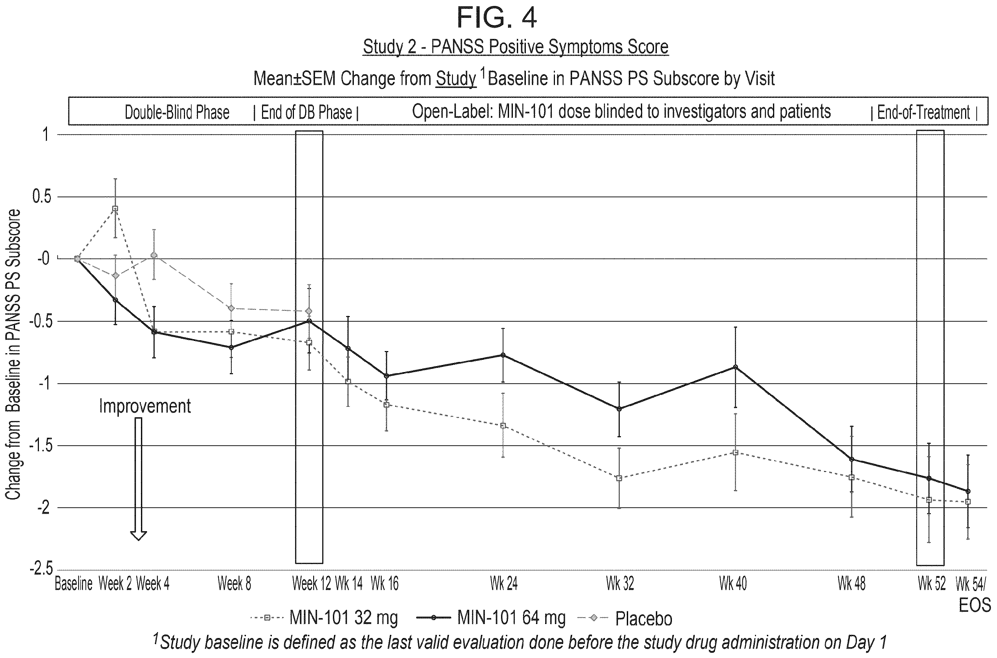

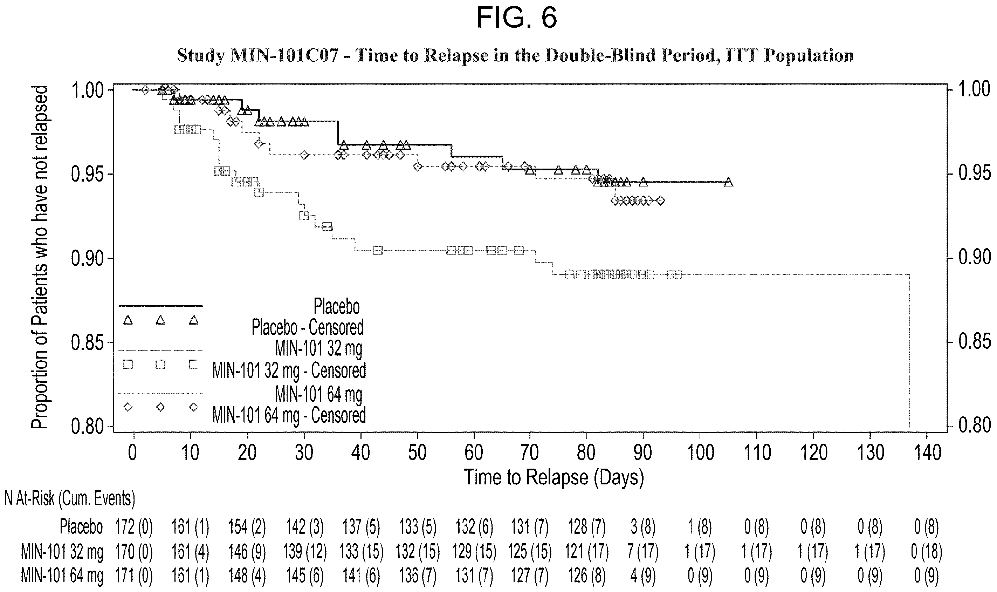

This was consistent with the change seen in the PANSS positive symptoms scores with 64 mg Roluperidone, which remained similar to placebo (FIG. 4). Among the patients who relapsed, the mean number of days to relapse was 79.8 days in the placebo group, and 82.1 days and 124.9 days in the 64 mg and 32 mg Roluperidone groups, respectively (Table 10 (above) and FIG. 6).

For Study MIN-101C07, the data provided above support the conclusion that 64 mg Roluperidone was superior to placebo, and that the observed treatment effect is true effect of Roluperidone.

### OPEN-LABEL PERIOD

Patient disposition: Of the 379 patients who completed the 12-week DB period, 333 patients participated in the 40-week OL period, including 107 patients who received 32 mg Roluperidone throughout the study, 104 patients who received 64 mg Roluperidone throughout the study, 59 patients who received placebo in the DB period and switched to 32 mg Roluperidone in the OL period, and 63 patients who received placebo in the DB period and switched to 64 mg in the OL period.

A total of 202 patients (72 in the 32 mg Roluperidone throughout group, 59 in the 64 mg Roluperidone throughout group, 35 in the placebo to 32 mg Roluperidone group, and 36 in the placebo to 64 mg Roluperidone group) completed the OL period.

### RESULTS IN THE OL PERIOD

The NSFS score continued to improve for all treatment groups during the OL period and the treatment effect persisted to Week 52 when study drug was continued. Similarly, continued improvement was seen in the PSP Total score during the OL period. Results for the additional secondary and exploratory endpoints in the OL period demonstrated either continued improvement or maintenance of stability from the DB period.

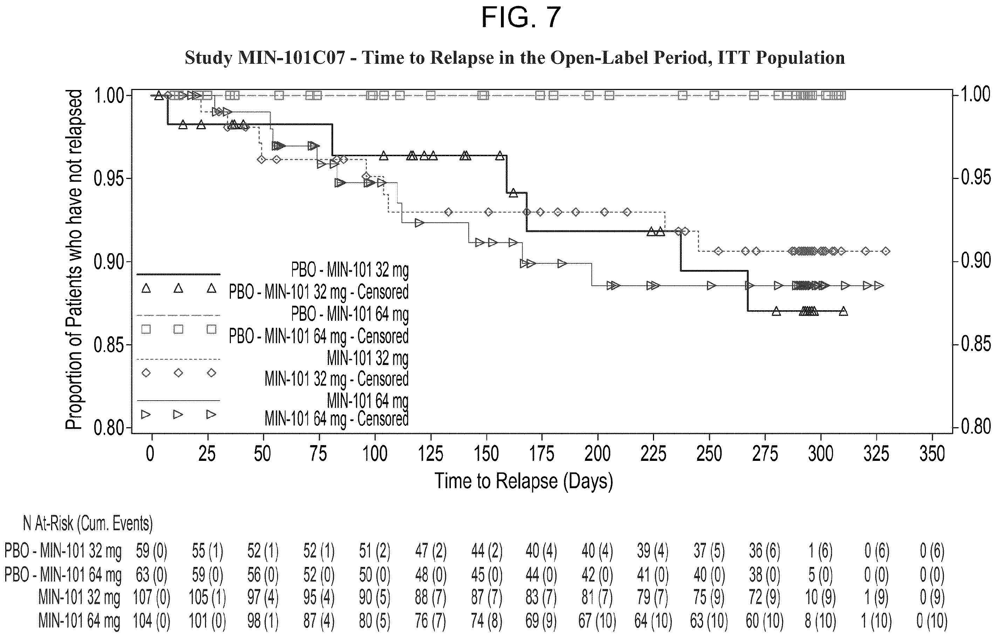

The number (percentage) of patients who experienced relapse in the ITT population was low for all 3 treatment groups (6 [5%] for total placebo to Roluperidone, 9 [8%] for 32 mg Roluperidone, and 10 [10%] for 64 mg Roluperidone) (Table 11). Among the patients who relapsed, the mean number of days to relapse was 260.4 in the placebo to Roluperidone group, 232.4 in the 32 mg Roluperidone group, and 186.7 in the 64 mg Roluperidone group. The time to relapse for the OL period is shown in Table 11 and in FIG. 7.

**Table 11. Study MIN-101C07 - Summary of Rate of Relapse in the Open-Label Period, ITT Population**

| | **Placebo in DB to Roluperidone in OL** | | | **Roluperidone Throughout the Study** | | | **Overall (N = 333)** |
|---|---|---|---|---|---|---|---|
| | **32 mg (N = 59)** | **64 mg (N = 63)** | **Total (N = 122)** | **32 mg (N = 107)** | **64 mg (N = 104)** | **Total (N = 211)** | |
| Patients who experienced relapse | 6 (10%) | 0 | 6 (5%) | 9 (8%) | 10 (10%) | 19 (9%) | 25 (8%) |
| Days to relapse (Kaplan-Meier estimates) | | | | | | | |
| n (number censored) | 59 (53) | 63 (63) | 122 (116) | 107 (98) | 104 (94) | 211 (192) | 333 (308) |
| Mean | 253.6 | NA | 260.4 | 232.4 | 186.7 | 230.9 | 254.1 |
| SE | 6.98 | NA | 3.50 | 4.86 | 3.67 | 3.45 | 2.77 |
| Median | NA | NA | NA | NA | NA | NA | NA |
| 95% CI | NA - NA | NA - NA | NA - NA | NA - NA | NA - NA | NA - NA | NA - NA |
| Q1, Q3 | NA, NA | NA, NA | NA, NA | NA, NA | NA, NA | NA, NA | NA, NA |

| | | | | | | | |
|---|---|---|---|---|---|---|---|
| AE = adverse event; CI = confidence interval; DB = double-blind period; ITT = intent-to-treat; NA = not applicable; OL = open-label; SAP = statistical analysis plan; SE = standard error; Q = quartile. Note: Relapse includes patients who may not have been included in the adjudicated list of relapse cases, but who discontinued due to AEs considered to be associated with relapse as specified in the SAP. | | | | | | | |

### EXAMPLE 4 - Comparison and Analyses of Results Across Studies #1 and #2

The Efficacy data from Study Nos. 1 and 2 were pooled. The majority of patients in the pooled ITT population completed the DB period (502 patients overall, 66.3%), with similar rates in each treatment group (placebo: 69.8%, 32 mg Roluperidone: 65.3%, and 64 mg Roluperidone: 63.8%) (Table 12). The majority (62.7%) of those who completed the DB period entered the OL period. The most common reason for discontinuation during the DB period among patients overall was withdrawal of consent (15.6%), followed by adverse events (7.3%) and lack of efficacy (5.4%) (Table 12). The percentage of patients who withdrew consent was comparable across treatment groups. Approximately twice as many patients in the Roluperidone groups (32 mg: 8.5%; 64 mg: 9.1%) discontinued due to adverse events compared with patients in the placebo group (4.3%). More patients in the placebo group discontinued due to lack of efficacy (6.7%) than in the Roluperidone groups (32 mg: 4.4%; 64 mg: 5.1%). The low number of patients who discontinued due to lack of efficacy or withdrawal of consent probably reflects a low level of worsening of positive symptoms.

**Table 12. Patient Disposition - Double-blind Period, Pooled ITT Population**

| | | **Roluperidone** | | | | |
|---|---|---|---|---|---|---|
| | | **Placebo (N = 255)** n (%) | **32 mg (N = 248)** n (%) | **64 mg (N = 254)** n (%) | **Total (N = 502)** n (%) | **Overall (N = 757)** n (%) |
| Completed double-blind period | | | | | | |
| | Yes | 178 (69.8) | 162 (65.3) | 162 (63.8) | 324 (64.5) | 502 (66.3) |
| | No | 77 (30.2) | 86 (34.7) | 92 (36.2) | 178 (35.5) | 255 (33.7) |

| Reason patient withdrawn from double-blind period | | | | | | |
|---|---|---|---|---|---|---|
| | Adverse event | 11 (4.3) | 21 (8.5) | 23 (9.1) | 44 (8.8) | 55 (7.3) |
| | Death | 0 | 1 (0.4) | 0 | 1 (0.2) | 1 (0.1) |
| | Lost to follow-up | 1 (0.4) | 4 (1.6) | 3 (1.2) | 7 (1.4) | 8 (1.1) |
| | Noncompliance with treatment | 4 (1.6) | 1 (0.4) | 1 (0.4) | 2 (0.4) | 6 (0.8) |
| | Patient moved out of area | 2 (0.8) | 2 (0.8) | 2 (0.8) | 4 (0.8) | 6 (0.8) |
| | Lack of efficacy | 17 (6.7) | 11 (4.4) | 13 (5.1) | 24 (4.8) | 41 (5.4) |
| | Withdrew consent | 35 (13.7) | 40 (16.1) | 43 (16.9) | 83 (16.5) | 118 (15.6) |
| | Protocol violation | 2 (0.8) | 1 (0.4) | 4 (1.6) | 5 (1.0) | 7 (0.9) |
| | Physician decision | 0 | 2 (0.8) | 0 | 2 (0.4) | 2 (0.3) |
| | Other | 5 (2.0) | 3 (1.2) | 3 (1.2) | 6 (1.2) | 11 (1.5) |

| | | | | | | |
|---|---|---|---|---|---|---|
| Note: ITT is defined as all patients who were assigned to study drug and received at least 1 dose of study drug. | | | | | | |

A similar proportion of patients in each OL treatment group entered the OL period from the DB period (range: 61.3% to 65.6%) (Table 13). The most common reason for discontinuation in the OL period among patients was withdrawal of consent (93 patients, 12.3%), followed by adverse event (45 patients, 5.9%) and lack of efficacy (16 patients, 2.1%). The percentages of patients who withdrew consent, discontinued due to adverse events, or discontinued due to lack of efficacy were comparable across treatment groups.

**Table 13. Patient Disposition - Open-label Period, Pooled ITT Population**

| **Time Point** | | **Placebo in DB to Roluperidone in OL** | | | **Roluperidone at Any Time** | | |
|---|---|---|---|---|---|---|---|
| | | **32 mg (N = 128) n (%)** | **64 mg (N = 127) n (%)** | **Total (N = 255) n (%)** | **32 mg (N = 376) n (%)** | **64 mg (N = 381) n (%)** | **Total (N = 757) n (%)** |
| Completed DB period | | 88 (68.8) | 90 (70.9) | 178 (69.8) | 250 (66.5) | 252 (66.1) | 502 (66.3) |
| Entered OL period | | 84 (65.6) | 82 (64.6) | 166 (65.1) | 236 (62.8) | 239 (62.7) | 475 (62.7) |
| Completed OL period | | | | | | | |
| | Yes | 50 (39.1) | 49 (38.6) | 99 (38.8) | 150 (39.9) | 140 (36.7) | 290 (38.3) |
| | No | 34 (26.6) | 33 (26.0) | 67 (26.3) | 86 (22.9) | 99 (26.0) | 185 (24.4) |

| Reason patient withdrawn from OL period | | | | | | | |
|---|---|---|---|---|---|---|---|
| | Adverse event | 8 (6.3) | 5 (3.9) | 13 (5.1) | 21 (5.6) | 24 (6.3) | 45 (5.9) |
| | Lost to follow-up | 4 (3.1) | 3 (2.4) | 7 (2.7) | 5 (1.3) | 9 (2.4) | 14 (1.8) |
| | Noncompliance with treatment | 0 | 1 (0.8) | 1 (0.4) | 1 (0.3) | 1 (0.3) | 2 (0.3) |
| | Patient moved out of area | 0 | 1 (0.8) | 1 (0.4) | 0 | 1 (0.3) | 1 (0.1) |
| | Lack of efficacy | 4 (3.1) | 2 (1.6) | 6 (2.4) | 8 (2.1) | 8 (2.1) | 16 (2.1) |
| | Withdrew consent | 18 (14.1) | 17 (13.4) | 35 (13.7) | 47 (12.5) | 46 (12.1) | 93 (12.3) |
| | Protocol violation | 0 | 0 | 0 | 1 (0.3) | 1 (0.3) | 2 (0.3) |
| | Other | 0 | 4 (3.1) | 4 (1.6) | 3 (0.8) | 9 (2.4) | 12 (1.6) |

| | | | | | | | |
|---|---|---|---|---|---|---|---|
| DB = double-blind; ITT = intent-to-treat; OL = open-label. | | | | | | | |

Demographic and Baseline characteristics were well-balanced among treatment groups in the DB period (Table 14). The majority (overall 85.7%) of patients had moderate severity of negative symptoms of schizophrenia (20 to less than 30 points on NSFS).

**Table 14. Demographics and Baseline Characteristics - Double-blind Period, Pooled ITT Population**

| | | | **Roluperidone** | | | | |
|---|---|---|---|---|---|---|---|
| | | | **Placebo (N = 255)** | **32 mg (N = 248)** | **64 mg (N = 254)** | **Total (N = 502)** | **Overall (N = 757)** |
| Age at informed consent | | | | | | | |
| | N | | 255 | 248 | 254 | 502 | 757 |
| | Mean (SD) | | 40.3 (9.16) | 40.4 (9.67) | 40.7 (9.70) | 40.5 (9.68) | 40.4 (9.50) |
| | Median | | 41.0 | 41.5 | 42.0 | 42.0 | 41.0 |
| | Min, max | | 18, 59 | 18, 59 | 18, 59 | 18, 59 | 18, 59 |
| Age group, n (%) | | | | | | | |
| | ≤ 40 | | 124 (48.6) | 115 (46.4) | 112 (44.1) | 227 (45.2) | 351 (46.4) |
| | > 40 | | 131 (51.4) | 133 (53.6) | 142 (55.9) | 275 (54.8) | 406 (53.6) |
| Sex, n (%) | | | | | | | |
| | Male | | 154 (60.4) | 147 (59.3) | 151 (59.4) | 298 (59.4) | 452 (59.7) |
| | Female | | 101 (39.6) | 101 (40.7) | 103 (40.6) | 204 (40.6) | 305 (40.3) |
| Race, n (%) | | | | | | | |
| | American Indian or Alaska Native | | 0 | 1 (0.4) | 0 | 1 (0.2) | 1 (0.1) |
| | Asian | | 0 | 1 (0.4) | 1 (0.4) | 2 (0.4) | 2 (0.3) |
| | Black or African American | | 20 (7.8) | 19 (7.7) | 18 (7.1) | 37 (7.4) | 57 (7.5) |
| | Native Hawaiian or Other Pacific Islander | | 0 | 2 (0.8) | 1 (0.4) | 3 (0.6) | 3 (0.4) |
| | White | | 235 (92.2) | 225 (90.7) | 234 (92.1) | 459 (91.4) | 694 (91.7) |

| Ethnicity, n (%) | | | | | | | |
|---|---|---|---|---|---|---|---|
| | Hispanic or Latino | | 3 (1.2) | 5 (2.0) | 4 (1.6) | 9 (1.8) | 12 (1.6) |
| | Not Hispanic or Latino | | 169 (66.3) | 165 (66.5) | 167 (65.7) | 332 (66.1) | 501 (66.2) |
| | Not collected^{a} | | 83 (32.5) | 78 (31.5) | 83 (32.7) | 161 (32.1) | 244 (32.2) |
| BMI, mean (SD) (kg/m²) | | | 25.9 (4.24) | 25.5 (4.36) | 25.7 (4.13) | 25.6 (4.24) | 25.7 (4.24) |

| BMI group, n (%) | | | | | | | |
|---|---|---|---|---|---|---|---|
| | < 25 | | 129 (50.6) | 121 (48.8) | 113 (44.5) | 234 (46.6) | 363 (48.0) |
| | | < 18.5 | 3 (1.2) | 6 (2.4) | 5 (2.0) | 11 (2.2) | 14 (1.8) |
| | | 18.5 to < 25 | 126 (49.4) | 115 (46.4) | 108 (42.5) | 223 (44.4) | 349 (46.1) |
| | 25 to < 30 | | 74 (29.0) | 82 (33.1) | 97 (38.2) | 179 (35.7) | 253 (33.4) |
| | ≥ 30 | | 52 (20.4) | 45 (18.1) | 43 (16.9) | 88 (17.5) | 140 (18.5) |

| CYP2D6 metabolizer^{b}, n (%) | | | | | | | |
|---|---|---|---|---|---|---|---|
| | Poor | | 0 | 0 | 0 | 0 | 0 |
| | Normal/Extensive | | 246 (96.5) | 243 (98.0) | 243 (95.7) | 486 (96.8) | 732 (96.7) |
| | Intermediate | | 1 (0.4) | 0 | 2 (0.8) | 2 (0.4) | 3 (0.4) |
| | Ultra-rapid | | 5 (2.0) | 5 (2.0) | 9 (3.5) | 14 (2.8) | 19 (2.5) |
| | Other | | 3 (1.2) | 0 | 0 | 0 | 3 (0.4) |
| Mean (SD) NSFS | | | 24.6 (3.37) | 25.3 (3.67) | 25.3 (3.51) | 25.3 (3.59) | 25.0 (3.53) |
| | | | | | | | |

| | | | **Roluperidone** | | | | |
|---|---|---|---|---|---|---|---|
| | | | **Placebo (N = 255)** | **32 mg (N = 248)** | **64 mg (N = 254)** | **Total (N = 502)** | **Overall (N = 757)** |
| Baseline NSFS score group, n (%) | | | | | | | |
| | < 20 | | 13 (5.1) | 12 (4.8) | 8 (3.1) | 20 (4.0) | 33 (4.4) |
| | ≥ 20 to < 30 | | 222 (87.1) | 206 (83.1) | 221 (87.0) | 427 (85.1) | 649 (85.7) |
| | ≥ 30 | | 20 (7.8) | 30 (12.1) | 25 (9.8) | 55 (11.0) | 75 (9.9) |
| | | ≥ 30 to < 40 | 20 (7.8) | 30 (12.1) | 25 (9.8) | 55 (11.0) | 75 (9.9) |
| | | ≥ 40 to < 49 | 0 | 0 | 0 | 0 | 0 |
| Mean (SD) CGI-S score | | | 4.1 (0.63) | 4.1 (0.62) | 4.1 (0.64) | 4.1 (0.63) | 4.1 (0.63) |

| Baseline CGI-S score group, n (%) | | | | | | | |
|---|---|---|---|---|---|---|---|
| | < 4 | | 36 (14.1) | 29 (11.7) | 36 (14.2) | 65 (12.9) | 101 (13.3) |
| | ≥ 4 | | 219 (85.9) | 219 (88.3) | 218 (85.8) | 437 (87.1) | 656 (86.7) |
| | | 4 to 5 | 213 (83.5) | 211 (85.1) | 210 (82.7) | 421 (83.9) | 634 (83.8) |
| | | ≥ 6 | 6 (2.4) | 8 (3.2) | 8 (3.1) | 16 (3.2) | 22 (2.9) |

| Prior antipsychotics^{c}, n (%) | | | | | | | |
|---|---|---|---|---|---|---|---|
| | Taking antipsychotic medications prior to the study | | 245 (96.1) | 242 (97.6) | 242 (95.3) | 484 (96.4) | 729 (96.3) |
| | Not taking antipsychotic medications prior to the study | | 10 (3.9) | 6 (2.4) | 12 (4.7) | 18 (3.6) | 28 (3.7) |

| | | | | | | | |
|---|---|---|---|---|---|---|---|
| BMI = body mass index; CGI-S = Clinical Global Impression - Severity Scale; CYP2D6 (N = 76) cytochrome P450 2D6; ITT = intent-to-treat; NSFS = PANSS Marder's negative symptoms factor score; SD = standard deviation. ^{a} Ethnicity was not collected in Study #1. ^{b} Genotype was assessed during the first screening visit and was not re-collected during the second. ^{c} A patient who was taking antipsychotic medications prior to the study is defined as a patient who was taking antipsychotic medications that were stopped during the washout period of the pretreatment phase as described in the study protocols. | | | | | | | |

### Prior Medications

Almost all patients were on prior medications (98.4%), a finding seen across treatment groups (> 97% patients in each group). The majority of the patients, as expected, were on antipsychotic medication. Antipsychotic medications taken by > 10% of patients overall were risperidone (40.3%), olanzapine (19.8%), aripiprazole (14.0%), haloperidol (13.6%), and amisulpride (10.8%) and were evenly distributed across treatment groups.

### COMPARISON OF EFFICACY RESULTS OF ALL STUDIES

The integrated analysis supporting the efficacy of Roluperidone as a treatment for the negative symptoms of schizophrenia in adults is presented below. An overview of the results of the integrated efficacy analyses is provided in Table 15. Clinical efficacy of 64 mg Roluperidone administered once daily is demonstrated by the results of the primary efficacy endpoint of NSFS and is supported by results of the PSP Total score, as well as the CGI-S and the CGI-I.

**Table 15. Overview of Roluperidone Integrated Efficacy Analyses - Summary and Change from Baseline in Primary, and Selected Key Endpoints, Week 12 (ITT Populations)**

| | | **Change from Baseline (LS Means [SEM])** | | | **p-value** | |
|---|---|---|---|---|---|---|
| | | **Roluperidone** | | | **Roluperidone vs Placebo** | |
| | | **Placebo (N = 255)** | **32 mg (N = 248)** | **64 mg (N = 254)** | **32 mg** | **64 mg** |
| **Primary Endpoint** | | | | | | |
| NSFS | | -2.6 (0.25) | -3.5 (0.26) | -3.9 (0.26) | **0.023** | **< 0.001** |

| **Key Secondary Endpoint** | | | | | | |
|---|---|---|---|---|---|---|
| PSP Total Score | | 2.8 (0.69) | 3.3 (0.71) | 6.4 (0.69) | 0.614 | **< 0.001** |
| | **Secondary and Exploratory Endpoints** | | | | | |

| PANSS Constructs and Subscales | | | | | | |
|---|---|---|---|---|---|---|
| | Total Score | -3.5 (0.69) | -5.5 (0.71) | -6.3 (0.70) | 0.051 | **0.004** |
| | Negative Symptoms Subscale | -2.8 (0.26) | -3.6 (0.27) | -4.1 (0.27) | 0.050 | **< 0.001** |
| | Positive Symptoms Subscale | 0.2 (0.21) | -0.1 (0.21) | -0.2 (0.21) | 0.394 | **0.190** |
| | General Psychopathology Subscale | -1.1 (0.36) | -2.0 (0.37) | -2.2 (0.37) | 0.088 | **0.038** |
| | NSFS Emotional Experience Score | -1.0 (0.12) | -1.3 (0.13) | -1.6 (0.13) | **0.048** | **< 0.001** |
| | NSFS Emotional Expression Score | -1.7 (0.16) | -2.2 (0.16) | -2.3 (0.16) | 0.051 | **0.011** |

| PSP Domains | | | | | | |
|---|---|---|---|---|---|---|
| | Self-Care | -0.2 (0.05) | -0.3 (0.05) | -0.3 (0.05) | 0.112 | **0.167** |
| | Socially Useful Activities | -0.2 (0.05) | -0.3 (0.05) | -0.4 (0.05) | 0.665 | **0.005** |
| | Personal and Social Relationships | -0.2 (0.05) | -0.3 (0.05) | -0.4 (0.05) | 0.138 | **0.024** |
| | Disturbing and Aggressive Behavior | -0.0 (0.04) | -0.1 (0.04) | -0.0 (0.04) | 0.258 | **0.496** |
| Clinical Global Impression of Severity | | -0.2 (0.05) | -0.3 (0.05) | -0.4 (0.05) | 0.084 | **0.007** |
| Clinical Global Impression of Improvement^{b} | | 3.6 (0.06) | 3.5 (0.07) | 3.4 (0.07) | 0.760 | **0.037** |

| | | **Change from Baseline (LS Means [SEM])** | | | **p-value** | |
|---|---|---|---|---|---|---|
| | | **Roluperidone** | | | **Roluperidone vs Placebo** | |
| | | **Placebo (N = 255)** | **32 mg (N = 248)** | **64 mg (N = 254)** | **32 mg** | **64 mg** |
| Responder Analysis^{c} | | | | | | |
| | Number of Patients with 20% Reduction in NSFS at Week 12 | 43/182 (24%) | 51/167 (31%) | 68/176 (39%) | 0.136 | **0.002** |
| | Number of Patients with 20% Reduction in PANSS Total Score at Week 12 | 16/182 (9%) | 24/167 (14%) | 34/176 (19%) | 0.100 | **0.005** |
| | Number of Patients with 7-Point Improvement in PSP Total Score at Week 12 | 53/180 (29%) | 54/166 (33%) | 76/176 (43%) | 0.442 | **0.005** |

| | | | | | | |
|---|---|---|---|---|---|---|
| CGI-S = Clinical Global Impression - Severity Scale, LS = least square; MMRM = mixed model of repeated measures; NSFS = Negative Symptoms Factor Score; PANSS = Positive and Negative Syndrome Scale, PSP = Personal and Social Performance; SEM = standard error of mean. ^{a} Based on Cohen's d. ^{b} Observed data analyzed using MMRM with Baseline CGI-S score as covariate. ^{c} Effect size is based on Cohen's W. | | | | | | |

### NSFS

The primary efficacy endpoint was the change from Baseline in NSFS to Week 12 in the pooled ITT population. At Baseline, the mean (SD) NSFS scores were similar across the treatment groups, with 24.6 (3.37) for placebo, 25.3 (3.67) for 32 mg Roluperidone, and 25.3 (3.51) for 64 mg Roluperidone. At Week 12, there was a statistically significant improvement shown as LS mean decrease from Baseline compared with placebo in both the 32 mg Roluperidone group (-3.5; 95% CI: -4.0, -2.9; p ≤ 0.023) and the 64 mg Roluperidone group (-3.9; 95% CI: -4.4, -3.4, p < 0.001) (Table 16).

Unadjusted p-values for the 64 mg Roluperidone group at Weeks 2, 4, and 8 demonstrated early and consistent significantly different decreases from Baseline in NSFS scores compared with placebo (Table 16 ). A similar trend was seen for the unadjusted p-values for the 32 mg Roluperidone group, starting at Week 4. Similar results for the change in NSFS score were seen for the pooled mITT population.

**Table 16. Summary and Change from Baseline of NSFS Score by Visit - Double-blind Period, Pooled ITT Population**

| **Time Point** | | **Roluperidone** | | | |
|---|---|---|---|---|---|
| | | **Placebo (N = 255)** | **32 mg (N = 248)** | **64 mg (N = 254)** | **Total (N = 502)** |
| Baseline^{a} | | | | | |
| | N | 255 | 248 | 254 | 502 |
| | Mean (SD) | 24.6 (3.37) | 25.3 (3.67) | 25.3 (3.51) | 25.3 (3.59) |
| | Median | 24.0 | 25.0 | 25.0 | 25.0 |
| | Min, max | 17, 35 | 16, 39 | 17, 36 | 16, 39 |

| Change from Baseline to Week 2 | | | | | |
|---|---|---|---|---|---|
| | N | 238 | 233 | 233 | 466 |
| | Mean (SD) | -0.9 (2.32) | -1.1 (2.15) | -1.4 (2.34) | -1.3 (2.25) |
| | Median | -1.0 | -1.0 | -1.0 | -1.0 |
| | Min, max | -11, 5 | -9, 5 | -13, 7 | -13, 7 |
| | LS mean (SE) | -1.0 (0.15) | -1.1 (0.15) | -1.4 (0.15) | |
| | 95% CI of LS mean | -1.2, -0.7 | -1.4, -0.8 | -1.7, -1.1 | |
| | Difference (95% CI) in LS mean (vs placebo) | | -0.2 (-0.6, 0.3) | -0.4 (-0.8, - 0.0) | |
| | p-value (vs placebo) | | 0.459 | 0.033 | |

| **Time Point** | | **Roluperidone** | | | |
|---|---|---|---|---|---|
| | | **Placebo** (N **= 255)** | **32 mg (N = 248)** | **64 mg (N = 254)** | **Total (N = 502)** |
| Change from Baseline to Week 4 | | | | | |
| | N | 224 | 208 | 212 | 420 |
| | Mean (SD) | -1.4 (2.96) | -2.2 (2.53) | -2.6 (2.73) | -2.4 (2.64) |
| | Median | -1.0 | -2.0 | -2.0 | -2.0 |
| | Min, max | -14, 8 | -15, 3 | -12, 4 | -15, 4 |
| | LS mean (SE) | -1.4 (0.18) | -2.0 (0.19) | -2.5 (0.19) | |
| | 95% CI of LS mean | -1.7, -1.0 | -2.4, -1.7 | -2.9, -2.1 | |
| | Difference (95% CI) in LS mean (vs placebo) | | -0.7 (-1.2, - 0.2) | -1.1 (-1.6, -0.6) | |
| | p-value (vs placebo) | | 0.010 | < 0.001 | |

| Change from Baseline to Week 8 | | | | | |
|---|---|---|---|---|---|
| | N | 194 | 185 | 188 | 373 |
| | Mean (SD) | -2.3 (3.46) | -3.2 (3.21) | -3.6 (3.28) | -3.4 (3.25) |
| | Median | -2.0 | -3.0 | -3.0 | -3.0 |
| | Min, max | -16, 8 | -20, 4 | -15, 5 | -20, 5 |
| | LS mean (SE) | -2.2 (0.23) | -3.0 (0.23) | -3.4 (0.23) | |
| | 95% CI of LS mean | -2.6, -1.7 | -3.4, -2.5 | -3.9, -3.0 | |
| | Difference (95% CI) in LS mean (vs placebo) | | -0.8 (-1.5, - 0.2) | -1.2 (-1.9, -0.6) | |
| | p-value (vs placebo) | | 0.012 | < 0.001 | |

| Change from Baseline to Week 12 | | | | | |
|---|---|---|---|---|---|
| | N | 182 | 167 | 176 | 343 |
| | Mean (SD) | -2.9 (3.91) | -3.6 (3.08) | -4.1 (3.64) | -3.8 (3.39) |
| | Median | -2.0 | -4.0 | -3.5 | -4.0 |
| | Min, max | -19, 6 | -14, 2 | -17, 5 | -17, 5 |
| | LS mean (SE) | -2.6 (0.25) | -3.5 (0.26) | -3.9 (0.26) | |
| | 95% CI of LS mean | -3.1, -2.1 | -4.0, -2.9 | -4.4, -3.4 | |
| | Difference (95% CI) in LS mean (vs placebo) | | -0.8 (-1.6, - 0.1) | -1.3 (-2.0, -0.6) | |
| | p-value (vs placebo) | | 0.023 | < 0.001 | |

| | | | | | |
|---|---|---|---|---|---|
| CI = confidence interval; ITT = intent-to-treat; LS = least square; NSFS = PANSS Marder's negative symptoms factor score; PANSS = Positive and Negative Syndrome Scale; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

At Week 12, there was a larger magnitude of change in the NSFS for the 64 mg Roluperidone group (19% and 13% showing moderate (magnitude of change 6-8) and high improvements (magnitude of change >8), respectively; total of 32%) compared with the 32 mg Roluperidone (18% and 5% showing moderate and high improvements, respectively; total of 23%) and placebo groups (9% and 8% showing moderate and high improvements, respectively; total of 17%). In line with this observation, a responder analysis demonstrated that there were statistically significantly more patients in the 64 mg Roluperidone group compared to the placebo group who had a reduction in NSFS score from Baseline of 20% (p ≤ 0.002) and of 30% (p ≤ 0.037).

Consistent with the robust NSFS outcomes seen at Week 12, the 64 mg Roluperidone group showed a statistically significant improvement in change from Baseline to Week 12 compared with placebo for PANSS Marder's positive symptoms (p ≤ 0.004). No statistically significant difference from placebo was found for change from Baseline in scores for disorganized thought uncontrolled hostility/excitement, or anxiety/depression for either Roluperidone group.

### PANSS TOTAL SCORE

At Baseline, the mean (SD) PANSS total scores were comparable across the treatment groups (78.1 [(10.52], 80.3 [11.25], and 79.3 [10.67] for the placebo, 32 mg Roluperidone, and 64 mg Roluperidone groups, respectively). At Week 12, there was a greater LS mean change from Baseline compared with placebo for both the 32 mg Roluperidone group (-1.9; 95% CI: -3.9, 0.0; p ≤ 0.051) and the 64 mg Roluperidone group (-2.8; 95% CI: -4.7, -0.9; p ≤ 0.004) (Table 17).

Unadjusted p-values for the 64 mg Roluperidone group at Weeks 4 and 8 demonstrated early and consistent significantly larger improvements from Baseline in PANSS total score compared with placebo (Table 17). Unadjusted p-values for the 32 mg Roluperidone group were only statistically significant at Week 4.

A responder analysis demonstrated that there were statistically significantly more patients in the 64 mg Roluperidone group with 20% (p ≤ 0.005) reduction in PANSS total score from Baseline compared with the placebo group.

**Table 17. Summary and Change from Baseline in PANSS Total Score, by Visit - Double- blind Period, Pooled ITT Population**

| **Time Point** | | **Roluperidone** | | | |
|---|---|---|---|---|---|
| | | **Placebo (N = 255)** | **32 mg (N = 248)** | **64 mg (N = 254)** | **Total** (N **= 502)** |
| Baseline^{a} | | | | | |
| | N | 255 | 248 | 254 | 502 |
| | Mean (SD) | 78.1 (10.52) | 80.3 (11.25) | 79.3 (10.67) | 79.8 (10.96) |
| | Median | 78.0 | 81.0 | 79.0 | 80.0 |
| | Min, max | 56, 118 | 53, 117 | 56, 109 | 53, 117 |

| Change from Baseline to Week 2 | | | | | |
|---|---|---|---|---|---|
| | N | 238 | 233 | 233 | 466 |
| | Mean (SD) | -1.3 (6.20) | -1.8 (6.62) | -2.2 (7.15) | -2.0 (6.89) |
| | Median | -2.0 | -2.0 | -2.0 | -2.0 |
| | Min, max | -20, 28 | -23, 33 | -29, 35 | -29, 35 |
| | LS mean (SE) | -1.3 (0.43) | -1.7 (0.44) | -2.2 (0.44) | |
| | 95% CI of LS mean | -2.1, -0.5 | -2.6, -0.9 | -3.1, -1.4 | |
| | Difference (95% CI) in LS mean (vs placebo) | | -0.4 (-1.6, 0.8) | -0.9 (-2.1, 0.3) | |
| | p-value (vs placebo) | | 0.487 | 0.125 | |

| Change from Baseline to Week 4 | | | | | |
|---|---|---|---|---|---|
| | N | 224 | 208 | 212 | 420 |
| | Mean (SD) | -2.3 (7.82) | -4.4 (7.09) | -5.1 (7.16) | -4.8 (7.13) |
| | Median | -3.0 | -4.0 | -5.0 | -5.0 |
| | Min, max | -24, 26 | -26, 22 | -31, 18 | -31, 22 |
| | LS mean (SE) | -2.0 (0.51) | -3.6 (0.53) | -4.5 (0.52) | |
| | 95% CI of LS mean | -3.0, -1.0 | -4.6, -2.6 | -5.5, -3.5 | |
| | Difference (95% CI) in LS mean (vs placebo) | | -1.6 (-3.1, -0.2) | -2.5 (-4.0, -1.1) | |
| | p-value (vs placebo) | | 0.028 | < 0.001 | |

| Change from Baseline to Week 8 | | | | | |
|---|---|---|---|---|---|
| | N | 194 | 185 | 188 | 373 |
| | Mean (SD) | -4.2 (8.85) | -6.2 (7.89) | -6.8 (8.79) | -6.5 (8.35) |
| | Median | -5.0 | -6.0 | -6.0 | -6.0 |
| | Min, max | -31, 27 | -37, 18 | -34, 36 | -37, 36 |
| | LS mean (SE) | -3.2 (0.64) | -4.9 (0.66) | -5.7 (0.65) | |
| | 95% CI of LS mean | -4.4, -1.9 | -6.2, -3.6 | -6.9, -4.4 | |
| | Difference (95% CI) in LS mean (vs placebo) | | -1.7 (-3.5, 0.1) | -2.5 (-4.3, -0.7) | |
| | p-value (vs placebo) | | 0.061 | 0.007 | |

| **Time Point** | | Roluperidone | | | |
|---|---|---|---|---|---|
| | | **Placebo (N = 255)** | **32 mg (N = 248)** | **64 mg (N = 254)** | **Total (N = 502)** |
| Change from Baseline to Week 12 | | | | | |
| | N | 182 | 167 | 176 | 343 |
| | Mean (SD) | -4.9 (8.83) | -6.9 (7.77) | -7.7 (9.43) | -7.3 (8.66) |
| | Median | -5.0 | -7.0 | -7.0 | -7.0 |
| | Min, max | -33, 19 | -28, 15 | -36, 25 | -36, 25 |
| | LS mean (SE) | -3.5 (0.69) | -5.5 (0.71) | -6.3 (0.70) | |
| | 95% CI of LS mean | -4.9, -2.2 | -6.9, -4.1 | -7.7, -5.0 | |
| | Difference (95% CI) in LS mean (vs placebo) | | -1.9 (-3.9, 0.0) | -2.8 (-4.7, -0.9) | |
| | p-value (vs placebo) | | 0.051 | 0.004 | |

| | | | | | |
|---|---|---|---|---|---|
| CI = confidence interval; ITT = intent-to-treat; LS = least square; PANSS = Positive and Negative Syndrome Scale; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

### PANSS POSITIVE SUBSCALE SCORES

At Baseline, the mean (SD) PANSS positive subscale scores were comparable across the treatment groups (14.2 [3.48], 14.7 [3.83], and 14.1 [3.72] for the placebo, 32 mg Roluperidone, and 64 mg Roluperidone groups, respectively.

At Week 12, the LS mean change from Baseline showed a stable PANSS positive subscale score and a trend toward improvement compared with placebo for both the 32 mg Roluperidone group (-0.3; 95% CI: -0.8, 0.3; p ≤ 0.394) and 64 mg Roluperidone group (-0.4; 95% CI: -1.0, 0.2; p ≤ 0.190) (Table 18). The PANSS positive subscale scores at Baseline and after 12 weeks of treatment are consistent with the patient selection criteria in being mild and stable, and more importantly, remained as such at Week 12.

**Table 18. Summary and Change from Baseline in PANSS Positive Subscale Score, by Visit - Double-blind Period, Pooled ITT Population**

| Time Point | | **Roluperidone** | | | |
|---|---|---|---|---|---|
| | | **Placebo (N** = **255)** | **32 mg (N = 248)** | **64 mg (N = 254)** | **Total (N = 502)** |
| Baseline^{a} | | | | | |
| | N | 255 | 248 | 254 | 502 |
| | Mean (SD) | 14.2 (3.48) | 14.7 (3.83) | 14.1 (3.72) | 14.4 (3.78) |
| | Median | 14.0 | 15.0 | 14.0 | 14.0 |
| | Min, max | 7, 23 | 7, 25 | 7, 27 | 7, 27 |

| Change from Baseline (BL) to Week 2 | | | | | |
|---|---|---|---|---|---|
| | N | 238 | 233 | 233 | 466 |
| | Mean (SD) | -0.0 (2.23) | 0.2 (2.71) | -0.1 (2.85) | 0.1 (2.78) |
| | Median | 0.0 | 0.0 | 0.0 | 0.0 |
| | Min, max | -5, 9 | -7, 17 | -8, 17 | -8, 17 |
| | LS mean (SE) | -0.0 (0.17) | 0.3 (0.17) | -0.1 (0.17) | |
| | 95% CI of LS mean | -0.4, 0.3 | -0.1, 0.6 | -0.4, 0.2 | |
| | Difference (95% CI) in LS mean (vs placebo) | | 0.3 (-0.2, 0.8) | -0.1 (-0.5, 0.4) | |
| | p-value (vs placebo) | | 0.202 | 0.732 | |

| Change from BL to Week 4 | | | | | |
|---|---|---|---|---|---|
| | N | 224 | 208 | 212 | 420 |
| | Mean (SD) | 0.1 (2.71) | -0.4 (2.49) | -0.4 (2.49) | -0.4 (2.49) |
| | Median | 0.0 | 0.0 | -1.0 | 0.0 |
| | Min, max | -7, 11 | -8, 11 | -7, 9 | -8, 11 |
| | LS mean (SE) | 0.2 (0.18) | -0.1 (0.18) | -0.2 (0.18) | |
| | 95% CI of LS mean | -0.1, 0.6 | -0.4, 0.3 | -0.6, 0.1 | |
| | Difference (95% CI) in LS mean (vs placebo) | | -0.3 (-0.8, 0.2) | -0.5 (-1.0, 0.0) | |
| | p-value (vs placebo) | | 0.253 | 0.070 | |

| Change from BL to Week 8 | | | | | |
|---|---|---|---|---|---|
| | N | 194 | 185 | 188 | 373 |
| | Mean (SD) | -0.1 (2.57) | -0.4 (2.50) | -0.6 (2.61) | -0.5 (2.55) |
| | Median | 0.0 | 0.0 | -1.0 | 0.0 |
| | Min, max | -6, 9 | -8, 8 | -8, 13 | -8, 13 |
| | LS mean (SE) | 0.2 (0.19) | -0.1 (0.20) | -0.3 (0.20) | |
| | 95% CI of LS mean | -0.2, 0.6 | -0.5, 0.3 | -0.7, 0.1 | |
| | Difference (95% CI) in LS mean (vs placebo) | | -0.3 (-0.8, 0.3) | -0.5 (-1.0, 0.0) | |
| | p-value (vs placebo) | | 0.312 | 0.070 | |

| | | | | | |
|---|---|---|---|---|---|
| | | | | | |

| **Time Point** | | **Roluperidone** | | | |
|---|---|---|---|---|---|
| | | **Placebo (N = 255)** | **32 mg (N = 248)** | **64 mg (N = 254)** | **Total (N = 502)** |
| Change from Baseline to Week 12 | | | | | |
| | N | 18 2 | 167 | 176 | 343 |
| | Mean (SD) | -0.1 (2.60) | -0.6 (2.37) | -0.5 (2.88) | -0.6 (2.64) |
| | Median | 0.0 | 0.0 | 0.0 | 0.0 |
| | Min, max | -6, 10 | -6, 7 | -9, 9 | -9, 9 |
| | LS mean (SE) | 0.2 (0.21) | -0.1 (0.21) | -0.2 (0.21) | |
| | 95% CI of LS mean | -0.2, 0.6 | -0.5, 0.3 | -0.6, 0.2 | |
| | Difference (95% CI) in LS mean (vs placebo) | | -0.3 (-0.8, 0.3) | -0.4 (-1.0, 0.2) | |
| | p-value (vs placebo) | | 0.394 | 0.190 | |

| | | | | | |
|---|---|---|---|---|---|
| CI = confidence interval; ITT = intent-to-treat; LS = least square; PANSS = Positive and Negative Syndrome Scale; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

### PANSS NEGATIVE SUBSCALE SCORE

At Baseline, the mean (SD) PANSS negative subscale scores were comparable across the treatment groups (26.5 [3.51], 27.0 [3.61], and 27.1 [3.51] for the placebo, 32 mg Roluperidone, and 64 mg Roluperidone groups, respectively). At Week 12, there was a greater LS mean change from Baseline compared with placebo for both the 32 mg Roluperidone group (-0.7; 95% CI: -1.5, 0.0; p ≤ 0.05) and the 64 mg Roluperidone group (-1.3; 95% CI: -2.0, -0.6; p ≤ 0.050); the difference was statistically significantly superior for the 64 mg Roluperidone group and trended toward statistical significance for the 32 mg Roluperidone group (Table 19).

Unadjusted p-values for the 64 mg Roluperidone group at Weeks 4 and 8 demonstrated early and consistent significantly different changes from Baseline in PANSS negative subscale score compared with placebo (Table 19). Unadjusted p-values in the 32 mg Roluperidone group were statistically significant at Weeks 4 and 8.

**Table 19. Summary and Change from Baseline in PANSS Negative Subscale Score, by Visit - Double-blind Period, Pooled ITT Population**

| **Time Point** | | **Roluperidone** | | | |
|---|---|---|---|---|---|
| | | **Placebo (N = 255)** | **32 mg (N = 248)** | **64 mg (N = 254)** | **Total (N = 502)** |
| **Baseline^{a}** | | | | | |
| | N | 255 | 248 | 254 | 502 |
| | Mean (SD) | 26.5 (3.51) | 27.0 (3.61) | 27.1 (3.51) | 27.1 (3.56) |
| | Median | 26. 0 | 26.5 | 27.0 | 27.0 |
| | Min, max | 20, 40 | 21, 43 | 21, 39 | 21, 43 |

| Change from Baseline to Week 2 | | | | | |
|---|---|---|---|---|---|
| | N | 238 | 233 | 233 | 466 |
| | Mean (SD) | -1.0 (2.23) | -1.2 (2.11) | -1.4 (2.28) | -1.3 (2.20) |
| | Median | - 1.0 | -1.0 | -1.0 | -1.0 |
| | Min, max | -12, 5 | -11, 4 | -13, 8 | -13, 8 |
| | LS mean (SE) | -1.0 (0.14) | -1.2 (0.14) | -1.4 (0.14) | |
| | 95% CI of LS mean | -1.3, -0.8 | -1.4, -0.9 | -1.7, -1.1 | |
| | Difference (95% CI) in LS | | -0.1 | -0.4 | |
| | mean (vs placebo) | | (-0.5, 0.3) | (-0.8, 0.0) | |
| | p-value (vs placebo) | | 0.576 | 0.070 | |

| Change from Baseline to Week 4 | | | | | |
|---|---|---|---|---|---|
| | N | 224 | 208 | 212 | 420 |
| | Mean (SD) | -1.5 (2.94) | -2.3 (2.67) | -2.8 (2.82) | -2.5 (2.75) |
| | Median | - 1.0 | -2.0 | -2.0 | -2.0 |
| | Min, max | -14, 8 | -17, 3 | -12, 5 | -17, 5 |
| | LS mean (SE) | -1.5 (0.19) | -2.1 (0.19) | -2.6 (0.19) | |
| | 95% CI of LS mean | -1.8, -1.1 | -2.5, -1.7 | -3.0, -2.3 | |
| **Time Point** | | **Roluperidone** | | | |
| | | **Placebo (N = 255)** | **32** mg (N = **248)** | **64 mg** (N **= 254)** | **Total** (N = **502)** |
| | Difference (95% CI) in LS | | -0.6 | -1.2 | |
| | mean (vs placebo) | | (-1.1, - 0.1) | (-1.7, -0.6) | |
| | p-value (vs placebo) | | 0.024 | < 0.001 | |

| Change from Baseline to Week 8 | | | | | |
|---|---|---|---|---|---|
| | N | 194 | 185 | 188 | 373 |
| | Mean (SD) | -2.6 (3.45) | -3.2 (3.46) | -3.9 (3.27) | -3.6 (3.38) |
| | Median | -2.0 | -3.0 | -3.0 | -3.0 |
| | Min, max | -19, 9 | -25, 3 | -20, 5 | -25, 5 |
| | LS mean (SE) | -2.4 (0.23) | -3.1 (0.24) | -3.7 (0.23) | |
| | 95% CI of LS mean | -2.9, -1.9 | -3.5, -2.6 | -4.1, -3.2 | |
| | Difference (95% CI) in LS mean (vs placebo) | | -0.7 (-1.3, - 0.0) | -1.3 (-1.9, -0.6) | |
| | p-value (vs placebo) | | 0.039 | < 0.001 | |

| Change from Baseline to Week 12 | | | | | |
|---|---|---|---|---|---|
| | N | 182 | 167 | 176 | 343 |
| | Mean (SD) | -3.1 (3.85) | -3.6 (3.37) | -4.4 (3.75) | -4.0 (3.59) |
| | Median | -3.0 | -3.0 | -4.0 | -3.0 |
| | Min, max | -22, 6 | -16, 3 | -21, 2 | -21, 3 |
| | LS mean (SE) | -2.8 (0.26) | -3.6 (0.27) | -4.1 (0.27) | |
| | 95% CI of LS mean | -3.3, -2.3 | -4.1, -3.0 | -4.6, -3.6 | |
| | Difference (95% CI) in LS mean (vs placebo) | | -0.7 (-1.5, 0.0) | -1.3 (-2.0, -0.6) | |
| | p-value (vs placebo) | | 0.050 | < 0.001 | |

| | | | | | |
|---|---|---|---|---|---|
| CI = confidence interval; ITT = intent-to-treat; LS = least square; PANSS = Positive and Negative Syndrome Scale; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

### PSP TOTAL SCORE

The results of the analysis of change from Baseline in PSP Total score followed the same trend as the results for the change from Baseline in PANSS Marder NSFS for the 64 mg Roluperidone group, showing a statistically significant improvement compared with placebo at Week 12. At Baseline, the mean (SD) PSP Total scores were comparable across the treatment groups (52.3 [12.06], 52.7 [12.23], and 51.9 [11.92] for the placebo, 32 mg Roluperidone, and 64 mg Roluperidone groups, respectively). At Week 12, there was a greater LS mean change from Baseline compared with placebo for both the 32 mg Roluperidone group (0.5; 95% CI: -1.4, 2.4; p ≤ 0.614) and the 64 mg Roluperidone group (3.6; 95% CI: 1.7, 5.5; p ≤ 0.001); the difference was statistically significant superior for the 64 mg Roluperidone group and did not reach statistical significance for the 32 mg Roluperidone group (Table 20). Unadjusted p-values for the 64 mg Roluperidone group at Weeks 4 and 8 demonstrated early and consistent statistically significant increases (improvement) from Baseline in PSP Total score compared with placebo (Table 20). Similar results for the change in PSP Total score were seen for the pooled mITT population.

**Table 20. Summary and Change from Baseline in PSP Total Score, by Visit - Double- blind Period, Pooled ITT Population**

| **Time Point** | | **Placebo (N = 255)** | **Roluperidone** | | |
|---|---|---|---|---|---|
| | | | **32 mg (N = 248)** | **64 mg (N = 254)** | **Total (N = 502)** |
| Baseline (BL)^{a} | | | | | |
| | N | 254 | 248 | 254 | 502 |
| | Mean (SD) | 52.3 (12.06) | 52.7 (12.23) | 51.9 (11.92) | 52.3 (12.07) |
| | Median | 50.0 | 50.0 | 50.0 | 50.0 |
| | Min, max | 21, 80 | 20, 100 | 15, 80 | 15, 100 |

| Change from BL to Week 4 | | | | | |
|---|---|---|---|---|---|
| | N | 222 | 208 | 212 | 420 |
| | Mean (SD) | 1.0 (7.23) | 1.5 (7.87) | 3.1 (8.33) | 2.3 (8.14) |
| | Median | 0.0 | 0.0 | 1.0 | 0.0 |
| | Min, max | -30, 30 | -50, 25 | -30, 30 | -50, 30 |
| | LS mean (SE) | 0.9 (0.51) | 1.6 (0.52) | 3.1 (0.52) | |
| | 95% CI of LS mean | -0.1, 1.9 | 0.6, 2.7 | 2.1, 4.1 | |
| | Difference (95% CI) in LS mean (vs placebo) | | 0.7 (-0.7, 2.1) | 2.1 (0.7, 3.6) | |

| **Time Point** | | Roluperidone | | | |
|---|---|---|---|---|---|
| | | **Placebo (N = 255)** | **32 mg (N = 248)** | **64 mg (N = 254)** | **Total (N = 502)** |
| | p-value (vs placebo) | | 0.328 | 0.003 | |

| Change from Baseline to Week 8 | | | | | |
|---|---|---|---|---|---|
| | N | 131 | 127 | 128 | 255 |
| | Mean (SD) | 3.2 (7.71) | 3.6 (7.04) | 4.8 (6.61) | 4.2 (6.84) |
| | Median | 2.0 | 2.0 | 3.0 | 3.0 |
| | Min, max | -20, 32 | -15, 25 | -7, 30 | -15, 30 |
| | LS mean (SE) | 2.3 (0.60) | 2.9 (0.62) | 4.9 (0.61) | |
| | 95% CI of LS mean | 1.1, 3.5 | 1.7, 4.1 | 3.7, 6.1 | |
| | Difference (95% CI) in LS mean (vs placebo) | | 0.6 (-1.0, 2.3) | 2.6 (0.9, 4.3) | |
| | p-value (vs placebo) | | 0.452 | 0.003 | |

| Change from Baseline to Week 12 | | | | | |
|---|---|---|---|---|---|
| | N | 179 | 166 | 176 | 342 |
| | Mean (SD) | 3.3 (8.95) | 3.5 (10.03) | 6.4 (10.32) | 5.0 (10.27) |
| | Median | 2.0 | 2.0 | 4.5 | 3.0 |
| | Min, max | -40, 22 | -40, 26 | -50, 40 | -50, 40 |
| | LS mean (SE) | 2.8 (0.69) | 3.3 (0.71) | 6.4 (0.69) | |
| | 95% CI of LS mean | 1.5, 4.2 | 1.9, 4.7 | 5.1, 7.8 | |
| | Difference (95% CI) in LS mean (vs placebo) | | 0.5 (-1.4, 2.4) | 3.6 (1.7, 5.5) | |
| | p-value (vs placebo) | | 0.614 | < 0.001 | |

| | | | | | |
|---|---|---|---|---|---|
| CI = confidence interval; ITT = intent-to-treat; LS = least square; PSP = Personal and Social Performance; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

There was a larger response in the 64 mg Roluperidone group, 7% and 36% showing moderate improvements (magnitude of change 7 - 9) to high improvements (magnitude of change ≥ 10), respectively; (total of 43%) compared with the 32 mg Roluperidone (7% and 26% showing moderate to high improvements, respectively; total of 33%) and placebo (5% and 25% showing moderate to high improvements, respectively; total of 30%) groups. In line with this observation, a responder analysis demonstrated that there were statistically significantly more patients in the 64 mg Roluperidone group (p ≤ 0.005) with 7-point increase in PSP Total score from Baseline compared with the placebo group.

### CGI-S

At Baseline, the mean (SD) CGI-S scores were comparable across the treatment groups (4.1 [0.63], 4.1 [0.62], and 4.1 [0.64] for the placebo, 32 mg Roluperidone, and 64 mg Roluperidone groups, respectively). At Week 12, there was a greater LS mean change from Baseline compared with placebo for both the 32 mg Roluperidone group (-0.1; 95% CI: -0.2, 0.0; p ≤ 0.084) and the 64 mg Roluperidone group (-0.2; 95% CI: -0.3, -0.0; p ≤ 0.007); the difference was statistically significantly superior for the 64 mg Roluperidone group (Table 21).

Unadjusted p-values for the 64 mg Roluperidone group at Weeks 4 and 8 demonstrated early and consistent significantly different changes from Baseline in CGI-S score compared with placebo (Table 21).

**Table 21. Summary and Change from Baseline in CGI-S Score, by Visit - Double-blind Period, Pooled ITT Population**

| **Time Point** | | **Roluperidone** | | | |
|---|---|---|---|---|---|
| | | **Placebo (N = 255)** | **32 mg (N = 248)** | **64 mg (N = 254)** | **Total (N = 502)** |
| Baseline (BL)^{a} | | | | | |
| | N | 255 | 248 | 254 | 502 |
| | Mean (SD) | 4.1 (0.63) | 4.1 (0.62) | 4.1 (0.64) | 4.1 (0.63) |
| | Median | 4.0 | 4.0 | 4.0 | 4.0 |
| | Min, max | 2, 6 | 3, 6 | 3, 6 | 3, 6 |

| Change from BL to Week 2 | | | | | |
|---|---|---|---|---|---|
| | N | 164 | 168 | 169 | 337 |
| | Mean (SD) | -0.1 (0.51) | -0.1 (0.47) | -0.1 (0.55) | -0.1 (0.51) |
| | Median | 0.0 | 0.0 | 0.0 | 0.0 |
| | Min, max | -2, 2 | -2, 2 | -3, 1 | -3, 2 |
| | LS mean (SE) | -0.1 (0.04) | -0.1 (0.04) | -0.1 (0.04) | |
| | 95% CI of LS mean | -0.1, -0.0 | -0.1, 0.0 | -0.2, -0.0 | |
| | Difference (95% CI) in LS mean (vs placebo) | | 0.0 (-0.1, 0.1) | -0.0 (-0.1, 0.1) | |
| | p-value (vs placebo) | | 0.752 | 0.826 | |

| Change from BL to Week 4 | | | | | |
|---|---|---|---|---|---|
| | N | 224 | 208 | 212 | 420 |
| | Mean (SD) | -0.0 (0.49) | -0.1 (0.50) | -0.3 (0.56) | -0.2 (0.54) |
| | Median | 0.0 | 0.0 | 0.0 | 0.0 |
| | Min, max | -2, 2 | -2, 1 | -3, 2 | -3, 2 |
| | LS mean (SE) | -0.0 (0.03) | -0.1 (0.03) | -0.2 (0.03) | |
| | 95% CI of LS mean | -0.1, 0.0 | -0.2, -0.0 | -0.3, -0.2 | |
| | Difference (95% CI) in LS mean (vs placebo) | | -0.1 (-0.2, 0.0) | -0.2 (-0.3, -0.1) | |
| | p-value (vs placebo) | | 0.151 | < 0.001 | |

| Change from BL to Week 8 | | | | | |
|---|---|---|---|---|---|
| | N | 194 | 185 | 188 | 373 |
| | Mean (SD) | -0.2 (0.59) | -0.3 (0.58) | -0.3 (0.66) | -0.3 (0.62) |
| | Median | 0.0 | 0.0 | 0.0 | 0.0 |
| | Min, max | -2, 2 | -3, 1 | -3, 1 | -3, 1 |
| | LS mean (SE) | -0.1 (0.04) | -0.2 (0.04) | -0.3 (0.04) | |
| | 95% CI of LS mean | -0.2, -0.0 | -0.3, -0.1 | -0.4, -0.2 | |
| | Difference (95% CI) in LS mean (vs placebo) | | -0.1 (-0.2, 0.1) | -0.2 (-0.3, -0.1) | |
| | p-value (vs placebo) | | 0.364 | 0.003 | |
| **Time Point** | | **Roluperidone** | | | |
| | | **Placebo (N = 255)** | **32 mg (N = 248)** | **64 mg (N = 254)** | **Total (N = 502)** |

| Change from Baseline to Week 12 | | | | | |
|---|---|---|---|---|---|
| | N | 182 | 167 | 176 | 343 |
| | Mean (SD) | -0.2 (0.63) | -0.4 (0.65) | -0.4 (0.69) | -0.4 (0.67) |
| | Median | 0.0 | 0.0 | 0.0 | 0.0 |
| | Min, max | -3, 2 | -3, 1 | -3, 1 | -3, 1 |
| | LS mean (SE) | -0.2 (0.05) | -0.3 (0.05) | -0.4 (0.05) | |
| | 95% CI of LS mean | -0.3, -0.1 | -0.4, -0.2 | -0.5, -0.3 | |
| | Difference (95% CI) in LS mean (vs placebo) | | -0.1 (-0.2, 0.0) | -0.2 (-0.3, -0.0) | |
| | p-value (vs placebo) | | 0.084 | 0.007 | |

| | | | | | |
|---|---|---|---|---|---|
| CGI-S = Clinical Global Impression - Severity Scale; CI = confidence interval; ITT = intent-to-treat; LS = least square; SD = standard deviation; SE = standard error. ^{a} Baseline is defined as the last valid evaluation done before the study drug administration on Day 1 of the double-blind period. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline value as a covariate. An unstructured covariance structure is used. | | | | | |

### CGI-I

For the CGI-I scores at Week 12, there was no difference in LS mean for the 32 mg Roluperidone group compared with placebo (-0.0; 95% CI: -0.2, 0.2; p ≤ 0.760), and there was a statistically significantly superior LS mean for the 64 mg Roluperidone group compared with placebo (-0.2; 95% CI: -0.4, -0.0; p ≤ 0.037) (Table 22). Unadjusted p-values for the 64 mg Roluperidone group for Weeks 4 and 8 demonstrated early and consistent significantly different changes from placebo in CGI-I score (Table 22). Unadjusted p-values for the 32 mg Roluperidone group were only statistically significant at Week 4.

**Table 22. Summary of CGI-I Score by Visit - Double-blind Period, Pooled ITT Population**

| **Time Point** | | **Roluperidone** | | | |
|---|---|---|---|---|---|
| | | **Placebo (N = 255)** | **32 mg (N = 248)** | **64 mg (N = 254)** | **Total (N = 502)** |
| Week 2 | | | | | |
| | N | 162 | 162 | 166 | 32 8 |
| | Mean (SD) | 3.8 (0.67) | 3.9 (0.68) | 3.8 (0.74) | 3.9 (0.71) |
| | Median | 4.0 | 4.0 | 4.0 | 4.0 |
| | Min, max | 2, 6 | 2, 6 | 2, 6 | 2, 6 |
| | LS mean (SE) | 3.8 (0.05) | 3.9 (0.05) | 3.8 (0.05) | |
| | 95% CI of LS mean | 3.7, 3.9 | 3.8, 4.0 | 3.7, 3.9 | |
| | Difference (95% CI) in LS mean (vs placebo) | | 0.0 (-0.1, 0.2) | 0.0 (-0.1, 0.2) | |
| | p-value (vs placebo) | | 0.518 | 0.841 | |

| Week 4 | | | | | |
|---|---|---|---|---|---|
| | N | 224 | 208 | 212 | 42 0 |
| | Mean (SD) | 3.8 (0.73) | 3.6 (0.76) | 3.5 (0.72) | 3.6 (0.74) |
| | Median | 4.0 | 4.0 | 4.0 | 4.0 |
| | Min, max | 2, 6 | 2, 6 | 2, 6 | 2, 6 |
| | LS mean (SE) | 3.8 (0.05) | 3.6 (0.05) | 3.6 (0.05) | |
| | 95% CI of LS mean | 3.7, 3.9 | 3.5, 3.7 | 3.5, 3.7 | |
| | Difference (95% CI) in LS mean (vs placebo) | | -0.2 (-0.3, - 0.0) | -0.2 (-0.4, -0.1) | |
| | p-value (vs placebo) | | 0.022 | 0.003 | |

| **Time** Point | | **Roluperidone** | | | |
|---|---|---|---|---|---|
| | | **Placebo (N = 255)** | **32 mg (N = 248)** | **64 mg (N = 254)** | **Total (N = 502)** |
| Week 8 | | | | | |
| | N | 194 | 185 | 188 | 37 3 |
| | Mean (SD) | 3.6 (0.78) | 3.4 (0.80) | 3.4 (0.81) | 3.4 (0.81) |
| | Median | 4.0 | 3.0 | 3.0 | 3.0 |
| | Min, max | 1, 6 | 2, 6 | 2, 6 | 2, 6 |
| | LS mean (SE) | 3.6 (0.06) | 3.5 (0.06) | 3.4 (0.06) | |
| | 95% CI of LS mean | 3.5, 3.8 | 3.4, 3.7 | 3.3, 3.6 | |
| | Difference (95% CI) in LS mean (vs placebo) | | -0.1 (-0.3, 0.1) | -0.2 (-0.4, -0.0) | |
| | p-value (vs placebo) | | 0.205 | 0.019 | |

| Week 12 | | | | | |
|---|---|---|---|---|---|
| | N | 182 | 167 | 176 | 34 3 |
| | Mean (SD) | 3.5 (0.86) | 3.4 (0.82) | 3.3 (0.85) | 3.3 (0.83) |
| | Median | 3.0 | 3.0 | 3.0 | 3.0 |
| | Min, max | 1, 6 | 1, 5 | 1, 5 | 1, 5 |
| | LS mean (SE) | 3.6 (0.06) | 3.5 (0.07) | 3.4 (0.07) | |
| | 95% CI of LS mean | 3.4, 3.7 | 3.4, 3.7 | 3.2, 3.5 | |
| | Difference (95% CI) in LS mean (vs placebo) | | -0.0 (-0.2, 0.2) | -0.2 (-0.4, -0.0) | |
| | p-value (vs placebo) | | 0.760 | 0.037 | |

| | | | | | |
|---|---|---|---|---|---|
| CGI-I = Clinical Global Impression - Improvement Scale; CI = confidence interval; ITT = intent-to-treat; LS = least square; SD = standard deviation; SE = standard error. Note: LS mean, standard error, and p-value are from a mixed repeated measures model, with treatment (32 mg Roluperidone, 64 mg Roluperidone, and placebo), visit, and treatment-by-visit interaction as factors, a random effect for patient within treatment, and Baseline CGI-S values as a covariate. An unstructured covariance structure is used. | | | | | |

### GLOBAL STATISTICAL TEST (GST) OF POOLED EFFICACY DATA

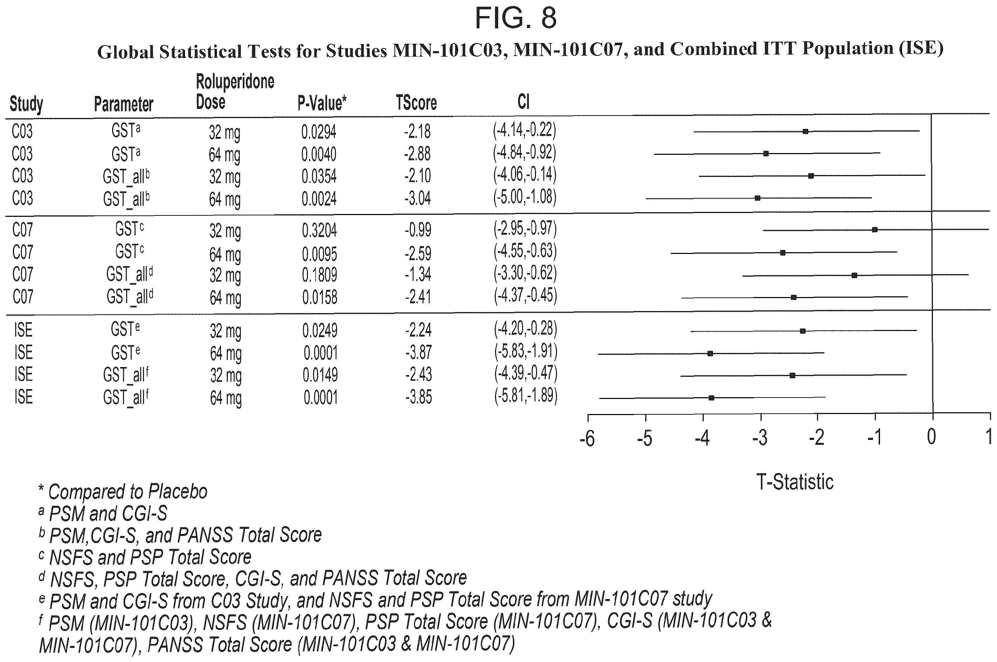

For the pooled data, using the mITT population from Study MIN-101C07, the GST combining the primary and secondary efficacy endpoints from Study MIN-101C03 and the primary and key secondary efficacy endpoints from Study MIN-101C07 studies for both Roluperidone dose groups achieved overall significant efficacy with p-values of 0.0249 (32 mg) and 0.0001 (64 mg; FIG. 8). An additional GST after adding several secondary endpoints from Study MIN-101C03 and Study MIN-101C07 as described for the individual studies showed that both Roluperidone 32 mg and 64 mg dose groups achieved overall significant efficacy with p-values of 0.0149 and 0.0001, respectively (FIG. 8). These findings support the conclusion that for the pooled data, Roluperidone at both doses was superior to placebo, and that the observed treatment effects are true effects of Roluperidone.

### RELAPSE

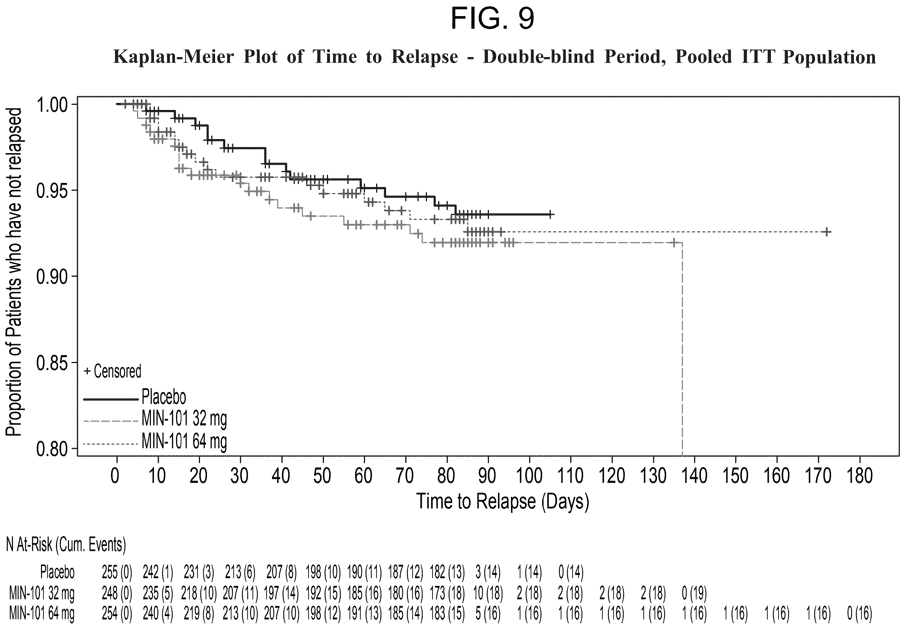

The number (percentage) of patients who experienced relapse in the pooled ITT population was similar in the placebo (14 [5%]) and 64 mg Roluperidone groups (16 [6%]), and higher in the 32 mg Roluperidone group (19 [8%]) (Table 23). Among the patients who relapsed, the mean number of days to relapse was 79.4 days for the placebo group, and 128.3 days and 81.4 days for the 32 mg and 64 mg Roluperidone groups, respectively (Table 23 and FIG. 9).

**Table 23. Summary of Rate of Relapse - Double blind Period, Pooled ITT Population**

| | | **Roluperidone** | | | |
|---|---|---|---|---|---|
| | | **Placebo (N = 255)** | **32 mg (N = 248)** | **64 mg (N = 254)** | **Total (N = 502)** |
| Patients who experienced relapse | | 14 (5%) | 19 (8%) | 16 (6%) | 35 (7%) |
| | Logistic Regression | | 0.434 | 0.773 | 0.546 |
| | p-value ^{a} | | | | |

| Days to Relapse (Kaplan-Meier Estimates) | | | | | |
|---|---|---|---|---|---|
| | n (Number Censored) | 255 (241) | 248 (229) | 254 (238) | 502 (467) |
| | Mean | 79.4 | 128.3 | 81.4 | 128.9 |
| | SE | 0.79 | 2.04 | 0.99 | 1.37 |
| | Median | NA | 137.0 | NA | 137.0 |
| | 95% CI | NA, NA | NA, NA | NA, NA | 137.0, NA |
| | Q1, Q3 | NA, NA | 137.0, 137.0 | NA, NA | 137.0, NA |
| | p-value ^{b} | | | | 0.618 |

| | | | | | |
|---|---|---|---|---|---|
| CI = confidence interval; ITT = intent-to-treat; NA = not applicable; PANSS = Positive and Negative Syndrome Scale; Q = quartile; SE = standard error. ^{a} p-value is from a logistic regression with a factor for treatment and Baseline PANSS total score as a covariate. ^{b} p-value is from a test of no treatment difference based on a Cox Proportional Hazards model with covariates for treatment group and Baseline PANSS total score. | | | | | |

### ANTIPSYCHOTIC EFFECTIVENESS AFTER USE OF ROLUPERIDONE

An analysis of the potential impact of Roluperidone administration on the efficacy of antipsychotic drugs was performed specifically to evaluate if patients taking Roluperidone who manifested worsening of positive symptoms experienced a diminished benefit after stopping Roluperidone and starting antipsychotic treatment. Across MIN-101C03 and MIN-101C07, 57 patients reported AEs/SAEs during the DB period that required discontinuation of Roluperidone and treatment with antipsychotics. The median times to symptom improvement was comparable irrespective of the treatment received (19 days on placebo, 12 days on 32 mg Roluperidone, and 17 days on 64 mg Roluperidone). The median time to symptom improvement after starting an antipsychotic was numerically greater in patients who received placebo compared with patients who received Roluperidone, although the numbers are too small to infer any difference. These durations of symptom improvement by antipsychotics are consistent with the range reported in the literature (Agid, O., et al. "The "delayed onset" of antipsychotic action - an idea whose time has come and gone." J Psychiatry Neurosci. 2006;31(2):93-100). These data demonstrate that the efficacy of antipsychotics was not diminished or impacted by exposure to Roluperidone.

### Comparison of Results in Subpopulations

Subgroup analyses were performed on NSFS, PSP Total score, CGI-S, and CGI-I for the DB period in the pooled ITT population. The subgroup analyses included age (≤ 40 years, > 40 years), BMI (< 25 kg/m², 25 to < 30 kg/m², ≥ 30 kg/m²), sex (male, female), race (White, Other), prior antipsychotic use (the group of patients taking antipsychotic medications prior to the study only), Baseline PANSS Marder NSFS score (< 20, ≥ 20 to < 30, ≥ 30), Baseline CGI-S (< 4, ≥ 4), and CYP2D6 (normal/extensive group only). In addition, the subgroup analysis by region for patients in the USA compared to patients in the Rest of the World are presented for Study MIN-101C07, which was the only study to include patients in the USA.

**Age (≤ 40 Years vs > 40 Years) -** Overall, observed treatment differences on the various efficacy endpoints for the 32 mg and 64 mg Roluperidone groups versus placebo by age subgroups (≤ 40 years and > 40 years) were comparable.

**Body Mass Index -** Overall, the efficacy of the 64 mg and 32 mg Roluperidone groups compared with placebo was generally similar in the normal (< 25 kg/m2) and overweight (25 to < 30 kg/m2) BMI subgroups. In the 64 mg Roluperidone group, lower improvements in efficacy were seen in the obese (≥ 30 kg/m2) BMI subgroup compared with the other BMI categories. The lower efficacy seen in the 64 mg Roluperidone group in the obese subgroup should be interpreted with caution due to the small sample size and may be due to decreased exposure to Roluperidone. For the change from Baseline in NSFS scores at Week 12, the 64 mg Roluperidone group had a greater improvement compared with placebo for both the normal and overweight BMI subgroups (LS mean change from Baseline compared with placebo was -1.7 and -1.4, respectively), while the improvement for the subgroup of obese patients was small (LS mean change from Baseline compared with placebo was -0.1).

**Sex** - Overall, no sex differences were observed for the 64 mg Roluperidone group compared with placebo.

**Race** - Because patients in this study were predominantly White (694/757 [91.7%] patients were White and 63/757 [8.3%] patients were other races), no meaningful comparisons could be made between the White and Other race subgroups for any of the endpoints.

**Prior Antipsychotic Use** - Subgroup analyses demonstrate the superiority of 64 mg Roluperidone over placebo for most endpoints in these patients.

### Disease Severity (Moderate vs Severe vs Very Severe)

- Baseline NSFS Score: Overall, the effect of 64 mg Roluperidone compared with placebo seen in patients with NSFS < 20 at Baseline (less severe disease; n=8) was similar to the effect seen in patients with NSFS scores of ≥ 20 to < 30 at Baseline (moderately severe disease; n=221), whereas no effect compared with placebo was seen in patients with NSFS ≥ 30 at Baseline (severe disease; n=25). However, the findings in the severe group should be interpreted with caution based on the small sample size in this group and the large variability in the placebo group. Similar results were observed for PSP Total scores for the 64 mg Roluperidone group compared with placebo by NSFS Baseline scores. For the 32 mg Roluperidone compared with placebo by NSFS Baseline scores groups as well as for PSP Total score by Baseline scores groups similar patterns were observed.
- Baseline CGI-S Score: Overall, the effect of 64 mg Roluperidone compared with placebo seen in patients with moderate to severe disease at Baseline (CGI-S scores of ≥ 4; n=218) was similar to the effect seen in patients with mild disease at Baseline (CGI-S scores of < 4; n=36) The low number of patients with mild disease at Baseline does not allow for a conclusion to be drawn. A similar trend was observed for PSP Total scores for the 64 mg Roluperidone group compared with placebo by CGI-S Baseline scores. For the 32 mg Roluperidone compared with placebo by NSFS Baseline scores groups as well as for PSP Total score by Baseline scores groups similar patterns were observed.

**Efficacy in US vs Non-US Patients -** In the ITT population at Baseline of Study MIN-101C07, there were 81 patients in the US region ([27 patients each in of the placebo and Roluperidone groups) compared to 432 in the Rest of the World (145 patients in the placebo group, 143 patients in the 32 mg Roluperidone group, and 144 patients in the 64 mg Roluperidone group). A subgroup analysis of these 2 subgroups for the change from Baseline in NSFS to Week 12 showed generally similar results across all treatment groups in both regions. The 64 mg dose showed a robust effect at both the US sites and non-US sites, with a larger effect size in the US sites (0.32) compared to non-US sites (0.20) despite the larger placebo response in the US. These findings should be interpreted with caution due to the small sample size in the US region.

### Persistence of Efficacy and/or Tolerance Effects

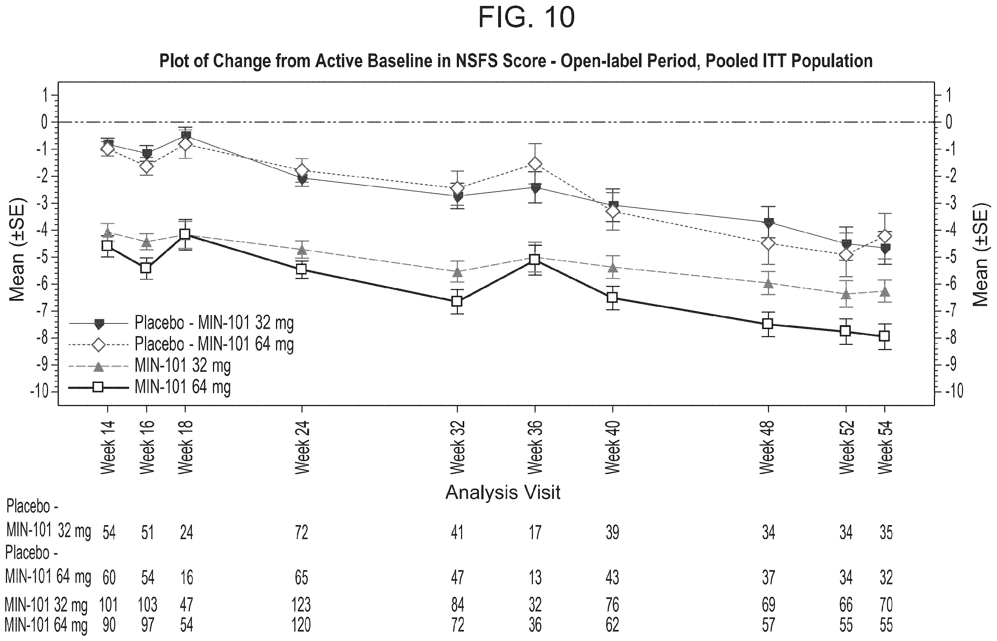

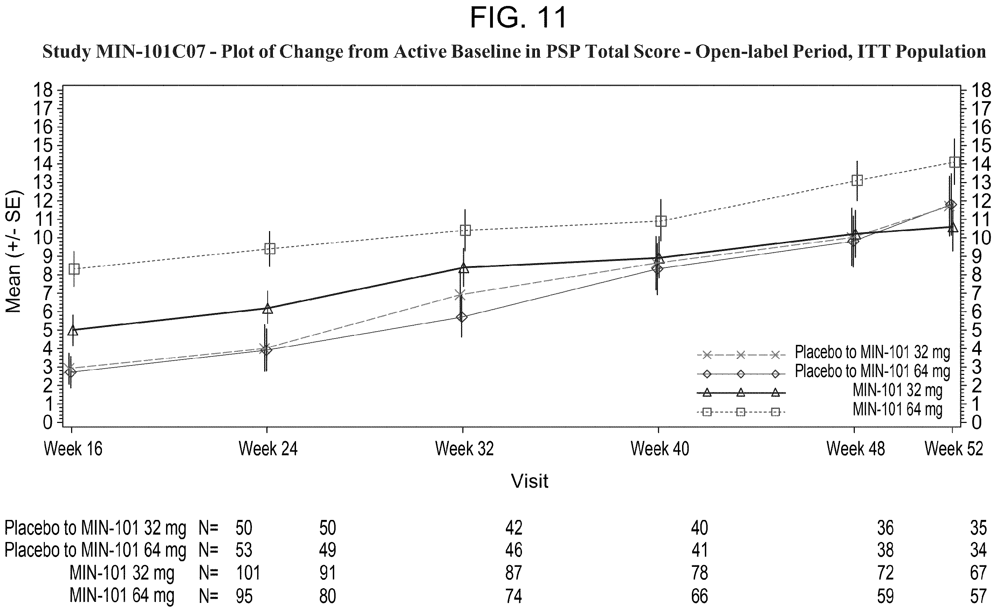

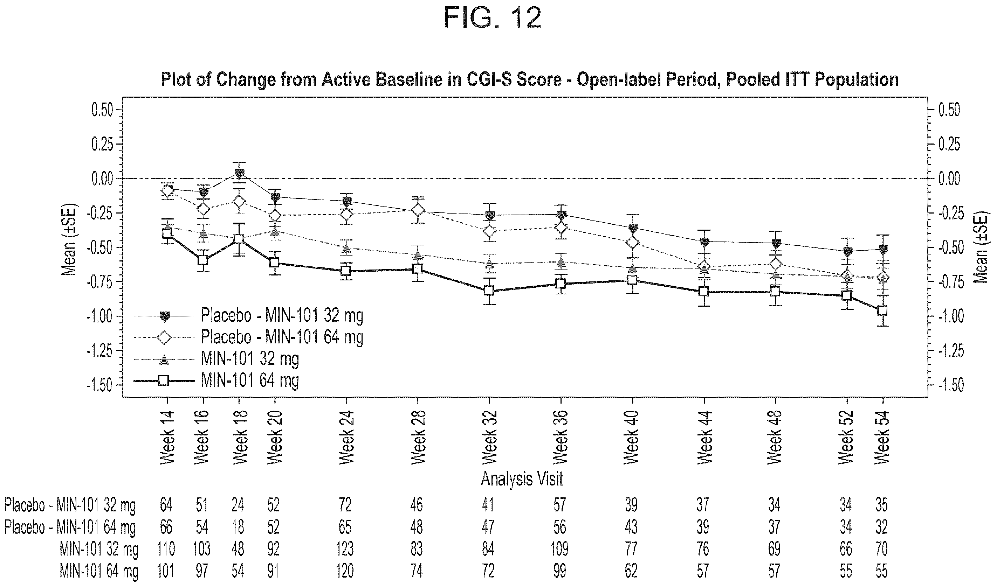

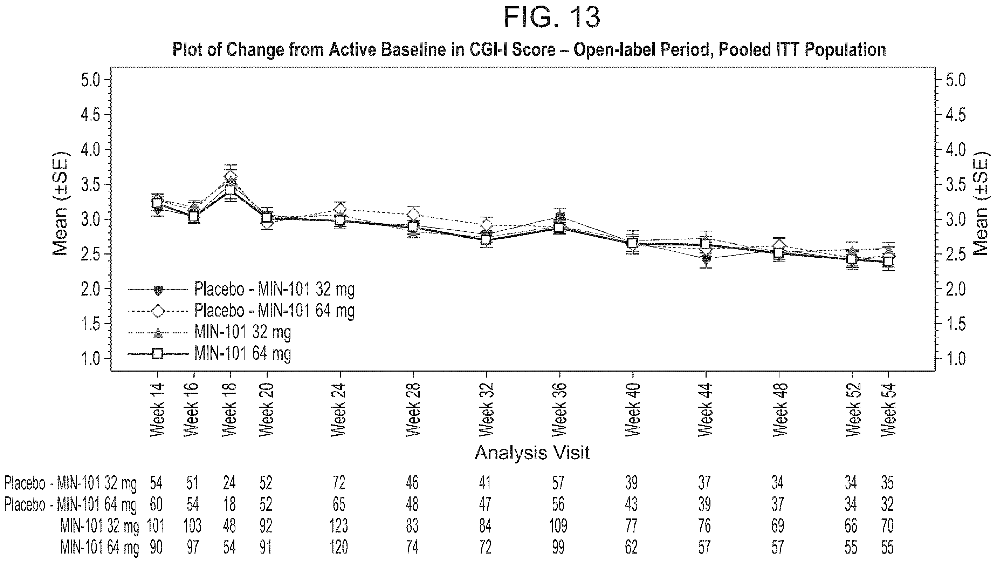

The improvements in the efficacy parameters observed during the DB period were sustained and continued during the OL period. Overall, the changes from Baseline in NSFS and PSP Total scores persisted through the OL period for patients in the 32 mg and 64 mg Roluperidone throughout groups, and the changes in efficacy parameters seen for the patients who switched from placebo to 32 mg and 64 mg Roluperidone were consistent to the Roluperidone groups during the DB period (FIG. 10 AND FIG. 11). Additionally, based on Study MIN-101C07, at the follow-up visit, the improvements in NSFS scores for both doses were sustained for at least 2 weeks after the last dose, when patients were no longer receiving Roluperidone (Table 24). Change from Baseline in CGI-S and CGI-I scores continued to show improvements during the OL period (FIG. 12 AND FIG. 13). Taken together, the results from the OL period demonstrate an ongoing and robust response in patients treated with 32 mg or 64 mg Roluperidone based on the primary efficacy endpoint of NSFS score and supported by results seen with the PSP Total score (the key secondary endpoint in Study MIN-101C07), as well as the CGI-S and the CGI-I.

### OPEN-LABEL INTEGRATED EFFICACY RESULTS

NSFS - the improvements in the mean NSFS score observed during the DB period for the 32 mg and 64 mg Roluperidone groups were sustained and continued during the OL period, including for 2 weeks after the end of study treatment (based on assessments at Week 54 in Study MIN-101C07). Improvements from Baseline in mean NSFS score were also seen for the patients who switched from placebo to 32 mg and 64 mg Roluperidone that were consistent to the Roluperidone treatment groups during the DB period (Table 24 and FIG. 10). Although Week 24 was the only common week shared between Study MIN-101C03 and MIN-101C07 in the OL period, from FIG. 10 it can be seen that the improvements in the NSFS score were maintained over the course of the OL period for the 32 and 64 mg Roluperidone groups.

**Table 24. Summary of NSFS Score Changes from Baseline at Weeks 24 52, and 54 - OL and WS, Pooled ITT Population**

| **Time Point** | | **Placebo in DB to Roluperidone in OL** | | | **Roluperidone at Any Time** | | |
|---|---|---|---|---|---|---|---|
| | | **32 mg (N = 128)** | **64 mg (N = 127)** | **Total (N = 255)** | **32 mg (N = 376)** | **64 mg (N = 381)** | **Total (N = 502)** |
| **Active Baseline (BL)^{a}** | | | | | | | |
| | N | 84 | 82 | 166 | 332 | 336 | 502 |
| | Mean (SD) | 21.6 (4.53) | 22.2 (4.04) | 21.9 (4.29) | 24.3 (4.21) | 24.6 (3.88) | 25.3 (3.59) |
| | Median | 22.0 | 23.0 | 22.0 | 24.0 | 24.0 | 25.0 |
| | Min, Max | 7, 31 | 11, 31 | 7, 31 | 7, 39 | 11, 36 | 16, 39 |

| **Open-label BL** | | | | | | | |
|---|---|---|---|---|---|---|---|
| | **N** | 84 | 82 | 166 | 236 | 239 | 309 |
| | Mean (SD) | 21.6 (4.53) | 22.2 (4.04) | 21.9 (4.29) | 21.5 (4.54) | 21.3 (4.53) | 21.2 (4.64) |
| | Median | 22.0 | 23.0 | 22.0 | 22.0 | 21.0 | 21.0 |
| | Min, max | 7, 31 | 11, 31 | 7, 31 | 7, 35 | 7, 31 | 7, 35 |

| **Study BL** | | | | | | | |
|---|---|---|---|---|---|---|---|
| | N | 128 | 127 | 255 | 376 | 381 | 502 |
| | Mean (SD) | 24.3 (3.41) | 24.8 (3.32) | 24.6 (3.37) | 24.9 (3.61) | 25.1 (3.45) | 25.3 (3.59) |
| | Median | 24.0 | 25.0 | 24.0 | 24.0 | 25.0 | 25.0 |
| | **Min, max** | 17, 35 | 17, 34 | 17, 35 | 16, 39 | 17, 36 | 16, 39 |

| **Change from Active BL to Week 24** | | | | | | | |
|---|---|---|---|---|---|---|---|
| | N | 72 | 65 | 137 | 195 | 185 | 243 |
| | Mean (SD) | -2.1 (2.56) | -1.8 (3.55) | -1.9 (3.06) | -3.7 (3.45) | -4.2 (3.92) | -5.1 (3.52) |
| | Median | -2.0 | -2.0 | -2.0 | -4.0 | -4.0 | -5.0 |
| | Min, max | -8, 5 | -11, 11 | -11, 11 | -15, 7 | -16, 11 | -16, 7 |

| **Change from Open-label BL to Week 24** | | | | | | | |
|---|---|---|---|---|---|---|---|
| | **N** | 72 | 65 | 137 | 195 | 185 | 243 |
| | Mean (SD) | -2.1 (2.56) | -1.8 (3.55) | -1.9 (3.06) | -1.4 (2.51) | -1.6 (3.02) | -1.3 (2.56) |
| | Median | -2.0 | -2.0 | -2.0 | -1.0 | -1.0 | -1.0 |
| | Min, max | -8, 5 | -11, 11 | -11, 11 | -9, 8 | -11, 11 | -9, 8 |

| **Time Point** | | **Placebo in DB to Roluperidone in OL** | | | **Roluperidone at Any Time** | | |
|---|---|---|---|---|---|---|---|
| | | **32 mg (N = 128)** | **64 mg (N = 127)** | **Total (N = 255)** | **32 mg (N = 376)** | **64 mg (N = 381)** | **Total (N = 502)** |
| **Change from Study BL to Week 24** | | | | | | | |
| | N | 72 | 65 | 137 | 195 | 185 | 243 |
| Mean (SD) | | -5.3 (3.72) | -4.2 (3.38) | -4.8 (3.60) | -4.9 (3.61) | -5.0 (3.49) | -5.1 (3.52) |
| | Median | -5.0 | -4.0 | -4.0 | -5.0 | -5.0 | -5.0 |
| | Min, max | -15, 3 | -10, 3 | -15, 3 | -15, 7 | -16, 3 | -16, 7 |

| **Change from Active BL to Week 52** | | | | | | | |
|---|---|---|---|---|---|---|---|
| | N | 34 | 34 | 68 | 100 | 89 | 121 |
| Mean (SD) | | -4.5 (3.60) | -4.9 (4.72) | -4.7 (4.17) | -5.7 (3.95) | -6.7 (4.23) | -7.0 (3.84) |
| | Median | -4.0 | -6.0 | -5.0 | -5.0 | -7.0 | -6.0 |
| | Min, max | -14, 1 | -15, 5 | -15, 5 | -16, 4 | -16, 5 | -16, 4 |

| **Change from Open-label BL to Week 52** | | | | | | | |
|---|---|---|---|---|---|---|---|
| | N | 34 | 34 | 68 | 100 | 89 | 121 |
| Mean (SD) | | -4.5 (3.60) | -4.9 (4.72) | -4.7 (4.17) | -3.3 (3.23) | -4.3 (3.77) | -3.2 (2.99) |
| | Median | -4.0 | -6.0 | -5.0 | -3.0 | -4.0 | -3.0 |
| | Min, max | -14, 1 | -15, 5 | -15, 5 | -14, 6 | -15, 5 | -10, 6 |

| **Change from Study BL to Week 52** | | | | | | | |
|---|---|---|---|---|---|---|---|
| | N | 34 | 34 | 68 | 100 | 89 | 121 |
| Mean (SD) | | -7.9 (3.69) | -7.0 (3.76) | -7.5 (3.72) | -6.9 (3.95) | -7.5 (3.61) | -7.0 (3.84) |
| | Median | -8.0 | -7.5 | -8.0 | -6.0 | -7.0 | -6.0 |
| | Min, max | -16, -1 | -14, 0 | -16, 0 | -16, 4 | -16, 1 | -16, 4 |

| **Change from Active BL to Week 54** | | | | | | | |
|---|---|---|---|---|---|---|---|
| | **N** | 35 | 32 | 67 | 105 | 87 | 125 |
| Mean (SD) | | -4.7 (3.60) | -4.2 (4.78) | -4.4 (4.18) | -5.7 (3.55) | -6.6 (4.40) | -7.0 (3.56) |
| | Median | -4.0 | -5.0 | -5.0 | -6.0 | -7.0 | -7.0 |
| | Min, max | -11, 1 | -14, 7 | -14, 7 | -14, 1 | -16, 7 | -16, 0 |

| **Time Point** | | **Placebo in DB to Roluperidone in OL** | | | **Roluperidone at Any Time** | | |
|---|---|---|---|---|---|---|---|
| | | **32 mg (N = 128)** | **64 mg (N = 127)** | **Total (N = 255)** | **32 mg (N = 376)** | **64 mg (N = 381)** | **Total (N = 502)** |
| **Change from Open-label BL to Week 54** | | | | | | | |
| | N | 35 | 32 | 67 | 105 | 87 | 125 |
| Mean (SD) | | -4.7 (3.60) | -4.2 (4.78) | -4.4 (4.18) | -3.3 (3.45) | -4.0 (3.88) | -3.2 (3.29) |
| | Median | -4.0 | -5.0 | -5.0 | -3.0 | -4.0 | -3.0 |
| | Min, max | -11, 1 | -14, 7 | -14, 7 | -11, 8 | -14, 7 | -11, 8 |

| **Change from Study BL to Week 54** | | | | | | | |
|---|---|---|---|---|---|---|---|
| | N | 35 | 32 | 67 | 105 | 87 | 125 |
| Mean (SD) | | -7.9 (4.29) | -6.6 (4.15) | -7.3 (4.25) | -6.8 (3.80) | -7.4 (3.81) | -7.0 (3.56) |
| | Median | -7.0 | -7.0 | -7.0 | -6.0 | -7.0 | -7.0 |
| | Min, max | -21, -1 | -18, 3 | -21, 3 | -21, 0 | -18, 3 | -16, 0 |

| | | | | | | | |
|---|---|---|---|---|---|---|---|
| DB = double-blind; ITT = intent-to-treat; NSFS = PANSS Marder's negative symptoms factor score; OL = open-label; PANSS = Positive and Negative Syndrome Scale; SD = standard deviation. ^{a} Active Baseline is defined as the last valid value obtained prior to the first dose of Roluperidone. ^{b} OL Baseline is defined as the last valid value obtained prior to the first dose of OL study drug. ^{c} Study Baseline is defined as the last valid value obtained prior to the first dose of study drug during the double-blind period. Note: The number of patients decreased at Weeks 52 and 54 because Study MIN-101C03 patients are not included. | | | | | | | |

### PSP TOTAL SCORE

Improvements in PSP Total score observed during the DB period for the 32 mg and 64 mg Roluperidone groups were sustained and continued during the OL period in Study MIN-101C07 (FIG. 11) (PSP Total score was not captured in Study MIN-101C03 in the OL period). A similar mean change in PSP Total score from Active Baseline at Week 52 was seen in the placebo to 32 mg treatment group (11.7) compared with the placebo to 64 mg treatment group (11.8).

### CGI-S

The improvements in the CGI-S score observed during the DB period for the 32 mg and 64 mg Roluperidone groups were sustained and continued during the OL period, and changes from Baseline in CGI-S seen for patients who switched from placebo to 32 mg and 64 mg Roluperidone were consistent with the Roluperidone treatment groups during the DB period (FIG. 12).

### CGI-I

The improvements in CGI-I score observed during the DB period for the 32 mg and 64 mg Roluperidone groups were sustained and continued during the OL period, and the improvements in the CGI-I score seen for the patients who switched from placebo to 32 mg and 64 mg Roluperidone were consistent with the Roluperidone treatment groups during the DB period (FIG. 13).

### Relapse

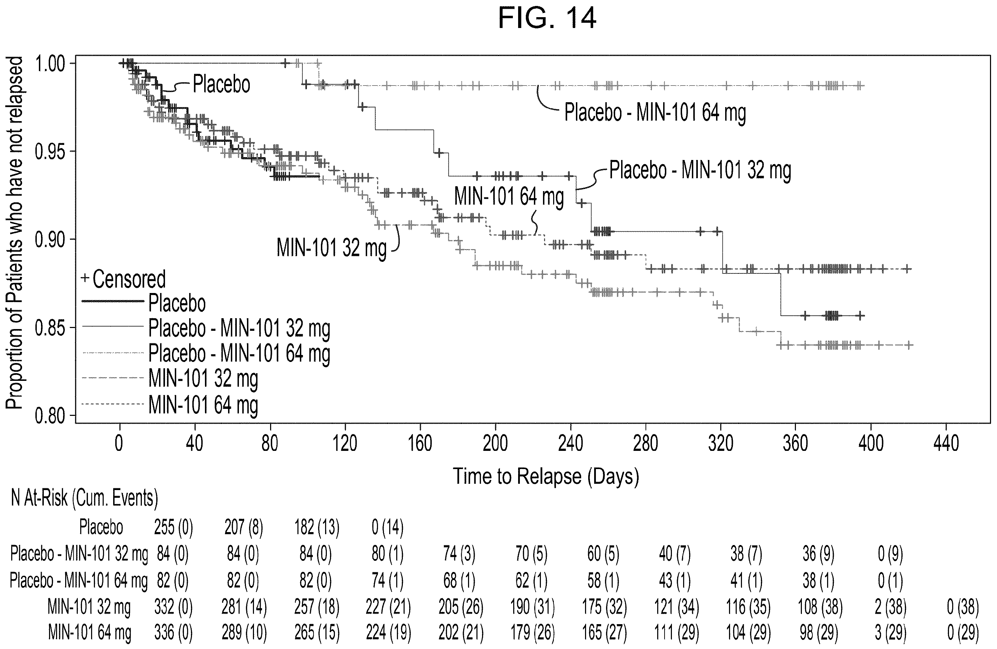

The number (percentage) of patients who experienced relapse (worsening of symptoms) in the ITT population during the OL period was low relative to literature reports for all 4 treatment groups (9 [11%] for placebo to 32 mg Roluperidone, 1 [1%] for placebo to 64 mg Roluperidone, 10 [7%] for 32 mg Roluperidone throughout, and 12 [8%] for 64 mg Roluperidone throughout). Among the patients who relapsed, the mean number of days to relapse was 256.3 in the total placebo to Roluperidone group, 234.8 in the 32 mg Roluperidone group, and 188.1 in the 64 mg Roluperidone group. The Kaplan-Meier plot of time to relapse for the whole period is shown in FIG. 14.

While rates of study discontinuation vary widely between different studies and the antipsychotic drugs assessed, the reported discontinuation rate in the literature over similar study duration is around 40% for patients on placebo and around 20% or higher for patients on antipsychotic drugs. For example, see Leucht et al., "Antipsychotic drugs versus placebo for relapse prevention in schizophrenia: a systematic review and meta-analysis," www.thelancet.com, June 2, 2012, 379, 2063-2071, (drug 27% relapse rate after 1 year - data taken from 116 reports from 65 trials with 6,493 patients); Arato et al., "A 1-year, double-blind, placebo-controlled trial of ziprasidone 40, 80 and 160 mg/day in chronic schizophrenia: the Ziprasidone Extended Use in Schizophrenia (ZEUS) study," International Clinical Psychopharmacology, 2002, 17, 207-215, (ziprasidone 40, 80, and 160 mg/day groups, 43%, 35% and 36%, relapse rates, respectively, after one year); and Durgam et al., "Long-term cariprazine treatment for the prevention of relapse in patients with schizophrenia: A randomized, double-blind, placebo-controlled trial," Schizophrenia Research, 2016, 176, 264-271, (cariprazine, relapse rate of 24.8%).

### EXAMPLE 5 - SUMMARY OF TWO LATE-STAGE CLINICAL STUDIES

In the 36-weeks in Study #1, and 52-weeks in Study #2, re-emergence of, or increase in, positive symptoms occurred only in a limited number of patients. This is reflected by the stability of both the positive symptoms PANSS subscale score and the PANSS total score, and by the low incidence of relapses. Only 15.2% of subjects in Study #1 and 11.7% of subjects in Study #2 discontinued study participation because of positive symptoms worsening. (Table 25.)

**Table 25. Relapse by Study Phase and Overall**

| Phase | Study # 1 (n = 244) | Study #2 (n = 513) |
|---|---|---|
| Double-blind (12-weeks) | 11.5% | 6.8% |
| Open-label | 6% (over 24 weeks) | 7.5% (over 40 weeks) |
| Overall | 15.2% (over 36 weeks) | 11.7% (over 52 weeks) |

In contrast, the reported relapse rate in the literature for other antipsychotic drugs is higher (vide supra). This low rate of discontinuation observed in the Applicant's studies compared to the rate reported in the literature further underlines the unique characteristics of Roluperidone in preventing relapse in schizophrenic patients in general, and in the schizophrenia patient populations selected by the enrolment criteria for these two studies.

### EXAMPLE 6: ROLUPERIDONE MONOTHERAPY PREVENTS RELAPSE OVER 2+ YEARS

Patient X presented with flat effect and restricted emotional expression, poor motivation to do much more than watch television and occasionally participate in occupational therapy for a few hours a week. Patient could not fully concentrate enough to even read. During the course of monotherapy with Roluperidone (64 mg/day), patient saw gradual improvement in her negative and cognitive symptoms. This allowed the patient to find employment. Her affect is no longer restricted and she reads full novels on a regular basis with no trouble concentrating, and enjoys and looks forward to all her various daily activities. She has not had a relapse in the two years she has taken Roluperidone (64 mg/day) as a monotherapy.
The invention is further described with reference to the following numbered clauses:
1. A method of preventing relapse in a schizophrenia patient, wherein the method comprises administering a therapeutically effective amount of Roluperidone to the schizophrenia patient.
2. The method of clause 1, wherein the therapeutically effective amount of Roluperidone is administered to the schizophrenia patient once or twice a day.
3. The method of clause 1 or 2, wherein the therapeutically effective amount of Roluperidone is administered to the schizophrenia patient once a day.
4. The method of any one of clauses 1-3, wherein the therapeutically effective amount of Roluperidone is administered orally to the schizophrenia patient.
5. The method of any one of clauses 1-4, wherein the therapeutically effective amount of Roluperidone is between about 1 and about 100 mg.
6. The method of clause 5, wherein the therapeutically effective amount of Roluperidone is about 16 mg, about 24 mg, about 32 mg, about 40 mg, about 48 mg, about 56 mg, about 64 mg, about 72 mg, about 80 mg, about 88 mg, or about 96 mg.
7. The method of clause 5, wherein the therapeutically effective amount of Roluperidone is about 32 mg.
8. The method of clause 5, wherein the therapeutically effective amount of Roluperidone is about 64 mg.
9. The method of any one of clauses 1-8, wherein the schizophrenia patient does not manifest any behavior which puts the patient, or those around the patient, at risk of bodily harm.
10. The method of any one of clauses 1-9, wherein the schizophrenia patient has low levels of symptoms related to agitation, impulse control, hostility, suspiciousness, or uncooperativeness.
11. The method of any one of clauses 1-10, wherein the schizophrenia patient does not experience a high level of depression or anxiety.
12. The method of any one of clauses 1-11, wherein the schizophrenia patient: (i) has positive symptoms that are stable before starting treatment with Roluperidone; or (ii) does not have positive symptoms before starting treatment with Roluperidone.
13. The method of any one of clauses 1-12, wherein the schizophrenia patient: (i) has positive symptoms that are stable for about 1 to about 6 months, or about 3 to about 6 months, before starting treatment with Roluperidone; or (ii) does not have positive symptoms for about 1 to about 6 months, or about 3 to about 6 months, before starting treatment with Roluperidone.
14. The method of any one of clauses 1-13, wherein the schizophrenia patient has:
   (i) negative symptoms that are moderate to severe; or
   (ii) a PANSS negative subscore that is greater than 20.
15. The method of clause 14, wherein the schizophrenia patient's negative symptoms are primary negative symptoms.
16. The method of clause 14 or 15, wherein the schizophrenia patient's negative symptoms are stable for about 1 to about 6 months before starting treatment with Roluperidone.
17. The method of any one of clauses 14-16, wherein the schizophrenia patient's negative symptoms are stable for about 3 to about 6 months before starting treatment with Roluperidone.
18. The method of any one of clauses 1-17, wherein the schizophrenia patient was previously administered an antipsychotic.
19. The method of clause 18, wherein the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months, or at least 12 months before the schizophrenia patient was administered Roluperidone.
20. The method of any one of clauses 1-19, wherein relapse is an increase in positive symptoms in the schizophrenia patient, optionally wherein the increase in positive symptoms in the schizophrenia patient is marked by an increase in the patient's PANSS positive subscore on one or more consecutive visits.
21. A method of selecting a schizophrenia patient and preventing relapse in the schizophrenia patient, wherein the method comprises:
   (a) selecting the schizophrenia patient as having a form of schizophrenia characterized by the schizophrenia patient having moderate to severe negative symptoms that are stable for about 3 to about 6 months; and
   (b) administering a therapeutically effective amount of Roluperidone to the schizophrenia patient.
22. The method of clause 21, wherein the schizophrenia patient has a PANSS negative subscore that is greater than 20.
23. The method of clause 21 or 22, wherein the schizophrenia patient's negative symptoms are primary negative symptoms.
24. The method of any one of clauses 21-23, wherein the schizophrenia patient has positive symptoms that are stable before starting treatment with Roluperidone; optionally wherein the schizophrenia patient has positive symptoms that are stable for about 1 to about 6 months, or about 3 to about 6 months, before starting treatment with Roluperidone.
25. The method of any one of clauses 21-23, wherein the schizophrenia patient does not have positive symptoms before starting treatment with Roluperidone; optionally wherein the schizophrenia patient does not have positive symptoms for about 1 to about 6 months, or about 3 months to about 6 months, before starting treatment with Roluperidone.
26. The method of any one of clauses 21-25, wherein the schizophrenia patient does not manifest any behavior which puts the patient, or those around the patient, at risk of bodily harm; has low levels of symptoms related to agitation, impulse control, hostility, suspiciousness, or uncooperativeness; and/or does not experience a high level of depression or anxiety.
27. The method of any one of clauses 21-26, wherein the schizophrenia patient was previously administered an antipsychotic.
28. The method of clause 27, wherein the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months, or at least 12 months before the schizophrenia patient was administered Roluperidone.
29. The method of any one of clauses 21-28, wherein relapse is an increase in positive symptoms in the schizophrenia patient, optionally wherein the increase in positive symptoms in the schizophrenia patient is marked by an increase in the patient's PANSS positive subscore on one or more consecutive visits.
30. The method of any one of clauses 21-29, wherein the therapeutically effective amount of Roluperidone is administered to the schizophrenia patient once or twice a day.
31. The method of any one of clauses 21-30, wherein the therapeutically effective amount of Roluperidone is administered to the schizophrenia patient once a day.
32. The method of any one of clauses 21-31, wherein the therapeutically effective amount of Roluperidone is administered orally to the schizophrenia patient.
33. The method of any one of clauses 21-32, wherein the therapeutically effective amount of Roluperidone is between about 1 and about 100 mg.
34. The method of clause 33, wherein the therapeutically effective amount of Roluperidone is about 16 mg, about 24 mg, about 32 mg, about 40 mg, about 48 mg, about 56 mg, about 64 mg, about 72 mg, about 80 mg, about 88 mg, or about 96 mg.
35. The method of clause 33, wherein the therapeutically effective amount of Roluperidone is about 32 mg.
36. The method of clause 33, wherein the therapeutically effective amount of Roluperidone is about 64 mg.

## Claims

1. Roluperidone for use in a method of preventing relapse in a schizophrenia patient, wherein the method comprises administering a therapeutically effective amount of Roluperidone to the schizophrenia patient, and wherein the schizophrenia patient has:
(i) positive symptoms that are stable before starting treatment with Roluperidone; and
(ii) negative symptoms that are moderate to severe or a PANSS negative subscore that is greater than 20.

2. Roluperidone for use as claimed in claim 1, wherein the therapeutically effective amount of Roluperidone is administered to the schizophrenia patient:
a) once or twice a day, preferably once a day; and/or
b) orally.

3. Roluperidone for use as claimed in claim 1 or 2, wherein the therapeutically effective amount of Roluperidone is between 1 and 100 mg ± 20%;
optionally wherein the therapeutically effective amount of Roluperidone is:
a) 16 mg ± 20%, 24 mg ± 20%, 32 mg ± 20%, 40 mg ± 20%, 48 mg ± 20%, 56 mg ± 20%, 64 mg ± 20%, 72 mg ± 20%, 80 mg ± 20%, 88 mg ± 20% or 96 mg ± 20%;
b) 32 mg ± 20%; or
c) 64 mg ± 20%.

4. Roluperidone for use as claimed in any one of claims 1-3, wherein the schizophrenia patient:
a) does not manifest any behavior which puts the patient, or those around the patient, at risk of bodily harm;
b) has low levels of symptoms related to agitation, impulse control, hostility, suspiciousness or uncooperativeness; and/or
c) does not experience a high level of depression or anxiety.

5. Roluperidone for use as claimed in any one of claims 1-4, wherein the schizophrenia patient's negative symptoms are:
a) primary negative symptoms;
b) stable for 1 to 6 months before starting treatment with Roluperidone; and/or
c) stable for 3 to 6 months before starting treatment with Roluperidone.

6. Roluperidone for use as claimed in any one of claims 1-5, wherein the schizophrenia patient was previously administered an antipsychotic;
optionally wherein the administration of the antipsychotic to the schizophrenia patient was discontinued at least 1 day, at least 2 days, at least 3 days, at least 4 days, at least 5 days, at least 6 days, at least 1 week, at least 2 weeks, at least 3 weeks, at least 1 month, at least 2 months, at least 3 months, at least 6 months, at least 9 months or at least 12 months before the schizophrenia patient was administered Roluperidone.

7. Roluperidone for use as claimed in any one of claims 1-6, wherein relapse is an increase in positive symptoms in the schizophrenia patient,
optionally wherein the increase in positive symptoms in the schizophrenia patient is marked by an increase in the patient's PANSS positive subscore on one or more consecutive visits.

8. Roluperidone for use as claimed in any one of claims 1-7, wherein the schizophrenia patient has at least one functional CYP2D6 allele.
